(19) Europäisches Patentamt
European Patent Office
Office européen des brevets

(11) EP 1 469 071 A1

(12) # EUROPEAN PATENT APPLICATION
published in accordance with Art. 158(3) EPC

(43) Date of publication:
**20.10.2004 Bulletin 2004/43**

(21) Application number: **03703025.1**

(22) Date of filing: **22.01.2003**

(51) Int Cl.[7]: **C12N 15/12**, C12N 15/62, C12N 15/81, C07K 14/705, C07K 16/18, C12N 1/19, C12N 1/21, A61P 3/10, C12Q 1/66

(86) International application number:
**PCT/JP2003/000546**

(87) International publication number:
**WO 2003/062427 (31.07.2003 Gazette 2003/31)**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **23.01.2002 JP 2002013721**
**03.09.2002 JP 2002025703**

(71) Applicant: **YAMANOUCHI PHARMACEUTICAL CO. LTD.**
**Tokyo 103-8411 (JP)**

(72) Inventors:
- **ENDOH, Hideki**
**Yamanouchi Pharmaceutical Co., Ltd.**
**Tsukuba-shi, Ibaraki 305-8585 (JP)**
- **NAKANO, Ryosuke**
**Yamanouchi Pharmaceutical Co.,Ltd**
**Tsukuba-shi, Ibaraki 305-8585 (JP)**
- **KUROSAKI, Eiji**
**Yamanouchi Pharmaceutical Co.,Ltd.**
**Tsukuba-shi, Ibaraki 305-8585 (JP)**
- **KATO, Miyuki**
**Yamanouchi Pharmaceutical Co., Ltd.**
**Tsukuba-shi, Ibaraki 305-8585 (JP)**
- **YOKOTA,Hiroyuki**
**Yamanouchi Pharmaceutical Co., Ltd**
**Tsukuba-shi, Ibaraki 305-8585 (JP)**
- **INABE, Kazunori**
**Yamanouchi Pharmaceutical Co.,Ltd**
**Tsukuba-shi, Ibaraki 305-8585 (JP)**

(74) Representative: **Bates, Philip Ian et al**
**Reddie & Grose**
**16 Theobalds Road**
**London WC1X 8PL (GB)**

(54) **METHOD OF SCREENING DRUG FOR IMPROVING INSULIN RESISTANCE**

(57) Disclosing a method for screening a protein interactive with PPAR in a ligand-dependent manner, works as a useful tool for screening a drug ameliorating insulin resistance. By the method, ECHLP as a main action ligand-dependent PPAR binding molecule, FLJ13111 as a main action ligand-selective factor interactive with PPARγ and AOP2 as an adverse action ligand-dependent PPAR binding molecule were obtained. By using ECHLP interactive with PPAR, FLJ13111 interactive with PPAR and AOP2 interactive with PPAR, a screening system for a drug ameliorating insulin resistance is constructed and disclosed, the drug giving selectively the main action with no occurrence of the adverse action. Additionally, a method for producing a pharmaceutical composition for ameliorating insulin resistance is disclosed, which contains as the active component, a promoting agent of the main action through PPAR, an agonist specific to the main action through PPAR, an inhibitor of ECHLP interactive with PPAR to promote the main action through PPAR, a substance suppressing the adverse action through PPARγ, an inhibitor of AOP2 interactive with PPAR to suppress the adverse action through PPARγ, an activating agent of FLJ13111 interactive with PPAR to promote the main action through PPAR or an activator of FLJ13111 expression.

EP 1 469 071 A1

SEQUENCE LISTING

<110> Yamanouchi Pharmaceutical Co.,Ltd.

<120> A screening method for detecting PPAR modulators

<130> Y0304-PCT

<150> JP2002-013721
<151> 2002-01-23

<150> JP2002-257703
<151> 2002-09-03

<160> 28

<170> PatentIn version 3.1

<210> 1
<211> 1518
<212> DNA
<213> Homo sapiens

<220>
<221> CDS
<222> (1)..(1518)
<223> Inventor: Endoh, Hideki; Nakano, Ryosuke; Kurosaki, Eiji; Kato, Miyuki; Yokota, Hiroyuki; Inabe, Kazunori

<400> 1
```
atg ggt gaa act ctg gga gat tct cct att gac cca gaa agc gat tcc        48
Met Gly Glu Thr Leu Gly Asp Ser Pro Ile Asp Pro Glu Ser Asp Ser
1               5                   10                  15

ttc act gat aca ctg tct gca aac ata tca caa gaa atg acc atg gtt        96
```

Phe Thr Asp Thr Leu Ser Ala Asn Ile Ser Gln Glu Met Thr Met Val
20 25 30

gac aca gag atg cca ttc tgg ccc acc aac ttt ggg atc agc tcc gtg 144
Asp Thr Glu Met Pro Phe Trp Pro Thr Asn Phe Gly Ile Ser Ser Val
35 40 45

gat ctc tcc gta atg gaa gac cac tcc cac tcc ttt gat atc aag ccc 192
Asp Leu Ser Val Met Glu Asp His Ser His Ser Phe Asp Ile Lys Pro
50 55 60

ttc act act gtt gac ttc tcc agc att tct act cca cat tac gaa gac 240
Phe Thr Thr Val Asp Phe Ser Ser Ile Ser Thr Pro His Tyr Glu Asp
65 70 75 80

att cca ttc aca aga aca gat cca gtg gtt gca gat tac aag tat gac 288
Ile Pro Phe Thr Arg Thr Asp Pro Val Val Ala Asp Tyr Lys Tyr Asp
85 90 95

ctg aaa ctt caa gag tac caa agt gca atc aaa gtg gag cct gca tct 336
Leu Lys Leu Gln Glu Tyr Gln Ser Ala Ile Lys Val Glu Pro Ala Ser
100 105 110

cca cct tat tat tct gag aag act cag ctc tac aat aag cct cat gaa 384
Pro Pro Tyr Tyr Ser Glu Lys Thr Gln Leu Tyr Asn Lys Pro His Glu
115 120 125

gag cct tcc aac tcc ctc atg gca att gaa tgt cgt gtc tgt gga gat 432
Glu Pro Ser Asn Ser Leu Met Ala Ile Glu Cys Arg Val Cys Gly Asp
130 135 140

aaa gct tct gga ttt cac tat gga gtt cat gct tgt gaa gga tgc aag 480
Lys Ala Ser Gly Phe His Tyr Gly Val His Ala Cys Glu Gly Cys Lys
145 150 155 160

ggt ttc ttc cgg aga aca atc aga ttg aag ctt atc tat gac aga tgt 528

```
Gly Phe Phe Arg Arg Thr Ile Arg Leu Lys Leu Ile Tyr Asp Arg Cys
                165                 170                 175

gat ctt aac tgt cgg atc cac aaa aaa agt aga aat aaa tgt cag tac      576
Asp Leu Asn Cys Arg Ile His Lys Lys Ser Arg Asn Lys Cys Gln Tyr
            180                 185                 190

tgt cgg ttt cag aaa tgc ctt gca gtg ggg atg tct cat aat gcc atc      624
Cys Arg Phe Gln Lys Cys Leu Ala Val Gly Met Ser His Asn Ala Ile
        195                 200                 205

agg ttt ggg cgg atg cca cag gcc gag aag gag aag ctg ttg gcg gag      672
Arg Phe Gly Arg Met Pro Gln Ala Glu Lys Glu Lys Leu Leu Ala Glu
    210                 215                 220

atc tcc agt gat atc gac cag ctg aat cca gag tcc gct gac ctc cgg      720
Ile Ser Ser Asp Ile Asp Gln Leu Asn Pro Glu Ser Ala Asp Leu Arg
225                 230                 235                 240

gcc ctg gca aaa cat ttg tat gac tca tac ata aag tcc ttc ccg ctg      768
Ala Leu Ala Lys His Leu Tyr Asp Ser Tyr Ile Lys Ser Phe Pro Leu
                245                 250                 255

acc aaa gca aag gcg agg gcg atc ttg aca gga aag aca aca gac aaa      816
Thr Lys Ala Lys Ala Arg Ala Ile Leu Thr Gly Lys Thr Thr Asp Lys
            260                 265                 270

tca cca ttc gtt atc tat gac atg aat tcc tta atg atg gga gaa gat      864
Ser Pro Phe Val Ile Tyr Asp Met Asn Ser Leu Met Met Gly Glu Asp
        275                 280                 285

aaa atc aag ttc aaa cac atc acc ccc ctg cag gag cag agc aaa gag      912
Lys Ile Lys Phe Lys His Ile Thr Pro Leu Gln Glu Gln Ser Lys Glu
    290                 295                 300

gtg gcc atc cgc atc ttt cag ggc tgc cag ttt cgc tcc gtg gag gct      960
```

```
Val Ala Ile Arg Ile Phe Gln Gly Cys Gln Phe Arg Ser Val Glu Ala
305                 310                 315                 320

gtg cag gag atc aca gag tat gcc aaa agc att cct ggt ttt gta aat      1008
Val Gln Glu Ile Thr Glu Tyr Ala Lys Ser Ile Pro Gly Phe Val Asn
                325                 330                 335

ctt gac ttg aac gac caa gta act ctc ctc aaa tat gga gtc cac gag      1056
Leu Asp Leu Asn Asp Gln Val Thr Leu Leu Lys Tyr Gly Val His Glu
            340                 345                 350

atc att tac aca atg ctg gcc tcc ttg atg aat aaa gat ggg gtt ctc      1104
Ile Ile Tyr Thr Met Leu Ala Ser Leu Met Asn Lys Asp Gly Val Leu
        355                 360                 365

ata tcc gag ggc caa ggc ttc atg aca agg gag ttt cta aag agc ctg      1152
Ile Ser Glu Gly Gln Gly Phe Met Thr Arg Glu Phe Leu Lys Ser Leu
    370                 375                 380

cga aag cct ttt ggt gac ttt atg gag ccc aag ttt gag ttt gct gtg      1200
Arg Lys Pro Phe Gly Asp Phe Met Glu Pro Lys Phe Glu Phe Ala Val
385                 390                 395                 400

aag ttc aat gca ctg gaa tta gat gac agc gac ttg gca ata ttt att      1248
Lys Phe Asn Ala Leu Glu Leu Asp Asp Ser Asp Leu Ala Ile Phe Ile
                405                 410                 415

gct gtc att att ctc agt gga gac cgc cca ggt ttg ctg aat gtg aag      1296
Ala Val Ile Ile Leu Ser Gly Asp Arg Pro Gly Leu Leu Asn Val Lys
            420                 425                 430

ccc att gaa gac att caa gac aac ctg cta caa gcc ctg gag ctc cag      1344
Pro Ile Glu Asp Ile Gln Asp Asn Leu Leu Gln Ala Leu Glu Leu Gln
        435                 440                 445

ctg aag ctg aac cac cct gag tcc tca cag ctg ttt gcc aag ctg ctc      1392
```

Leu Lys Leu Asn His Pro Glu Ser Ser Gln Leu Phe Ala Lys Leu Leu
    450                 455                 460

cag aaa atg aca gac ctc aga cag att gtc acg gaa cac gtg cag cta    1440
Gln Lys Met Thr Asp Leu Arg Gln Ile Val Thr Glu His Val Gln Leu
465                 470                 475                 480

ctg cag gtg atc aag aag acg gag aca gac atg agt ctt cac ccg ctc    1488
Leu Gln Val Ile Lys Lys Thr Glu Thr Asp Met Ser Leu His Pro Leu
                485                 490                 495

ctg cag gag atc tac aag gac ttg tac tag    1518
Leu Gln Glu Ile Tyr Lys Asp Leu Tyr
            500                 505

<210> 2
<211> 505
<212> PRT
<213> Homo sapiens

<400> 2

Met Gly Glu Thr Leu Gly Asp Ser Pro Ile Asp Pro Glu Ser Asp Ser
1               5                   10                  15

Phe Thr Asp Thr Leu Ser Ala Asn Ile Ser Gln Glu Met Thr Met Val
            20                  25                  30

Asp Thr Glu Met Pro Phe Trp Pro Thr Asn Phe Gly Ile Ser Ser Val
        35                  40                  45

Asp Leu Ser Val Met Glu Asp His Ser His Ser Phe Asp Ile Lys Pro

50 55 60

Phe Thr Thr Val Asp Phe Ser Ser Ile Ser Thr Pro His Tyr Glu Asp
65 70 75 80

Ile Pro Phe Thr Arg Thr Asp Pro Val Val Ala Asp Tyr Lys Tyr Asp
85 90 95

Leu Lys Leu Gln Glu Tyr Gln Ser Ala Ile Lys Val Glu Pro Ala Ser
100 105 110

Pro Pro Tyr Tyr Ser Glu Lys Thr Gln Leu Tyr Asn Lys Pro His Glu
115 120 125

Glu Pro Ser Asn Ser Leu Met Ala Ile Glu Cys Arg Val Cys Gly Asp
130 135 140

Lys Ala Ser Gly Phe His Tyr Gly Val His Ala Cys Glu Gly Cys Lys
145 150 155 160

Gly Phe Phe Arg Arg Thr Ile Arg Leu Lys Leu Ile Tyr Asp Arg Cys
165 170 175

Asp Leu Asn Cys Arg Ile His Lys Lys Ser Arg Asn Lys Cys Gln Tyr
180 185 190

Cys Arg Phe Gln Lys Cys Leu Ala Val Gly Met Ser His Asn Ala Ile

195 200 205

Arg Phe Gly Arg Met Pro Gln Ala Glu Lys Glu Lys Leu Leu Ala Glu
210 215 220

Ile Ser Ser Asp Ile Asp Gln Leu Asn Pro Glu Ser Ala Asp Leu Arg
225 230 235 240

Ala Leu Ala Lys His Leu Tyr Asp Ser Tyr Ile Lys Ser Phe Pro Leu
245 250 255

Thr Lys Ala Lys Ala Arg Ala Ile Leu Thr Gly Lys Thr Thr Asp Lys
260 265 270

Ser Pro Phe Val Ile Tyr Asp Met Asn Ser Leu Met Met Gly Glu Asp
275 280 285

Lys Ile Lys Phe Lys His Ile Thr Pro Leu Gln Glu Gln Ser Lys Glu
290 295 300

Val Ala Ile Arg Ile Phe Gln Gly Cys Gln Phe Arg Ser Val Glu Ala
305 310 315 320

Val Gln Glu Ile Thr Glu Tyr Ala Lys Ser Ile Pro Gly Phe Val Asn
325 330 335

Leu Asp Leu Asn Asp Gln Val Thr Leu Leu Lys Tyr Gly Val His Glu

340 345 350

Ile Ile Tyr Thr Met Leu Ala Ser Leu Met Asn Lys Asp Gly Val Leu
355 360 365

Ile Ser Glu Gly Gln Gly Phe Met Thr Arg Glu Phe Leu Lys Ser Leu
370 375 380

Arg Lys Pro Phe Gly Asp Phe Met Glu Pro Lys Phe Glu Phe Ala Val
385 390 395 400

Lys Phe Asn Ala Leu Glu Leu Asp Asp Ser Asp Leu Ala Ile Phe Ile
405 410 415

Ala Val Ile Ile Leu Ser Gly Asp Arg Pro Gly Leu Leu Asn Val Lys
420 425 430

Pro Ile Glu Asp Ile Gln Asp Asn Leu Leu Gln Ala Leu Glu Leu Gln
435 440 445

Leu Lys Leu Asn His Pro Glu Ser Ser Gln Leu Phe Ala Lys Leu Leu
450 455 460

Gln Lys Met Thr Asp Leu Arg Gln Ile Val Thr Glu His Val Gln Leu
465 470 475 480

Leu Gln Val Ile Lys Lys Thr Glu Thr Asp Met Ser Leu His Pro Leu

485 490 495

Leu Gln Glu Ile Tyr Lys Asp Leu Tyr
500 505

<210> 3
<211> 987
<212> DNA
<213> Homo sapiens

<220>
<221> CDS
<222> (1)..(987)
<223>

<400> 3
atg gcg gcg ggg ata gtg gct tct cgc aga ctc cgc gac cta ctg acc 48
Met Ala Ala Gly Ile Val Ala Ser Arg Arg Leu Arg Asp Leu Leu Thr
1 5 10 15

cgg cga ctg aca ggc tcc aac tac ccg gga ctc agt att agc ctt cgc 96
Arg Arg Leu Thr Gly Ser Asn Tyr Pro Gly Leu Ser Ile Ser Leu Arg
20 25 30

ctc act ggc tcc tct gca caa gag gcg gct tcc gga gta gcc ctc ggt 144
Leu Thr Gly Ser Ser Ala Gln Glu Ala Ala Ser Gly Val Ala Leu Gly
35 40 45

gaa gcc cca gac cac agc tat gag tcc ctt cgt gtg acg tct gcg cag 192
Glu Ala Pro Asp His Ser Tyr Glu Ser Leu Arg Val Thr Ser Ala Gln
50 55 60

aaa cat gtt ctg cat gtc cag ctc aac cgg ccc aac aag agg aat gcc 240
Lys His Val Leu His Val Gln Leu Asn Arg Pro Asn Lys Arg Asn Ala

65 70 75 80

atg aac aag gtc ttc tgg aga gag atg gta gag tgc ttc aac aag att 288
Met Asn Lys Val Phe Trp Arg Glu Met Val Glu Cys Phe Asn Lys Ile
85 90 95

tcg aga gac gct gac tgt cgg gcg gtg gtg atc tct ggt gca gga aaa 336
Ser Arg Asp Ala Asp Cys Arg Ala Val Val Ile Ser Gly Ala Gly Lys
100 105 110

atg ttc act gca ggt att gac ctg atg gac atg gct tcg gac atc ctg 384
Met Phe Thr Ala Gly Ile Asp Leu Met Asp Met Ala Ser Asp Ile Leu
115 120 125

cag ccc aaa gga gat gat gtg gcc cgg atc agc tgg tac ctc cgt gac 432
Gln Pro Lys Gly Asp Asp Val Ala Arg Ile Ser Trp Tyr Leu Arg Asp
130 135 140

atc atc act cga tac cag gag acc ttc aac gtc atc gag agg tgc ccc 480
Ile Ile Thr Arg Tyr Gln Glu Thr Phe Asn Val Ile Glu Arg Cys Pro
145 150 155 160

aag ccc gtg att gct gcc gtc cat ggg ggc tgc att ggc gga ggt gtg 528
Lys Pro Val Ile Ala Ala Val His Gly Gly Cys Ile Gly Gly Gly Val
165 170 175

gac ctt gtc acc gcc tgt gac atc cgg tac tgt gcc cag gat gct ttc 576
Asp Leu Val Thr Ala Cys Asp Ile Arg Tyr Cys Ala Gln Asp Ala Phe
180 185 190

ttc cag gtg aag gag gtg gac gtg ggt ttg gct gcc gat gta gga aca 624
Phe Gln Val Lys Glu Val Asp Val Gly Leu Ala Ala Asp Val Gly Thr
195 200 205

ctg cag cgc ctg ccc aag gtc atc ggg aac cag agc ctg gtc aac gag 672
Leu Gln Arg Leu Pro Lys Val Ile Gly Asn Gln Ser Leu Val Asn Glu

210 215 220

ctg gcc ttc acc gcc cgc aag atg atg gct gac gag gcc ctg ggc agt 720
Leu Ala Phe Thr Ala Arg Lys Met Met Ala Asp Glu Ala Leu Gly Ser
225 230 235 240

ggg ctg gtc agc cgg gtg ttc cca gac aaa gag gtc atg ctg gat gct 768
Gly Leu Val Ser Arg Val Phe Pro Asp Lys Glu Val Met Leu Asp Ala
245 250 255

gcc tta gcg ctg gcg gcc gag att tcc agc aag agc ccc gtg gcg gtg 816
Ala Leu Ala Leu Ala Ala Glu Ile Ser Ser Lys Ser Pro Val Ala Val
260 265 270

cag agc acc aag gtc aac ctg ctg tat tcc cgc gac cat tcg gtg gcc 864
Gln Ser Thr Lys Val Asn Leu Leu Tyr Ser Arg Asp His Ser Val Ala
275 280 285

gag agc ctc aac tac gtg gcg tcc tgg aac atg agc atg ctg cag acc 912
Glu Ser Leu Asn Tyr Val Ala Ser Trp Asn Met Ser Met Leu Gln Thr
290 295 300

caa gac ctc gtg aag tcg gtc cag gcc acg act gag aac aag gaa ctg 960
Gln Asp Leu Val Lys Ser Val Gln Ala Thr Thr Glu Asn Lys Glu Leu
305 310 315 320

aaa acc gtc acc ttc tcc aag ctc tga 987
Lys Thr Val Thr Phe Ser Lys Leu
325

<210> 4
<211> 328
<212> PRT
<213> Homo sapiens

<400> 4

Met Ala Ala Gly Ile Val Ala Ser Arg Arg Leu Arg Asp Leu Leu Thr
1 5 10 15

Arg Arg Leu Thr Gly Ser Asn Tyr Pro Gly Leu Ser Ile Ser Leu Arg
20 25 30

Leu Thr Gly Ser Ser Ala Gln Glu Ala Ala Ser Gly Val Ala Leu Gly
35 40 45

Glu Ala Pro Asp His Ser Tyr Glu Ser Leu Arg Val Thr Ser Ala Gln
50 55 60

Lys His Val Leu His Val Gln Leu Asn Arg Pro Asn Lys Arg Asn Ala
65 70 75 80

Met Asn Lys Val Phe Trp Arg Glu Met Val Glu Cys Phe Asn Lys Ile
85 90 95

Ser Arg Asp Ala Asp Cys Arg Ala Val Val Ile Ser Gly Ala Gly Lys
100 105 110

Met Phe Thr Ala Gly Ile Asp Leu Met Asp Met Ala Ser Asp Ile Leu
115 120 125

Gln Pro Lys Gly Asp Asp Val Ala Arg Ile Ser Trp Tyr Leu Arg Asp
130 135 140

Ile Ile Thr Arg Tyr Gln Glu Thr Phe Asn Val Ile Glu Arg Cys Pro
145                 150                 155                 160

Lys Pro Val Ile Ala Ala Val His Gly Gly Cys Ile Gly Gly Gly Val
                165                 170                 175

Asp Leu Val Thr Ala Cys Asp Ile Arg Tyr Cys Ala Gln Asp Ala Phe
            180                 185                 190

Phe Gln Val Lys Glu Val Asp Val Gly Leu Ala Ala Asp Val Gly Thr
        195                 200                 205

Leu Gln Arg Leu Pro Lys Val Ile Gly Asn Gln Ser Leu Val Asn Glu
    210                 215                 220

Leu Ala Phe Thr Ala Arg Lys Met Met Ala Asp Glu Ala Leu Gly Ser
225                 230                 235                 240

Gly Leu Val Ser Arg Val Phe Pro Asp Lys Glu Val Met Leu Asp Ala
                245                 250                 255

Ala Leu Ala Leu Ala Ala Glu Ile Ser Ser Lys Ser Pro Val Ala Val
            260                 265                 270

Gln Ser Thr Lys Val Asn Leu Leu Tyr Ser Arg Asp His Ser Val Ala
        275                 280                 285

Glu Ser Leu Asn Tyr Val Ala Ser Trp Asn Met Ser Met Leu Gln Thr
290 295 300

Gln Asp Leu Val Lys Ser Val Gln Ala Thr Thr Glu Asn Lys Glu Leu
305 310 315 320

Lys Thr Val Thr Phe Ser Lys Leu
325

<210> 5
<211> 1407
<212> DNA
<213> Homo sapiens

<220>
<221> CDS
<222> (1)..(1407)
<223>

<400> 5
atg gtg gac acg gaa agc cca ctc tgc ccc ctc tcc cca ctc gag gcc 48
Met Val Asp Thr Glu Ser Pro Leu Cys Pro Leu Ser Pro Leu Glu Ala
1 5 10 15

ggc gat cta gag agc ccg tta tct gaa gag ttc ctg caa gaa atg gga 96
Gly Asp Leu Glu Ser Pro Leu Ser Glu Glu Phe Leu Gln Glu Met Gly
20 25 30

aac atc caa gag att tcg caa tcc atc ggc gag gat agt tct gga agc 144
Asn Ile Gln Glu Ile Ser Gln Ser Ile Gly Glu Asp Ser Ser Gly Ser
35 40 45

ttt ggc ttt acg gaa tac cag tat tta gga agc tgt cct ggc tca gat 192
Phe Gly Phe Thr Glu Tyr Gln Tyr Leu Gly Ser Cys Pro Gly Ser Asp
50 55 60

ggc tcg gtc atc acg gac acg ctt tca cca gct tcg agc ccc tcc tcg 240
Gly Ser Val Ile Thr Asp Thr Leu Ser Pro Ala Ser Ser Pro Ser Ser
65 70 75 80

gtg act tat cct gtg gtc ccc ggc agc gtg gac gag tct ccc agt gga 288
Val Thr Tyr Pro Val Val Pro Gly Ser Val Asp Glu Ser Pro Ser Gly
85 90 95

gca ttg aac atc gaa tgt aga atc tgc ggg gac aag gcc tca ggc tat 336
Ala Leu Asn Ile Glu Cys Arg Ile Cys Gly Asp Lys Ala Ser Gly Tyr
100 105 110

cat tac gga gtc cac gcg tgt gaa ggc tgc aag ggc ttc ttt cgg cga 384
His Tyr Gly Val His Ala Cys Glu Gly Cys Lys Gly Phe Phe Arg Arg
115 120 125

acg att cga ctc aag ctg gtg tat gac aag tgc gac cgc agc tgc aag 432
Thr Ile Arg Leu Lys Leu Val Tyr Asp Lys Cys Asp Arg Ser Cys Lys
130 135 140

atc cag aaa aag aac aga aac aaa tgc cag tat tgt cga ttt cac aag 480
Ile Gln Lys Lys Asn Arg Asn Lys Cys Gln Tyr Cys Arg Phe His Lys
145 150 155 160

tgc ctt tct gtc ggg atg tca cac aac gcg att cgt ttt gga cga atg 528
Cys Leu Ser Val Gly Met Ser His Asn Ala Ile Arg Phe Gly Arg Met
165 170 175

cca aga tct gag aaa gca aaa ctg aaa gca gaa att ctt acc tgt gaa 576
Pro Arg Ser Glu Lys Ala Lys Leu Lys Ala Glu Ile Leu Thr Cys Glu
180 185 190

cat gac ata gaa gat tct gaa act gca gat ctc aaa tct ctg gcc aag 624
His Asp Ile Glu Asp Ser Glu Thr Ala Asp Leu Lys Ser Leu Ala Lys
195 200 205

aga atc tac gag gcc tac ttg aag aac ttc aac atg aac aag gtc aaa 672
Arg Ile Tyr Glu Ala Tyr Leu Lys Asn Phe Asn Met Asn Lys Val Lys
210 215 220

gcc cgg gtc atc ctc tca gga aag gcc agt aac aat cca cct ttt gtc 720
Ala Arg Val Ile Leu Ser Gly Lys Ala Ser Asn Asn Pro Pro Phe Val
225 230 235 240

ata cat gat atg gag aca ctg tgt atg gct gag aag acg ctg gtg gcc 768
Ile His Asp Met Glu Thr Leu Cys Met Ala Glu Lys Thr Leu Val Ala
245 250 255

aag ctg gtg gcc aat ggc atc cag aac aag gag gcg gag gtc cgc atc 816
Lys Leu Val Ala Asn Gly Ile Gln Asn Lys Glu Ala Glu Val Arg Ile
260 265 270

ttt cac tgc tgc cag tgc acg tca gtg gag acc gtc acg gag ctc acg 864
Phe His Cys Cys Gln Cys Thr Ser Val Glu Thr Val Thr Glu Leu Thr
275 280 285

gaa ttc gcc aag gcc atc cca ggc ttc gca aac ttg gac ctg aac gat 912
Glu Phe Ala Lys Ala Ile Pro Gly Phe Ala Asn Leu Asp Leu Asn Asp
290 295 300

caa gtg aca ttg cta aaa tac gga gtt tat gag gcc ata ttc gcc atg 960
Gln Val Thr Leu Leu Lys Tyr Gly Val Tyr Glu Ala Ile Phe Ala Met
305 310 315 320

ctg tct tct gtg atg aac aaa gac ggg atg ctg gta gcg tat gga aat 1008
Leu Ser Ser Val Met Asn Lys Asp Gly Met Leu Val Ala Tyr Gly Asn
325 330 335

ggg ttt ata act cgt gaa ttc cta aaa agc cta agg aaa ccg ttc tgt 1056
Gly Phe Ile Thr Arg Glu Phe Leu Lys Ser Leu Arg Lys Pro Phe Cys
340 345 350

gat atc atg gaa ccc aag ttt gat ttt gcc atg aag ttc aat gca ctg 1104
Asp Ile Met Glu Pro Lys Phe Asp Phe Ala Met Lys Phe Asn Ala Leu
355 360 365

gaa ctg gat gac agt gat atc tcc ctt ttt gtg gct gct atc att tgc 1152
Glu Leu Asp Asp Ser Asp Ile Ser Leu Phe Val Ala Ala Ile Ile Cys
370 375 380

tgt gga gat cgt cct ggc ctt cta aac gta gga cac att gaa aaa atg 1200
Cys Gly Asp Arg Pro Gly Leu Leu Asn Val Gly His Ile Glu Lys Met
385 390 395 400

cag gag ggt att gta cat gtg ctc aga ctc cac ctg cag agc aac cac 1248
Gln Glu Gly Ile Val His Val Leu Arg Leu His Leu Gln Ser Asn His
405 410 415

ccg gac gat atc ttt ctc ttc cca aaa ctt ctt caa aaa atg gca gac 1296
Pro Asp Asp Ile Phe Leu Phe Pro Lys Leu Leu Gln Lys Met Ala Asp
420 425 430

ctc cgg cag ctg gtg acg gag cat gcg cag ctg gtg cag atc atc aag 1344
Leu Arg Gln Leu Val Thr Glu His Ala Gln Leu Val Gln Ile Ile Lys
435 440 445

aag acg gag tcg gat gct gcg ctg cac ccg cta ctg cag gag atc tac 1392
Lys Thr Glu Ser Asp Ala Ala Leu His Pro Leu Leu Gln Glu Ile Tyr
450 455 460

agg gac atg tac tga 1407
Arg Asp Met Tyr
465

<210> 6
<211> 468
<212> PRT
<213> Homo sapiens

<400> 6

Met Val Asp Thr Glu Ser Pro Leu Cys Pro Leu Ser Pro Leu Glu Ala
1 5 10 15

Gly Asp Leu Glu Ser Pro Leu Ser Glu Glu Phe Leu Gln Glu Met Gly
20 25 30

Asn Ile Gln Glu Ile Ser Gln Ser Ile Gly Glu Asp Ser Ser Gly Ser
35 40 45

Phe Gly Phe Thr Glu Tyr Gln Tyr Leu Gly Ser Cys Pro Gly Ser Asp
50 55 60

Gly Ser Val Ile Thr Asp Thr Leu Ser Pro Ala Ser Ser Pro Ser Ser
65 70 75 80

Val Thr Tyr Pro Val Val Pro Gly Ser Val Asp Glu Ser Pro Ser Gly
85 90 95

Ala Leu Asn Ile Glu Cys Arg Ile Cys Gly Asp Lys Ala Ser Gly Tyr
100 105 110

His Tyr Gly Val His Ala Cys Glu Gly Cys Lys Gly Phe Phe Arg Arg
115 120 125

Thr Ile Arg Leu Lys Leu Val Tyr Asp Lys Cys Asp Arg Ser Cys Lys
130 135 140

Ile Gln Lys Lys Asn Arg Asn Lys Cys Gln Tyr Cys Arg Phe His Lys
145 150 155 160

Cys Leu Ser Val Gly Met Ser His Asn Ala Ile Arg Phe Gly Arg Met
165 170 175

Pro Arg Ser Glu Lys Ala Lys Leu Lys Ala Glu Ile Leu Thr Cys Glu
180 185 190

His Asp Ile Glu Asp Ser Glu Thr Ala Asp Leu Lys Ser Leu Ala Lys
195 200 205

Arg Ile Tyr Glu Ala Tyr Leu Lys Asn Phe Asn Met Asn Lys Val Lys
210 215 220

Ala Arg Val Ile Leu Ser Gly Lys Ala Ser Asn Asn Pro Pro Phe Val
225 230 235 240

Ile His Asp Met Glu Thr Leu Cys Met Ala Glu Lys Thr Leu Val Ala
245 250 255

Lys Leu Val Ala Asn Gly Ile Gln Asn Lys Glu Ala Glu Val Arg Ile
260 265 270

Phe His Cys Cys Gln Cys Thr Ser Val Glu Thr Val Thr Glu Leu Thr
275 280 285

Glu Phe Ala Lys Ala Ile Pro Gly Phe Ala Asn Leu Asp Leu Asn Asp
290 295 300

Gln Val Thr Leu Leu Lys Tyr Gly Val Tyr Glu Ala Ile Phe Ala Met
305 310 315 320

Leu Ser Ser Val Met Asn Lys Asp Gly Met Leu Val Ala Tyr Gly Asn
325 330 335

Gly Phe Ile Thr Arg Glu Phe Leu Lys Ser Leu Arg Lys Pro Phe Cys
340 345 350

Asp Ile Met Glu Pro Lys Phe Asp Phe Ala Met Lys Phe Asn Ala Leu
355 360 365

Glu Leu Asp Asp Ser Asp Ile Ser Leu Phe Val Ala Ala Ile Ile Cys
370 375 380

Cys Gly Asp Arg Pro Gly Leu Leu Asn Val Gly His Ile Glu Lys Met
385 390 395 400

Gln Glu Gly Ile Val His Val Leu Arg Leu His Leu Gln Ser Asn His
405 410 415

Pro Asp Asp Ile Phe Leu Phe Pro Lys Leu Leu Gln Lys Met Ala Asp
420 425 430

Leu Arg Gln Leu Val Thr Glu His Ala Gln Leu Val Gln Ile Ile Lys
435 440 445

Lys Thr Glu Ser Asp Ala Ala Leu His Pro Leu Leu Gln Glu Ile Tyr
450 455 460

Arg Asp Met Tyr
465

<210> 7
<211> 675
<212> DNA
<213> Homo sapiens

<220>
<221> CDS
<222> (1)..(675)
<223>

<400> 7
atg ccc gga ggt ctg ctt ctc ggg gac gtg gct ccc aac ttt gag gcc 48
Met Pro Gly Gly Leu Leu Leu Gly Asp Val Ala Pro Asn Phe Glu Ala
1 5 10 15

aat acc acc gtc ggc cgc atc cgt ttc cac gac ttt ctg gga gac tca 96
Asn Thr Thr Val Gly Arg Ile Arg Phe His Asp Phe Leu Gly Asp Ser
20 25 30

tgg ggc att ctc ttc tcc cac cct cgg gac ttt acc cca gtg tgc acc 144
Trp Gly Ile Leu Phe Ser His Pro Arg Asp Phe Thr Pro Val Cys Thr
35 40 45

aca gag ctt ggc aga gct gca aag ctg gca cca gaa ttt gcc aag agg 192
Thr Glu Leu Gly Arg Ala Ala Lys Leu Ala Pro Glu Phe Ala Lys Arg
50 55 60

aat gtt aag ttg att gcc ctt tca ata gac agt gtt gag gac cat ctt 240
Asn Val Lys Leu Ile Ala Leu Ser Ile Asp Ser Val Glu Asp His Leu
65 70 75 80

gcc tgg agc aag gat atc aat gct tac aat tgt gaa gag ccc aca gaa 288
Ala Trp Ser Lys Asp Ile Asn Ala Tyr Asn Cys Glu Glu Pro Thr Glu
85 90 95

aag tta cct ttt ccc atc atc gat gat agg aat cgg gag ctt gcc atc 336
Lys Leu Pro Phe Pro Ile Ile Asp Asp Arg Asn Arg Glu Leu Ala Ile
100 105 110

ctg ttg ggc atg ctg gat cca gca gag aag gat gaa aag ggc atg cct 384
Leu Leu Gly Met Leu Asp Pro Ala Glu Lys Asp Glu Lys Gly Met Pro
115 120 125

gtg aca gct cgt gtg gtg ttt gtt ttt ggt cct gat aag aag ctg aag 432
Val Thr Ala Arg Val Val Phe Val Phe Gly Pro Asp Lys Lys Leu Lys
130 135 140

ctg tct atc ctc tac cca gct acc act ggc agg aac ttt gat gag att 480
Leu Ser Ile Leu Tyr Pro Ala Thr Thr Gly Arg Asn Phe Asp Glu Ile
145 150 155 160

ctc agg gta gtc atc tct ctc cag ctg aca gca gaa aaa agg gtt gcc 528
Leu Arg Val Val Ile Ser Leu Gln Leu Thr Ala Glu Lys Arg Val Ala
165 170 175

acc cca gtt gat tgg aag gat ggg gat agt gtg atg gtc ctt cca acc 576
Thr Pro Val Asp Trp Lys Asp Gly Asp Ser Val Met Val Leu Pro Thr
180 185 190

atc cct gaa gaa gaa gcc aaa aaa ctt ttc ccg aaa gga gtc ttc acc 624
Ile Pro Glu Glu Glu Ala Lys Lys Leu Phe Pro Lys Gly Val Phe Thr
195 200 205

aaa gag ctc cca tct ggc aag aaa tac ctc cgc tac aca ccc cag cct 672
Lys Glu Leu Pro Ser Gly Lys Lys Tyr Leu Arg Tyr Thr Pro Gln Pro
210 215 220

taa 675

<210> 8
<211> 224
<212> PRT
<213> Homo sapiens

<400> 8

Met Pro Gly Gly Leu Leu Leu Gly Asp Val Ala Pro Asn Phe Glu Ala
1 5 10 15

Asn Thr Thr Val Gly Arg Ile Arg Phe His Asp Phe Leu Gly Asp Ser
20 25 30

Trp Gly Ile Leu Phe Ser His Pro Arg Asp Phe Thr Pro Val Cys Thr
35 40 45

Thr Glu Leu Gly Arg Ala Ala Lys Leu Ala Pro Glu Phe Ala Lys Arg
50 55 60

Asn Val Lys Leu Ile Ala Leu Ser Ile Asp Ser Val Glu Asp His Leu
65 70 75 80

Ala Trp Ser Lys Asp Ile Asn Ala Tyr Asn Cys Glu Glu Pro Thr Glu
85 90 95

Lys Leu Pro Phe Pro Ile Ile Asp Asp Arg Asn Arg Glu Leu Ala Ile
100 105 110

Leu Leu Gly Met Leu Asp Pro Ala Glu Lys Asp Glu Lys Gly Met Pro
115 120 125

Val Thr Ala Arg Val Val Phe Val Phe Gly Pro Asp Lys Lys Leu Lys
130 135 140

Leu Ser Ile Leu Tyr Pro Ala Thr Thr Gly Arg Asn Phe Asp Glu Ile
145 150 155 160

Leu Arg Val Val Ile Ser Leu Gln Leu Thr Ala Glu Lys Arg Val Ala
165 170 175

Thr Pro Val Asp Trp Lys Asp Gly Asp Ser Val Met Val Leu Pro Thr
180 185 190

Ile Pro Glu Glu Glu Ala Lys Lys Leu Phe Pro Lys Gly Val Phe Thr
195 200 205

Lys Glu Leu Pro Ser Gly Lys Lys Tyr Leu Arg Tyr Thr Pro Gln Pro
210 215 220

<210> 9
<211> 64
<212> DNA
<213> Artificial

<220>
<223> Description of Artificial Sequence: an artificially synthesized primer sequence

<400> 9
agagagtagt aacaaaggtc aaagacagtt gactgtatcg ggtacctctc ataatgccat 60

cagg 64

<210> 10
<211> 65
<212> DNA
<213> Artificial

<220>
<223> Description of Artificial Sequence: an artificially synthesized primer sequence

<400> 10
tggagacttg accaaacctc tggcgaagaa gtccaaagct cccgggctag tacaagtcct 60

tgtag 65

<210> 11
<211> 22
<212> DNA
<213> Artificial

<220>
<223> The sequence of the 5183th(5') to 5162th(3') bases in cloning vector pACT2 (GenBank U29899)

<400> 11
cgcgtttgga atcactacag gg 22

<210> 12
<211> 18
<212> DNA
<213> Homo sapiens

<400> 12
atggcggcgg ggatagtg 18

<210> 13
<211> 22
<212> DNA
<213> Artificial

<220>
<223> Description of Artificial Sequence: an artificially synthesized primer sequence

<400> 13

gtagagcttg gagaaggtga cg 22

<210> 14
<211> 19
<212> DNA
<213> Homo sapiens

<400> 14
atgccccggag gtctgcttc 19

<210> 15
<211> 18
<212> DNA
<213> Homo sapiens

<400> 15
aggctggggt gtgtagcg 18

<210> 16
<211> 900
<212> DNA
<213> Homo sapiens

<220>
<221> CDS
<222> (1)..(900)
<223>

<400> 16
atg gct gac cac aac cct gac agc gac tcc acg ccg cgc acg ctg ctg 48
Met Ala Asp His Asn Pro Asp Ser Asp Ser Thr Pro Arg Thr Leu Leu
1 5 10 15

cga cgc gtg ctg gat aca gcg gac ccg cgc acc ccg cgg cga ccc cgg 96
Arg Arg Val Leu Asp Thr Ala Asp Pro Arg Thr Pro Arg Arg Pro Arg
20 25 30

agt gct cgg gct gga gcc cgg aga gcc ctg ctt gaa acg gct tcc ccc 144
Ser Ala Arg Ala Gly Ala Arg Arg Ala Leu Leu Glu Thr Ala Ser Pro
35 40 45

agg aag ttg agt ggc caa aca agg acg ata gcc aga ggg cgt tcc cat 192
Arg Lys Leu Ser Gly Gln Thr Arg Thr Ile Ala Arg Gly Arg Ser His
50 55 60

gga gcc agg tct gtt ggc aga tcg gcc cat att cag gcc agt ggg cac 240
Gly Ala Arg Ser Val Gly Arg Ser Ala His Ile Gln Ala Ser Gly His
65 70 75 80

ttg gag gaa cag aca cct cgg acg ctg ctg aag aac atc cta cta act 288
Leu Glu Glu Gln Thr Pro Arg Thr Leu Leu Lys Asn Ile Leu Leu Thr
85 90 95

gcc cca gaa tct tcc atc ctg atg cct gag tcg gta gtg aag cca gtg 336
Ala Pro Glu Ser Ser Ile Leu Met Pro Glu Ser Val Val Lys Pro Val
100 105 110

cca gca ccg cag gcg gtc caa ccc tcc aga caa gag agc agt tgc ggc 384
Pro Ala Pro Gln Ala Val Gln Pro Ser Arg Gln Glu Ser Ser Cys Gly
115 120 125

agc ctg gag ctg caa ctt cct gag ctc gag ccc ccc aca acc ctg gct 432
Ser Leu Glu Leu Gln Leu Pro Glu Leu Glu Pro Pro Thr Thr Leu Ala
130 135 140

cca ggt ctg ctg gcc cct ggc agg agg aaa cag agg ctg aga ctg tca 480
Pro Gly Leu Leu Ala Pro Gly Arg Arg Lys Gln Arg Leu Arg Leu Ser
145 150 155 160

gtg ttt cag cag gga gtg gac cag ggg ctg tct ctc tcc caa gag cct 528
Val Phe Gln Gln Gly Val Asp Gln Gly Leu Ser Leu Ser Gln Glu Pro
165 170 175

caa ggg aat gct gat gcc tct tcc ctc acc aga tcc ctc aac ctg acc 576
Gln Gly Asn Ala Asp Ala Ser Ser Leu Thr Arg Ser Leu Asn Leu Thr
180 185 190

ttt gcc aca cct ctt cag cca cag tca gtg cag agg cct ggc ttg gcc 624
Phe Ala Thr Pro Leu Gln Pro Gln Ser Val Gln Arg Pro Gly Leu Ala
195 200 205

cgc aga cct cca gcc cgc cga gct gta gac gtg ggt gcc ttt ttg cgg 672
Arg Arg Pro Pro Ala Arg Arg Ala Val Asp Val Gly Ala Phe Leu Arg
210 215 220

gat ctg cga gat act tcc ctg gct cct cca aac att gtg ttg gag gac 720
Asp Leu Arg Asp Thr Ser Leu Ala Pro Pro Asn Ile Val Leu Glu Asp
225 230 235 240

acc cag ccg ttc tct cag ccc atg gtt ggc tcc ccc aac gtg tat cac 768
Thr Gln Pro Phe Ser Gln Pro Met Val Gly Ser Pro Asn Val Tyr His
245 250 255

tcc ctg ccc tgc acg cct cac act ggg gct gaa gac gct gag cag gct 816
Ser Leu Pro Cys Thr Pro His Thr Gly Ala Glu Asp Ala Glu Gln Ala
260 265 270

gcc ggt cgc aag aca cag agc agt ggg cct ggg ctg cag aag aat agt 864
Ala Gly Arg Lys Thr Gln Ser Ser Gly Pro Gly Leu Gln Lys Asn Ser
275 280 285

gag tgt gtg gca ctg gtg gcc tgg agc caa att tag 900
Glu Cys Val Ala Leu Val Ala Trp Ser Gln Ile
290 295

<210> 17
<211> 299
<212> PRT
<213> Homo sapiens

<400> 17

Met Ala Asp His Asn Pro Asp Ser Asp Ser Thr Pro Arg Thr Leu Leu
1 5 10 15

Arg Arg Val Leu Asp Thr Ala Asp Pro Arg Thr Pro Arg Arg Pro Arg
20 25 30

Ser Ala Arg Ala Gly Ala Arg Arg Ala Leu Leu Glu Thr Ala Ser Pro
35 40 45

Arg Lys Leu Ser Gly Gln Thr Arg Thr Ile Ala Arg Gly Arg Ser His
50 55 60

Gly Ala Arg Ser Val Gly Arg Ser Ala His Ile Gln Ala Ser Gly His
65 70 75 80

Leu Glu Glu Gln Thr Pro Arg Thr Leu Leu Lys Asn Ile Leu Leu Thr
85 90 95

Ala Pro Glu Ser Ser Ile Leu Met Pro Glu Ser Val Val Lys Pro Val
100 105 110

Pro Ala Pro Gln Ala Val Gln Pro Ser Arg Gln Glu Ser Ser Cys Gly
115 120 125

Ser Leu Glu Leu Gln Leu Pro Glu Leu Glu Pro Pro Thr Thr Leu Ala
130 135 140

Pro Gly Leu Leu Ala Pro Gly Arg Arg Lys Gln Arg Leu Arg Leu Ser
145 150 155 160

Val Phe Gln Gln Gly Val Asp Gln Gly Leu Ser Leu Ser Gln Glu Pro
165 170 175

Gln Gly Asn Ala Asp Ala Ser Ser Leu Thr Arg Ser Leu Asn Leu Thr
180 185 190

Phe Ala Thr Pro Leu Gln Pro Gln Ser Val Gln Arg Pro Gly Leu Ala
195 200 205

Arg Arg Pro Pro Ala Arg Arg Ala Val Asp Val Gly Ala Phe Leu Arg
210 215 220

Asp Leu Arg Asp Thr Ser Leu Ala Pro Pro Asn Ile Val Leu Glu Asp
225 230 235 240

Thr Gln Pro Phe Ser Gln Pro Met Val Gly Ser Pro Asn Val Tyr His
245 250 255

Ser Leu Pro Cys Thr Pro His Thr Gly Ala Glu Asp Ala Glu Gln Ala
260 265 270

Ala Gly Arg Lys Thr Gln Ser Ser Gly Pro Gly Leu Gln Lys Asn Ser
275 280 285

Glu Cys Val Ala Leu Val Ala Trp Ser Gln Ile
290 295

<210> 18
<211> 17
<212> DNA
<213> Homo sapiens

<400> 18
atggctgacc acaaccc 17

<210> 19
<211> 16
<212> DNA
<213> Homo sapiens

<400> 19
aatttggctc caggcc 16

<210> 20
<211> 24
<212> DNA
<213> Mus sp.

<400> 20

gccaaacaaa gacaagtgct agga 24

<210> 21
<211> 21
<212> DNA
<213> Mus sp.

<400> 21
cgaccttgaa cacgagtcga t 21

<210> 22
<211> 20
<212> DNA
<213> Mus sp.

<400> 22
aaagtggaga ttgttgccat 20

<210> 23
<211> 19
<212> DNA
<213> Mus sp.

<400> 23
ttgactgtgc cgttgaatt 19

<210> 24
<211> 26
<212> DNA
<213> Artificial

<220>

<223> Description of Artificial Sequence: an artificially synthesized primer sequence

<400> 24
ttagatctgt gtcctagaaa gtaccc 26

<210> 25
<211> 28
<212> DNA
<213> Artificial

<220>
<223> Description of Artificial Sequence: an artificially synthesized primer sequence

<400> 25
ttaagcttct acgaaggatc cggggatg 28

<210> 26
<211> 1870
<212> DNA
<213> Homo sapiens

<220>
<221> promoter
<222> (1)..(1870)
<223>

<400> 26
atctgtgtcc tagaaagtac ccacgcagac aatctacagg gtcctgaaat aactgcttct 60

agttctagat ttcacctcat tgtcctgtgg gtagtccctc cttattgccc agctccactg 120

gcaaagttgg cctctcctga gcctcttgga tgaacctgat ttccatgtcc tcatgggcca 180

ggatatggac taggaaagtt acatccagag agaaggcagg gcttggtaaa atctgagcag 240

cattcttgcc tattcttcct gaccagggag aataactagg cagagcaatt catatgcaag 300

aaaacttcta ggtcaagagt acttggaaga agtgaccaga aatgaacaga aaaacattac 360

ctgggaaaat attcaaggag gaagaaaaaa atatgcaggg tcagtggctt tcttcacctt 420

gggtctcaat cagcccttct aagagacctg atattctgct tgctcctgag gccagagctg 480

cagagattga gctgctgctg cttttttttt tttctttttc tttttttttg agacagagtc 540

ttgctctgtc gcccaggctg gagtgcagtg gcatgatctc tgcccactgc aacctctgcc 600

tcccggattc aagcgattct cctgcctcag cctcccaagt agctgggatt acaggtgcac 660

gccaccacac ccagcttttt ttattttgga gacagagtct tgccctgtca cccaggctgg 720

agtacagtgg catgatctca gttcactgcg acctccacct cccgggttca agcaattctc 780

ctgcctcagt ctcctgagta gctaggatta cagaagtgca cctccacgtt cagctaattt 840

ttgtattttt agtagagatg cgcttttgcc atgttggcca tgctagtctg gaaccccgga 900

cctcaggtga tccgctggcc ttggcctccc aaagtgctgg gattgcaggc gtgagccatc 960

gcgccaggcc tgagctactc ctttagtctc tggaaagact gcggctcaga gaaatcaacg 1020

ctttacatgc catctctccc ctagtcccaa ggtcttctct ggactggttc cttaatttac 1080

catctctcaa tcagaagcgc ctttacaaag gatatcagga tagtattttt tggtcaaaga 1140

taactcttcc cccagaactc agtagggtct ctgatggcat cagtaagggc aatttcataa 1200

agcaaccagg ttcctgcctg tttacaggca gctccagttg ttgggaagtg agcaagcaag 1260

tgaggggaag ccagactttc tggagcttcc agccctagtt ctgccaccca ggcttcggga 1320

gggccccaca aagtctgaaa tctacgtgcc ccacccccca aagccccttc cagcccaggt 1380

tctggtttag cactaggctc aagaacctca ggcctgaatt ctacttcccg tgcattaagt 1440

cccaccttaa catcccattc ttatgaagtc atcctgtcct gcggggcaat tctgtgttgc 1500

tgctggggcc tggtcactga tagagaaggg cgtgggctgg gcctacccgg caggagccgc 1560

ctcaccccat caggcccaga tcctctaaga caagacaggg ataaagtctc ccgtggaatc 1620

ccctcccgcc tcatattccc tgaccctgcc ctccctgcga gaactccagt ccgggctcca 1680

caaagggcgc ggtaagaaac gtacacttcc gaggggcaga caaggagggg gcgttcacct 1740

acaccccggc cgggctatgg tcgggaacag ggtgaggggc ccaagccccg gctgcggcag 1800

gcgggcgccc ttacccatgc tgactctgga gcgggccggg tggcgctgcc catccccgga 1860

tccttcgtag 1870

<210> 27
<211> 32
<212> DNA
<213> Artificial

<220>
<223> Description of Artificial Sequence: an artificially synthesized primer sequence

<400> 27
ggccgcgact acaaggacga tgacgataag tg 32

<210> 28
<211> 34
<212> DNA
<213> Artificial

<220>
<223> Description of Artificial Sequence: an artificially synthesized primer sequence

<400> 28
ctagatcact tatcgtcatc gtccttgtag tcgc 34

## Description

Technical Field

The present invention relates to a method for screening a protein interactive with PPAR in a ligand-dependent manner, and a method for screening a drug ameliorating insulin resistance, utilizing the protein.

Background of the Invention

It has been shown that thiazolidine derivative, which is recognized to have an effect as an insulin sensitizing agent, function as an agonist of the peroxisome proliferator activated receptor gamma (PPARγ) (see non-patent reference 1). Since the affinity of thiazolidine derivatives for PPARγ is in a correlation with hypoglycemic effect in human body, it is believed that the effect of this group of compound for ameliorating insulin resistance is caused via PPARγ (see non-patent reference 2). Therefore, it has been considered that a method for detecting PPARγ agonists is an effective tool to select drugs for insulin resistant diabetes mellitus

Diabetes mellitus is caused by insufficient action of insulin secreted from pancreas and mainly includes two types. So-called type 1 diabetes mellitus occurs due to the damage of pancreatic β cells, so insulin is essential for the treatment. Meanwhile, type 2 diabetes mellitus (non-insulin-dependent-diabetes mellitus) occurs due to daily habits physically burdensome such as overeating, lack of exercise and stress in addition to genetic factors. Type 2 diabetes mellitus occupies most of Japanese patients with diabetes mellitus, while the number of the patients with type 1 is very small. In patients with type 2 diabetes mellitus, insulin resistance emerges, so the promotion of glucose metabolism via insulin hardly occurs. Therefore, research works have been kept on going not only about agents for simply lowering blood glucose level as anti-diabetic agent but also about the therapeutic treatment of the subject with type 2 diabetes mellitus for promoting glucose metabolism by insulin sensitizing.

It is known that PPAR belongs to the nuclear receptor superfamily that binds to a response element upstream of a target gene and induces its transcription in a ligand-dependent manner (see non-patent reference 3).

It is known that PPAR includes three subtypes, which are referred to as PPARα, PPARβ, and PPARγ (see non-patent references 4 and 5). Further, various compounds activating these PPARs, which lower blood glucose or lipid, have been reported. For example, it is known that thiazolidine derivatives as anti-diabetic agents are PPARγ ligands and significantly lower serum triglyceride level (see non-patent references 6 to 9). Alternatively, fibrate having been traditionally used as hypolipidemic agents are known to act as a ligand for PPARα. Clinically, it is observed that the fibrate strongly lowers serum triacylglycerol level (see non-patent references 10 and 11).

It has been reported that PPARγ agonists terminate cell growth and promote cell differentiation (see non-patent reference 12). PPARγ expression is observed particularly in fat tissues (see non-patent references 13 and 14), and no induction of adipocyte differentiation occurs in PPARγ homo-deficient mice. Additionally, administration of thiazolidine derivatives acting as PPARγ agonists induces the decrease of large adipocytes and the increase of small adipocytes (see non-patent reference 15). Based on the findings described above, the mechanism of thiazolidine derivatives for insulin sensitizing is believed as follows: as the consequence of the rapid promotion of adipocyte differentiation by the PPARγ agonist, the production of TNFα causing insulin resistance is suppressed, together with the promotion of the expression of glucose transporter in peripheral tissues and the suppression of the generation of free fatty acid; consequently, then, glucose uptake in peripheral tissues is activated and hyperglycemia is ameliorated (see non-patent reference 16).

Lately, clinical reports findings using thiazolidine derivatives indicates that all of synthetic PPARγ agonist not only have an action of insulin sensitizing, but also induce edema with increase of plasma volume in vivo. (see non-patent references 17 and 18). The edema induced by the synthetic PPARγ agonists is a severe adverse action resulting cardiac hypertrophy. Therefore, it is strongly desired that the adverse action be separated from the main action thereof, namely the amelioration of insulin resistance. However, it has not yet been elucidated what kind of signal pathway works for a complex of PPARγ and its ligands to induce different responses, namely the adipocyte differentiation along with the insulin sensitizing and the induction of edema. In other words, the molecular mechanism for these inductions has not yet been revealed.

Interactions with a group of transcription cofactors are necessary for the transcriptional activation via PPARs, like other nuclear receptors. Therefore, attempts have been made so as to identify cofactors interacting with PPARs. Actually, biochemical approaches have been used to examine the binding between the known cofactors for nuclear receptors and PPARγ. It is reported that several some molecules such as SRC-1 (see non-patent reference 19), CBP/p300 (see non-patent reference 20), DRIP205 and TRAP220 (see non-patent reference 21), SMRT (see non-patent reference 22), Gadd45 (see non-patent reference 23) and RIP140 (see non-patent reference 24) interact with PPARγ. A report reveals that according to a biochemical approach, similarly, the retinoid X receptor (RXR) together with PPAR forms a heterodimer in a manner dependent on the presence of a ligand, to bind to a response element upstream a target gene (see non-patent reference 25). However, the detailed mechanism of the agonist dependency of these cofactors or of how these cofactors are involved in the downstream signaling of PPAR is still unclear.

Meanwhile, a method using the yeast two-hybrid system (see non-patent reference 26) with intervening ligands has been widely used as a method for screening new cofactors interactive with nuclear receptors. However, it has been difficult so far to find a ligand-dependent binding factor, in particular, for PPARγ by the yeast two-hybrid system. According to the results of screening such PPARγ-binding factors by a yeast two-hybrid system with no intervening ligand, PPARγ-binding factors such as PBP (see non-patent reference 27), PGC-1 (see non-patent reference 28), PGC-2 (see non-patent reference 29), and SHP (see non-patent reference 30) have been reported. However, all the factors interact with PPARγ even in the absence of ligand. Therefore, not any apparently ligand-dependent PPARγ binding factor could be obtained. Only a few reports have indicated about the detection of the ligand dependency of the binding between PPARγ and interactive factors by the yeast two-hybrid system. However, the interaction was detected in these reports using the highly concentrated cultured yeast cells expressing the known cofactors for nuclear receptors together with PPARγ (see patent reference 1 and non-patent reference 24). Therefore, no success was made in screening an apparently ligand-dependent interactive factor for PPARγ from a cDNA library by the yeast two-hybrid system. Concerning the aforementioned Gadd45 and PGC-1, for example, their ligand-dependent interactions with nuclear receptors including a PPARα were detected with the yeast two-hybrid system. However, the ligand-dependency of these cofactors to interact with PPARγ can be observed only by the biochemical approach (see non-patent reference 24). It has been explained that since the biochemical approach and the approach using yeast differ from each other in terms of sensitivity and the ratio of probe to interactive factor, the action of a PPARγ ligand cannot efficiently be detected by the yeast two-hybrid system (see non-patent reference 24). Although the biochemical approach is suitable for detecting the interaction between a pair of proteins, it is very difficult to screen for all proteins interactive with a certain specific protein by the biochemical approach. Meanwhile, the yeast two-hybrid system can screen proteins interactive with an objective protein from libraries.

As described above, it has been strongly desired to separate the adverse action of edema induction from the desirable action of ameliorating insulin resistance, but the molecular mechanism responsible for the separation has not yet been elucidated. Thus, it has been highly desired that the mechanism be elucidated, together with the development of a method for screening a drug ameliorating insulin with a lower level of the adverse action.

Herein, ECHLP/Ech1 includes a structure speculated as a region for two enzymes, namely enoyl-CoA hydratase and dienoyl-CoA isomerase functioning for the fatty acid metabolism within the molecule (see non-patent reference 31). Various reports are issued about the DNA sequence of ECHLP/Ech1 (see patent references 2 to 7). However, the physiological function is not yet elucidated. AOP2 is called anti-oxidant protein 2 (GenBank accession No. XM 001415) because AOP2 includes peroxidase-like sequence within the molecule. Various reports are issued about the DNA sequence of AOP2 (see patent reference 8 to 12). As an actual physiological activity, some report indicated that AOP2 functioned as a calcium-independent phospholipase A2 (see non-patent reference 32), while another report indicated that the gene locus of the AOP2 was revealed as a gene causing polycystic nephropathia in mouse (see non-patent reference 33). As described above, apparently, AOP2 has an action different from the molecular function deduced on the basis of the amino acid sequence and structure, and the original physiological function has not yet been identified. Although the sequence of FLJ13111 has been reported (see patent references 13 and 14), a function of the protein is still unknown. There is no information indicating the molecular function of FLJ13111 based on the amino acid sequence and structure except that the presence of a nucleus targeting sequence and the presence of a site to be possibly glycosylated within the molecule.


(Patent reference 1) The publication of JTP-A-11-56369
(Patent reference 2) The pamphlet of International Publication 00/55350
(Patent reference 3) The pamphlet of International Publication 02/29103
(Patent reference 4) The pamphlet of International Publication 02/00677
(Patent reference 5) The pamphlet of International Publication 01/49716
(Patent reference 6) The pamphlet of International Publication 00/37643
(Patent reference 7) The pamphlet of International Publication 01/75067
(Patent reference 8) The pamphlet of International Publication 98/43666
(Patent reference 9) The pamphlet of International Publication 02/12328
(Patent reference 10) The pamphlet of International Publication 02/29086
(Patent reference 11) The pamphlet of International Publication 02/06317
(Patent reference 12) The pamphlet of International Publication 01/55301
(Patent reference 13) The specification of European patent application 1 074 617
(Patent reference 14) International Publication No. 00/58473
(Non-patent reference 1) J. Biol. Chem., 1995, Vol. 270, p. 12953-12956
(Non-patent reference 2) J. Med. Chem., 1996, Vol. 39, p. 665-668

(Non-patent reference 3) Cell, 1995, Vol. 83, p. 835-839
(Non-patent reference 4) Proc. Nat. Acad. Sci. USA, 1994, Vol. 91, p. 7355-7359
(Non-patent reference 5) Protein /Nucleic Acid/Enzyme (Tanpakushitu/Kakusan/Koso), 1995, Vol. 40, No. 13, p.50-55
(Non-patent reference 6) Diabetes, 1997, Vol. 46, p. 433-439
(Non-patent reference 7) Diabetes Care, 1996, Vol. 19, No. 2, p.151-156
(Non-patent reference 8) Diabetes Care, 1992, Vol. 15, No.2, p. 193-203
(Non-patent reference 9) Diabetologia, 1996, Vol.39, p.701-709
(Non-patent reference 10) Proc. Natl. Acad. Sci. USA, 1997, Vol. 94, p. 4312-4317
(Non-patent reference 11) Drugs, 1990, Vol.40, No. 2, p. 260-290
(Non-patent reference 12) Jpn. J. Cancer Res., 1999, Vol. 90, p.75
(Non-patent reference 13) Genes and Dev., 1994, Vol. 8, p.1224-1234
(Non-patent reference 14) Cell, 1994, Vol. 79, p. 1147-1156
(Non-patent reference 15) Mol. Cell, 1999, Vol. 4, p. 597-609
(Non-patent reference 16) J. Biol. Chem., 1995, Vol. 270, p.12953-12956
(Non-patent reference 17) Diabetes Frontier, 1999, Vol. 10, p. 811-818
(Non-patent reference 18) Diabetes Frontier, 1999, Vol.10, p. 819-824
(Non-patent reference 19) Gene Expr., 1996, Vol. 6, p. 185-195
(Non-patent reference 20) J. Biol. Chem., 1999, Vol. 274, p.7681-7688
(Non-patent reference 21) Mol. Cell. Biol., 2000, Vol. 20, p. 8008-8017
(Non-patent reference 22) Pros. Natl. Acad. Sci. USA, 1998, Vol.95, p. 2920-2925
(Non-patent reference 23) Biochem. Biophys. Res. Commun., 2000, Vol. 272, No.1, p. 193-198
(Non-patent reference 24) Mol Endocrinol., 1998, Vol.12, No. 6, p.864-881
(Non-patent reference 25) Ann. Rev. Cell Dev. Biol., 1996, Vol.12, p.335-363
(Non-patent reference 26) Proc. Natl. Acad. Sci. USA, 1991, Vol.88, p.9578-9582
(Non-patent reference 27) J. Biol. Chem., 1999, Vol. 274, p.7681-7688
(Non-patent reference 28) Cell, 1998, Vol.92, p.829-839
(Non-patent reference 29) EMBO J., 1999, Vol. 18, No. 13, p.3676-3687
(Non-patent reference 30) Biochim. Biophys. Acta., 1997, Vol. 1, No.1350, p.27-32
(Non-patent reference 31) J. Biol. Chem., 1998, Vol. 273, No.1: p.349-355
(Non-patent reference 32) J. Biol. Chem., 1997, Vol. 272, No.16, p.10981
(Non-patent reference 33) Genomics, 1997, Vol. 42, No.3, p.474-478

Disclosure of the Invention

By a unique approach including the presence of a high concentration of a highly effective PPARγ agonist in the yeast two-hybrid system, the present inventors identified a group of proteins binding to PPARγ in a manner dependent on the presence of an agonist with a high effect of triggering an action of ameliorating glucose metabolism (main action) and a group of proteins binding to PPARγ in a manner dependent on the presence of an agonist with a high effect of triggering edema (adverse action). Consequently, the inventors found ECHLP (enoyl-CoA hydratase-like protein) as a molecule binding to PPARγ in a manner dependent on an agonist with main action and human anti-oxidant protein 2 (non-selenium glutathione peroxidase; acidic calcium-independent phospholipase A2; GenBank accession No. XM_001415; hereafter abbreviated as AOP2) as a molecule binding to PPARγ in a manner dependent on an agonist with adverse action.

The inventors found that overexpression of ECHLP protein in cells suppressed the ligand-dependent transcription-inducing activity of PPARγ distinctly. Further, the inventors found that the expression level of ECHLP gene was raised irrespective of the variation of blood glucose level in a diabetic model mouse compared with normal mouse by using the gene chip method. Then, the inventors confirmed that the protein was the factor causing diabetic mellitus. The inventors additionally found that overexpression of AOP2 in cells promoted the ligand-dependent transcription-inducing activity of PPARγ distinctly. Further, the inventors found the increase of AOP2 protein in the diabetic model mouse by two-dimensional electrophoresis and then confirmed that the excess presence of the protein in diabetic mellitus activates the expression of a specific gene group inducing edema through PPARγ.

By the unique approach including the presence of a high concentration of a highly active PPARγ agonist in the yeast two-hybrid system similarly, the inventors found FLJ13111 (GenBank Accession No. AK023173) as a molecule binding to PPARγ in a manner dependent on the agonist with desirable action. Further, the inventors found that overexpression of FLJ13111 in cells activated the ligand-dependent transcription-inducing activity of PPARγ distinctly. Additionally, the inventors confirmed that the expression of the FLJ13111 gene was significantly lowered in the muscle tissue of a diabetic model mouse compared with normal mouse. The inventors first recovered the promoter region of FLJ13111 and constructed an assay system to detect the promoter activity of FLJ13111 gene. The assay system can be utilized for screening PPARγ ligands or drugs ameliorating insulin resistance, with no use of the protein PPARγ.

Based on these findings, a new drug ameliorating insulin resistance, which makes a specific contribution to the desirable action via PPAR and does not induce the adverse action can be identified and additionally, a method for screening such drug is provided.

Specifically, the present invention relates to the following:

(1) A method for screening a protein interactive with PPARγ in a ligand-dependent manner, utilizing a yeast two-hybrid system in the presence of a PPAR ligand with a high potency of triggering the action for ameliorating glucose metabolism, wherein a polynucleotide encoding a region containing at least the position 204 to position 505 of the PPARγ protein represented by SEQ ID NO: 2 is used as bait and a cDNA library is used as prey.

(2) A method for screening a protein interactive with PPARγ in a ligand-dependent manner, utilizing a yeast two-hybrid system in the presence of a PPAR ligand with a high potency of triggering edema, wherein a polynucleotide encoding a region containing at least the position 204 to position 505 of the PPARγ protein represented by SEQ ID NO: 2 is used as bait and a cDNA library is used as prey.

(3) A cell transformed by i) a polynucleotide encoding a polypeptide consisting of an amino acid sequence of SEQ ID NO: 4 or a polynucleotide encoding a polypeptide comprising an amino acid sequence represented by SEQ ID NO: 4 wherein 1 to 10 amino acids therein are deleted, substituted and/or inserted and also interacting with PPAR in a ligand-dependent manner, ii) a gene encoding a fusion protein comprising at least the ligand binding region of the PPAR protein represented by SEQ ID NO: 2 or 6 and the DNA binding region of a transcription factor, and iii) a reporter gene fused to a response element to which said DNA binding region of the transcription factor is capable of binding; or

a cell transformed by i) a polynucleotide encoding a polypeptide consisting of an amino acid sequence of SEQ ID NO: 4 or a polynucleotide encoding a polypeptide comprising an amino acid sequence represented by SEQ ID NO: 4 wherein 1 to 10 amino acids therein are deleted, substituted and/or inserted and also interacting with PPAR in a ligand-dependent manner and ii) a reporter gene fused to a response element to which the DNA binding region of the PPAR protein represented by SEQ ID NO: 2 or 6 is capable of binding, said cell expressing a) a polypeptide consisting of an amino acid sequence of SEQ ID NO: 4 or a polypeptide comprising an amino acid sequence represented by SEQ ID NO: 4 wherein 1 to 10 amino acids therein are deleted, substituted and/or inserted and interacting with PPAR in a ligand-dependent manner and b) the PPAR protein represented by SEQ ID NO: 2 or 6.

(4) A cell transformed by i) a polynucleotide encoding a polypeptide consisting of an amino acid sequence of SEQ ID NO: 8 or a polynucleotide encoding a polypeptide comprising an amino acid sequence represented by SEQ ID NO: 8 wherein 1 to 10 amino acids therein are deleted, substituted and/or inserted and additionally interacting with PPAR in a ligand-dependent manner, ii) a gene encoding a fusion protein comprising at least the ligand binding region of the PPAR protein represented by SEQ ID NO: 2 or 6 and the DNA binding region of a transcription factor, and iii) a reporter gene fused to a response element to which said DNA binding region of the transcription factor is capable of binding, or

a cell transformed by i) a polynucleotide encoding a polypeptide consisting of an amino acid sequence of SEQ ID NO: 8 or a polynucleotide encoding a polypeptide comprising an amino acid sequence represented by SEQ ID NO: 8 wherein 1 to 10 amino acids therein are deleted, substituted and/or inserted and additionally interacting with PPAR in a ligand-dependent manner and ii) a reporter gene fused to a response element to which the PPAR protein represented by SEQ ID NO: 2 or 6 is capable of binding, said cell expressing a) a polypeptide consisting of an amino acid sequence of SEQ ID NO: 8 or a polypeptide comprising an amino acid sequence represented by SEQ ID NO: 8 wherein 1 to 10 amino acids therein are deleted, substituted and/or inserted and interacting with PPAR in a ligand-dependent manner, and b) the PPAR protein represented by SEQ ID NO: 2 or 6.

(5) A cell described in (3) or (4), wherein the transcription factor is the GAL4 protein of yeast.

(6) A cell described in (3) or (4), wherein the reporter gene is luciferase gene.

(7) A method for detecting whether or not a test substance promotes the action of ameliorating glucose metabolism via PPAR, comprising i) a step of allowing the cell described in (3), a PPAR ligand and a test substance in contact with each other, and ii) a step of analyzing the change of the ligand-dependent interaction or the change of the transcriptional activity induced by ligand-activated PPAR, using the expression of a reporter gene as a marker.

(8) A method for screening a drug ameliorating insulin resistance, comprising i) a step of allowing the cell described in (3), a PPAR ligand and a test substance in contact with each other, and ii) a step of analyzing the change of the ligand-dependent interaction or the change of the transcriptional activity induced by ligand-activated PPAR, using the expression of a reporter gene as a marker.

(9) A method for screening as described in (8), wherein the drug ameliorating insulin resistance is a drug ameliorating glucose metabolism.

(10) A method for detecting whether or not a test substance promotes the activity triggering edema via PPAR, comprising i) a step of allowing a test substance in contact with the cell described in (4), and ii) a step of analyzing the change of the interaction due to the test substance or the change of the transcriptional activity induced via PPAR due to the test substance using the expression of a reporter gene as a marker.

(11) A method for screening a drug ameliorating insulin resistance with no activity of triggering edema, comprising i) a step of allowing a test substance in contact with the cell described in (4), ii) a step of analyzing the change of the interaction due to the test substance or the change of the transcriptional activity induced via PPAR due to the test substance, using the expression of a reporter gene as a marker; and iii) a step of selecting a test substance not enhancing the reporter activity.

(12) A method for screening as described in (11), wherein the drug ameliorating insulin resistance is a drug ameliorating glucose metabolism.

(13) A cell transformed by i) a polynucleotide encoding a polypeptide consisting of an amino acid sequence of SEQ ID NO: 17 or a polynucleotide encoding a polypeptide comprising an amino acid sequence represented by SEQ ID NO: 17 wherein 1 to 10 amino acids therein are deleted, substituted and/or inserted and also interacting with PPAR in a ligand-dependent manner, ii) a gene encoding a fusion protein comprising at least the ligand binding region of the PPAR protein represented by SEQ ID NO: 2 or 6 and the DNA binding region of a transcription factor, and iii) a reporter gene fused to a response element to which said DNA binding region of the transcription factor is capable of binding; or

a cell transformed by i) a polynucleotide encoding a polypeptide consisting of an amino acid sequence of SEQ ID NO: 17 or a polynucleotide encoding a polypeptide comprising an amino acid sequence represented by SEQ ID NO: 17 wherein 1 to 10 amino acids therein are deleted, substituted and/or inserted and additionally interacting with PPAR in a ligand-dependent manner and ii) a reporter gene fused to a response element to which the PPAR protein represented by SEQ ID NO: 2 or 6 is capable of binding, said cell expressing a) a polypeptide consisting of an amino acid sequence of SEQ ID NO: 17 or a polypeptide comprising an amino acid sequence represented by SEQ ID NO: 17 wherein 1 to 10 amino acids therein are deleted, substituted and/or inserted and interacting with PPAR in a ligand-dependent manner, and b) the PPAR protein represented by SEQ ID NO: 2 or 6.

(14) A method for detecting whether or not a test substance promotes the action of ameliorating glucose metabolism via PPAR, comprising i) a step of allowing a test substance in contact with the cell described in (13), and ii) a step of analyzing the change of the interaction due to the test substance or the change of the transcriptional activity induced via PPAR due to the test substance, using the expression of a reporter gene as a marker.

(15) A method for screening a drug ameliorating insulin resistance, comprising i) a step of allowing the cell described in (13) in contact with a test substance, and ii) a step of analyzing the change of the interaction due to the test substance or the change of the transcription al activity induced via PPAR due to the test substance, using the expression of a reporter gene as a marker.

(16) A method for screening as described in (15), wherein the drug ameliorating insulin resistance is a drug ameliorating glucose metabolism.

(17) A method for screening a drug ameliorating insulin resistance, comprising i) a step of allowing a test substance in contact with a cell transformed with a reporter gene fused to a polynucleotide consisting of a nucleotide sequence of SEQ ID NO: 26 or a polynucleotide comprising a nucleotide sequence represented by SEQ ID NO: 26 wherein 1 to 10 bases therein are deleted, substituted and/or inserted and also having a transcription promoter activity, and ii) a step of analyzing the change of the activity for transcriptional induction due to the test substance, using the expression of a reporter gene as a marker.

(18) A method for screening as described in (17), wherein the reporter gene is the luciferase gene.

(19) A method for producing a pharmaceutical composition for ameliorating insulin resistance, comprising a screening step using a screening method described in (8), (11), (15) and/or (17) and a formulation step using a substance obtained by the screening.

Amino acid sequences highly homologous to the full-length ECHLP of SEQ ID NO: 4 or a partial sequence thereof, or nucleotide sequences encoding the amino acid sequences are reported in various papers (WO 00/55350, WO 02/29103, WO 02/00677, WO 01/49716, WO 00/37643, WO 01/75067). None of them includes any description that ECHLP is responsible for insulin resistance. Amino acid sequences highly homologous to the full-length AOP2 of SEQ ID NO: 8 or a partial sequence thereof, or nucleotide sequences encoding the amino acid sequences have been reported in various papers (WO 98/43666, Antioxid Redox Signal. 1999 Winter; 1(4): 571-84. Review, WO 2002 12328, WO 2002 29086, WO 2002 06317). However, none of them includes any description that AOP2 is responsible for insulin resistance. WO 01/55301 describes the same sequence as that of AOP2 identified by the inventors and includes a description of the use of the sequence as a substance for regulating functions for a therapeutic treatment of diabetes mellitus among therapeutic treatments of various diseases. However, the publication never includes any example or description supporting the involvement of the sequence in diabetes mellitus. EP 1 074 617 discloses the same amino acid sequence as that of FLJ13111 of SEQ ID NO: 17 and the nucleotide sequence encoding the sequence. However, the report never includes any description about the names of specific diseases wherein FLJ13111 is involved. WO 00/58473 discloses a sequence homologous to the nucleotide sequence of FLJ13111 and includes a description of the use of the sequence as a substance for regulating functions for a therapeutic treatment of diabetes mellitus among therapeutic treatments of various diseases. However, the publication never includes any examples or descriptions supporting the involvement of the sequence in diabetes mellitus. Thus, the binding of ECHLP, AOP2 and FLJ13111 to PPAR is a finding first found by the inventors. Further, the screening of a new drug ameliorating insulin resistance by detecting a substance making a specific contributions to the desirable action through PPAR and does not induce adverse actions using them is an invention first achieved by the inventors.

Brief Description of the Drawings

Fig. 1 is a graph showing the agonist selectivity in the binding of a ligand-dependent PPARγ interactive factor and PPARγ.

Fig. 2 is a graph showing the Ech1 expression levels in diabetic model mice KKA$^y$/Ta (KKA$^y$) and C57BL/KsJ-db/db(db/db) and normal mice for comparison.

Fig. 3 is a graph showing the distribution of Ech1 expressed in tissues.

Fig. 4 is a graph showing the suppressive action of ECHLP on the ligand-dependent transcriptional induction ability of PPARγ.

Fig. 5 is a graph showing the promoting action of AOP2 on the ligand-dependent transcriptional induction ability of PPARγ.

Fig. 6 is a graph showing the screening of a PPARγ ligand specific to the main action, utilizing the actions of ECHLP and AOP2 on the ligand-dependent transcriptional induction ability of PPARγ.

Fig. 7 is a graph showing the promoting action of FLJ13111 on the ligand-dependent transcriptional induction ability of PPARγ.

Fig. 8 is a graph showing the FLJ13111 expression levels in diabetic model mice KKA$^y$/Ta (KKA$^y$) and C57BL/KsJ-db/db(db/db) and normal mice [C57BL/6J (C57BL), C57BL/KsJ/+m(m+/m+)] for comparison.

Fig. 9 is a graph showing the transcriptional induction activity of FLJ1311 promoter and the influence of pioglitazone or the overexpression of FLJ13111 on the activity.

Fig. 10 is a graph showing the influence of ECHLP overexpression on the increase of the triglyceride content via pioglitazone in murine 3T3-L1 cells.

Fig. 11 is a graph showing the transcriptional induction ability of PPARγ in the presence or absence of FLJ13111, which depends on pioglitazone or the compound XF.

Fig. 12 is a graph showing the influence of pioglitazone or the compound XF on the expression level of sodium-potassium ATPase in renal epithelial cells.

Best Mode for Carrying out the Invention

The terms used for the invention are now described below.

The term " main action" used in this specification represents "action for ameliorating glucose metabolism", while the term "adverse action" means "action for triggering edema". The action for ameliorating glucose metabolism refers to an action for promoting a function to incorporate blood sugar (glucose) into cells to consume the sugar therein and accumulate the sugar in the form of an energy storage substance such as glycogen. The action for triggering edema refers to an effect of triggering edema (swelling) because of the accumulation and retention of extracellular fluids in interstitium. The term "main action ligand" means "ligand with a high potent of triggering an action for ameliorating glucose metabolism (main action)" while the "adverse action ligand" means "ligand with a high potent of triggering edema (adverse action)". Concerning the ligand with a high potent of triggering the action for ameliorating glucose metabolism, preferably, the concentration of a compound requiring a 25 % decrement of the blood glucose level compared with a control group is as low as 1/5-fold or less, more preferably 1/10-fold or less the concentration of a PPARγ ligand of the related art (for example, pioglitazone), according to the blood glucose assay method of Miwa I, et. al., more preferably under the conditions of Example 1. The compound includes for example GW-7282 and GI-262570 described below. According to the blood glucose assay method of Miwa I, et. al., blood glucose level is assayed by an enzyme method using a combination of mutarose and glucose oxidase. The ligand with a high potent of triggering edema preferably includes a compound giving a 25% or more increment of circulating plasma volume in two weeks compared with a control group, or giving a 15 % or more increment of circulating plasma volume in two weeks, compared with a well-known PPARγ ligand (for example, pioglitazone), when the compound is administered at 100 mg/kg, according to the method of Brizzee BL et. al. (J. Appl. Physiol. 69(6): 2091-2096, 1990) for assaying circulating plasma volume, more preferably under the conditions of Example 1. The compound includes for example GW-7282 and GI-100085 described below.

The term "cell for testing" refers to "cell wherein the ligand-dependent interaction between PPAR and ECHLP can be assayed using the expression of a reporter gene as a marker", "cell wherein the ligand-dependent interaction between PPAR and AOP2 can be assayed using the expression of a reporter gene as a marker", or "cell wherein the ligand-dependent interaction between PPAR and FLJ13111 can be assayed using the expression of a reporter gene as a marker". The term "yeast two-hybrid system" means a system for detecting protein-protein interaction by utilizing the two separate function of transcription factor of yeast. The transcription factor contains the DNA binding region and the transcription-activating region, and the interaction of both the two regions is necessary for transcriptional activation. In the yeast two-hybrid system consists of two components, 1. a target protein fused to a DNA binding region of the transcription factor and 2. a protein fused to a transcription-activating region of the transcription factor. and the interaction of the two components can be detected by monitoring transcriptional activation. In the yeast two-hybrid system, the bait refers to a target protein fused to a DNA binding region, while the prey refers to a protein fused to the transcription-activating region. The "cDNA library" is prepared by extracting and separating several ten thousands of mRNAs (copies of genetic information to instruct amino acid sequences of proteins) synthesized in cells, then synthesizing complimentary DNAs using the mRNAs as templates with reverse-transcription enzyme, processing the termini and integrating then the resulting cDNAs into a vector. In this specification, "PPAR ligand-binding region" refers to a region where a ligand of PPAR binds, individually including the region including the position 204 to position 505 of the amino acid sequence of SEQ ID NO: 2 for human PPARγ2 and the region including the position 167 to position 468 of the amino acid sequence of human PPARα. The "DNA binding region" is a region functioning for DNA binding and has a DNA binding ability to a response element but has no transcription-activating ability of its own. The DNA binding region of the GAL4 transcription factor exists on the N-terminal side (the region comprising amino acids at about position 1 to about position 147).

The present invention is now described in detail hereinbelow.

In this specification, the PPAR-interactive polypeptide encoded by the polynucleotide contained in the gene of a protein interactive with PPAR for preparing a cell for testing includes

(1) a polypeptide consisting of an amino acid of SEQ ID NO: 4, 8 or 17;
(2) a polypeptide comprising an amino acid sequence represented by SEQ ID NO: 4, 8 or 17 wherein 1 to 10 amino acids therein are deleted, substituted and/or inserted and which is a protein binding to PPAR in a ligand-dependent manner (referred to as functionally equivalent variant hereinafter); and
(3) a polypeptide consisting of an amino acid sequence with 90 % or more homology to the amino acid sequence of SEQ ID NO: 4, 8 or 17, which is a protein binding to PPAR in a ligand-dependent manner (referred to as homologous peptide hereinafter).

The functionally equivalent variant is preferably "a polypeptide comprising an amino acid of SEQ ID NO: 4, 8 or 17, which is a protein binding to PPAR in a ligand-dependent manner", "a polypeptide comprising an amino acid sequence represented by SEQ ID NO: 4 or 17 wherein 1 to 10, preferably 1 to 7, more preferably 1 to 5 amino acids therein are deleted, substituted and/or inserted and which is a protein binding to PPAR in a manner depending on the main action ligand"; or "a polypeptide comprising an amino acid sequence represented by SEQ ID NO: 8 wherein 1 to 10, preferably 1 to 7, more preferably 1 to 5 amino acids therein are deleted, substituted and/or inserted and which is a protein binding to PPAR in a manner depending on the adverse action ligand.

The homologous polypeptide consisting of an amino acid sequence with 90 % or more homology to the amino acid sequence of SEQ ID NO: 4, 8 or 17 and is a protein binding to PPAR in a ligand-dependent manner, with no specific limitation. The homologous polypeptide consisting of an amino acid sequence with preferably 90 % or more, more preferably 95 % or more, still more preferably 98 % or more homology to the amino acid sequence of SEQ ID NO: 4 or 17 and is a protein binding to PPAR, preferably in a manner depending on the main action ligand. The homologous polypeptide consisting of an amino acid sequence with preferably 90 % or more, more preferably 95 % or more, still more preferably 98 % or more homology to the amino acid sequence of SEQ ID NO: 8 and is a protein binding to PPAR, preferably in a manner depending on the adverse action ligand. In this specification, additionally, the 'homology' refers to the value obtained using parameters preset as default by the Clustal program (Higgins and Sharp, Gene 73, 237-244, 1998; Thompson et al., Nucleic Acid Res. 22, 4673-4680, 1994). The parameters are as follows.

Pairwise alignment parameters are as follows.

K tuple 1
Gap Penalty 3
Window 5
Diagonals Saved 5.

The PPAR-interactive polypeptides contained in the cell for testing in this specification are described above. The polypeptide consisting of an amino acid of SEQ ID NO: 4, functionally equivalent variants thereof and homologous polypeptides thereof are collectively referred to as "ECHLP interactive with PPAR" hereinbelow. The polypeptide consisting of an amino acid of SEQ ID NO: 8, functionally equivalent variants thereof and homologous polypeptides thereof are collectively referred to as "AOP2 interactive with PPAR" hereinbelow. The polypeptide consisting of an amino acid of SEQ ID NO: 17, functionally equivalent variants thereof and homologous polypeptides thereof are collectively referred to as "FLJ13111 interactive with PPAR" hereinbelow.

The polynucleotide of a nucleotide sequence encoding the ECHLP interactive with PPAR, the AOP2 interactive with PPAR or the FLJ13111 interactive with PPAR may be any of the polynucleotide encoding the amino acid sequence of SEQ ID NO: 4, 8 or 17, a functionally equivalent variant thereof or a polynucleotide comprising a nucleotide sequence encoding a homologous polypeptide thereof. Preferably, the polynucleotide is a polynucleotide consisting of a nucleotide sequence encoding the amino acid sequence of SEQ ID NO: 4, 8 or 17. More preferably, the polynucleotide consisting of a nucleotide sequence of SEQ ID NO: 3, 7 or 16.

A method for screening a protein interacting with PPAR in a ligand-dependent manner, which works as a useful tool for screening a drug ameliorating insulin resistance without the adverse action is now described below, together with a method for screening a drug ameliorating insulin resistance without the adverse action, utilizing the protein.

[A method for screening a protein interacting with PPAR in a ligand-depending manner]

In accordance with the present invention, all the protein factors interacting with PPARγ in a ligand-dependent manner can be identified from cDNA libraries using as a marker the expression of a reporter gene of the yeast two-hybrid system. In accordance with the present invention, the ligand-dependent interaction between PPAR and a transcription cofactor thereof is detected with no need of the detection of the transcriptional induction ability of PPAR per se. Accordingly, factor groups inherent to mammals are not necessary, which are involved in the expression of the transcription induction ability of PPAR. Therefore, mammalian cells are not necessarily used specifically as the cell for testing. Therefore, eukaryotic cells, for example yeast cells, insect cells and mammalian cells are also satisfactory. Among them, yeast cells can readily be cultured in a rapid way. Additionally, genetic recombination techniques such as the introduction of exogenous genes are readily applicable to the cells. Still additionally, the ligand dependency of the binding between PPAR and the interactive factors can be followed up and detected efficiently by a method using the same yeast two-hybrid system.

The yeast two-hybrid system is a method for detecting a protein-protein interaction using as a marker the expression of a reporter gene. Generally, a transcription factor includes functionally different two regions, namely a DNA binding region and a transcription activating region. In order to examine the interaction between two proteins types X and Y by the two-hybrid system, two protein types namely a fusion protein comprising the DNA binding region of a transcription factor and X and a fusion protein comprising the transcription activating region of a transcription factor and Y are simultaneously expressed in yeast cells. When the proteins X and Y interact with each other, the two types of the fusion proteins form one transcription complex, which binds to a response element (a DNA site for specific binding) of the transcription factor in the cell nucleus to activate the transcription of the reporter gene arranged downstream the response element. As described above, the interaction of the two proteins can be detected as the detection of the expression of the reporter gene.

The yeast two-hybrid system is generally used for identifying an unknown protein interacting with a specific protein, using the specific protein as probe. When the binding of the two occurs in a manner depending on the presence of a receptor ligand as observed in the case of a nuclear receptor and a group of some of the transcription coupling factors thereof, a two-hybrid system with a ligand added extraneously to the system should be used. As described above in the Section "Background of the Invention", however, it was difficult to detect the ligand dependency between PPARγ and the interactive factors by the yeast two-hybrid system. Thus, no success was made in screening for all the ligand-dependent PPARγ interactive factors. The inventors assumed that the reason might be that the PPARγ agonists might have low intracellular permeability because of the yeast properties so that the detection sensitivity of the ligand dependency would be low. By reacting a compound group with the highest activity as the PPARγ agonist among those reported with yeast, the inventors achieved a unique method for the yeast two-hybrid system applicable to the assay of the ligand dependency between PPARγ and the interactive factors and to the screening. More specifically, the screening can be carried out by a method described in Example 2.

Another embodiment of the method including detecting a ligand-dependent interactive factor with PPARγ to assay the action of a test substance on the interaction is a method for biochemically detecting the ligand-dependent binding between PPARγ and the interactive factor. By such a method, a protein binding to a fusion protein comprising an appropriate tag protein such as glutathione-S-transferase (GST), protein A, β-galactosidase, and maltose-binding protein (MBP) and the ligand binding region of PPARγ is directly detected in the presence of a test substance in an extract solution of a culture cell labeled with for example RI; then, the binding protein is purified and determined of its amino acid sequence, for identification.

[Method for detecting an action ameliorating glucose metabolism and method for screening a drug ameliorating insulin resistance, utilizing a protein interacting with PPAR in a ligand-dependent manner; method for detecting an activity trigger edema and method for screening a drug ameliorating insulin resistance with no activity triggering edema, utilizing a protein interacting with PPAR in a ligand-dependent manner]

1. Method for detecting an action ameliorating glucose metabolism and method for screening a drug ameliorating insulin resistance, utilizing ECHLP interactive with PPAR

One embodiment of the present invention is a method for detecting whether or not a test substance can selectively promote the main action through PPAR, using a cell for testing, which is preliminarily transformed with (i) a fusion gene of at least the ligand binding region of PPARα or γ and the DNA binding region of a transcription factor or the gene encoding the full-length PPARα or γ molecule, (ii) the gene encoding the ECHLP interactive with PPAR, and (iii) a reporter gene conjugated to a response element to which the DNA binding region of the transcription factor is capable of binding or a reporter gene conjugated to a response element to which PPARα or γ is capable of binding, comprising a step of making the cell for testing concurrently present with the test substance in the presence of a PPAR ligand, detecting and assaying the change of the suppressive action of the ECHLP interactive with PPAR on the transcription activating ability of PPAR due to the test substance in the cell for testing as the expression of the reporter gene as a marker. An additional embodiment is a method for screening a compound selectively promoting the main action via PPAR by selecting a compound enhancing the reporter activity as detected by the detection method.

2. Method for detecting the activity triggering edema and method for screening a drug ameliorating insulin resistance with no activity triggering edema, utilizing AOP2 interactive with PPAR

One embodiment of the present invention is a method for detecting a compound with the adverse action via PPAR, using a cell for testing, which is preliminarily transformed with (i) a fusion gene of at least the ligand binding region of PPARα or γ and the DNA binding region of a transcription factor or the gene encoding the full-length PPARα or γ molecule, (ii) the gene encoding the AOP2 interactive with PPAR, and (iii) a reporter gene conjugated to a response element to which the DNA binding region of the transcription factor is capable of binding or a reporter gene conjugated to a response element to which PPARα or γ is capable of binding, comprising a step of making the cell for testing concurrently present with a test substance, detecting and assaying the change of the promoting action of the AOP2 interactive with PPAR on the transcription activating ability of PPAR due to the test substance in the cell for testing as the expression of the reporter gene as a marker, together with a method for selecting and screening a compound selectively promoting the desirable action without the adverse action with the reporter system.

3. Method for detecting an action ameliorating glucose metabolism and method for screening a drug ameliorating insulin resistance, utilizing FLJ13111 interactive with PPAR

One embodiment of the present invention is a method for detecting whether or not a test substance can selectively promote the desirable action through PPAR, using a cell for testing, which is preliminarily transformed with (i) a fusion gene of at least the ligand binding region of PPARγ and the DNA binding region of a transcription factor or the gene encoding the full-length PPARγ molecule, (ii) the gene encoding the FLJ13111 interactive with PPAR, and (iii) a reporter gene conjugated to a response element to which the DNA binding region of the transcription factor is capable of binding or a reporter gene conjugated to a response element to which PPARα or γ is capable of binding, comprising a step of making the cell for testing concurrently present with the test substance, detecting and assaying the change of the promoting action of the FLJ13111 interactive with PPAR on the transcription activating ability of PPAR due to the test substance in the cell for testing as the expression of the reporter gene as a marker. An additional embodiment is a method for screening a compound selectively promoting the desirable action via PPAR by selecting a compound enhancing the reporter activity as detected by the detection method.

In the embodiment 1, 2 or 3 above, the transcription factor to be used for the detection of the transcriptional induction ability of PPAR includes but is not limited to any eukaryotic transcription factors with a region binding to a specific DNA sequence in cell nucleus. Additionally, the DNA binding region of such transcription factor has a DNA binding ability to a response element but does not have a transcription activating ability of its own. Such transcription factor includes for example yeast GAL4 protein (Keegan, et al., Science, Vol.231, p. 699-704, 1986, Ma, et al., Cell, Vol. 48, p. 847-853, 1987). In case of GAL4, for example, the DNA binding region and transcription activating region of the GAL4 transcription factor exist on the amino terminus(a region containing amino acids, approximately at position 1 to position 147).

As the response element, a DNA sequence to which the DNA binding region of a transcription factor is capable of binding is used. The region is cut out from the upstream region of the gene or the region may be chemically prepared synthetically for use.

The reporter gene to be arranged downstream the response element includes but is not specifically limited to any reporter gene for general use. As such, enzyme genes quantitatively assayable readily are preferable. The reporter gene includes for example chloramphenicol acetyltransferase gene (CAT), firefly-derived luciferase gene (Luc), and green fluorescence protein gene (GFP) from jellyfish. The reporter gene is functionally conjugated to the downstream of the response element.

A polynucleotide encoding PPARα or γ, the DNA binding region of a transcription factor, the ECHLP interactive with PPAR, AOP2 interactive with PPAR, or FLJ13111 interactive with PPAR can be isolated from cDNA libraries, by the polymerase chain reaction (PCR) or hybridization, using primers and probes designed and synthetically prepared on the basis of the information of known amino acid sequences and nucleotide sequences. The ECHLP interactive with PPAR may be derived from any species as long as the resulting ECHLP can be identified as the same molecular species and interacts with PPAR in a ligand-dependent manner to influence the transcription induction ability of the receptor. The ECHLP interactive with PPAR includes those from mammalian animals, for example humans (LOC115289; GenBank Accession No. XM_008904, HPXEL; GenBank Accession No. U16660, FitzPatrick DR, et al., Genomics 1995 Vol.27 (3): p. 457-466), mouse (Echl; GenBank Accession No. NM_016772), and rat (HPXEL; GenBank accession No. NM_022594, FitzPatrick DR, et al., Genomics 1995 Vol.27(3): p.457-466).

The AOP2 interactive with PPAR may be derived from any species as long as the resulting AOP2 can be identified as the same molecular species and interacts with PPAR in a ligand-dependent manner to influence the transcription induction ability of the receptor. The AOP2 interactive with PPAR includes those from mammalian animals for example humans (AOP2/KIAA0106; GenBank Accession No. XM_001415, D14662), mouse (AOP2/1-Cys Prx/nonselenium glutathione peroxidase; GenBank Accession No. AF004670, AF093852, Y12883), rat (AOX2; GenBank Accession No. AF014009), and cow (GPX/PHGPx; GenBank Accession No. AF080228, AF090194).

The FLJ13111 interactive with PPAR may be derived from any species as long as the resulting FLJ13111 can be identified as the same molecular species and interacts with PPAR in a ligand-dependent manner to influence the transcription induction ability of the receptor. The FLJ13111 interactive with PPAR includes those from mammalian animals for example humans (FLJ13111; GenBank Accession No. AK023173, NM_025082) and mouse (human FLJ13111-like protein; GenBank Accession No. XM_134598).

PPARγ may be derived from any species as long as the resulting PPARγ can be identified as the same molecular species and can function as a nuclear receptor in biological organisms. PPARγ includes for example those derived from mammalian animals such as human, mouse and rat and from Xenopus. The gene sequence and amino acid sequence of PPARγ have been reported (Dryer, et al., Cell, Vol. 68, p.879-887, 1992, Zhu, et al., Journal of Biological Chemistry, Vol. 268, p. 26817-26820, 1993, Kliewer, et al., Proc. Natl. Acad. Sci. USA, Vol. 91, p. 7355-7359, 1994, Mukherjee, et al., Journal of Biological Chemistry, Vol. 272, p. 8071-8076, 1997, Elbrecht, et al., Biochem. Biophys. Res. Commun., Vol.224, p. 431-437, 1996, Chem, et al., Biochem. Biophys. Res. Commun., Vol. 196, p. 671-677, 1993, Tontonoz, et al., Genes & Development, Vol. 8, p.1224-1234, 1994, Aperlo, et al., Gene, Vol. 162, p. 297-302, 1995). Additionally, PPARγ includes two isoform types, namely PPARγ1 and PPARγ2. Compared with PPARγ2, PPARγ1 is deficient in the 30 amino acids on the amino terminus thereof. The remaining amino acid sequence is totally the same. It is known that both of them are expressed in fat tissues.

A polynucleotide encoding PPARα or γ, the DNA binding region of a transcription factor, the ECHLP interactive with PPAR, AOP2 interactive with PPAR or FLJ13111 interactive with PPAR can be obtained for example in the following way. With no limitation to the method, the polynucleotide can be obtained by the known procedure described in "Molecular Cloning", "Sambrook, J., et al., Cold Spring Harbor Laboratory Press, 1989".

mRNA comprising one encoding the protein can be extracted by a known method from cells or tissues with an ability to generate the protein, for example a fat tissue with the ability. The extraction method includes for example the guanidine/thiocyanate/hot phenol method and the guanidine/thiocyanate-guanidine/hydrochloric acid method, preferably guanidine/thiocyanate cesium chloride method. A cell or tissue with an ability to generate PPARα or γ, ECHLP interactive with PPAR, AOP2 interactive with PPAR or FLJ13111 interactive with PPAR can be identified by Northern blotting using a gene comprising the nucleotide sequence encoding the protein or a part thereof, Western blotting using an antibody specific to the protein, and the like.

mRNA can be purified by conventional methods. For example, mRNA is adsorbed onto oligo (dT) cellulose column, which can then be eluted for purification. Further, mRNA can be fractionated further by sucrose density gradient centrifugation method. Additionally, commercially available mRNA extracted may satisfactorily be used, without the mRNA extraction procedure.

Then, the purified mRNA is applied to a reverse-transcription enzyme reaction in the presence of random primer or oligo dT primer, to synthetically prepare a first cDNA chain. The synthesis can be done by conventional methods. Using the resulting first cDNA chain and two primer types directed for a partial region of the intended gene, for example SEQ ID NOs: 9 and 10 for PPARγ, SEQ ID NOs: 12 and 13 for the ECHLP interactive with PPAR, SEQ ID NOs: 14 and 15 for the AOP2 interactive with PPAR, or SEQ ID NOs: 18 and 19 for the FLJ13111 interactive with PPAR, the cDNA is treated by PCR, to amplify the sequence of the intended gene. Using a commercially available cDNA library, additionally, similar two primer types directed for a partial region of the intended gene may be used for PCR, to amplify the sequence of the intended gene. The resulting DNA is fractionated by agarose gel electrophoresis and the like. If desired, the DNA is digested with restriction enzymes. By subsequently conjugating the resulting products, an intended DNA fragment can be obtained. Specifically, such intended DNA fragment can be obtained by the methods described in Examples 2, 4, 5, 7, 8, 10 and 11.

The determination of the sequence of the DNA obtained by the methods described above can be done by the chemical modification method of Maxam and Gilbert (Maxam, A. M. and Gilbert, W., "Methods in Enzymology", 65, 499-559, 1980) or the dideoxynucleotide chain termination method (Messing, J. and Vieira, J., Gene, 19, 269-276, 1982) or the like.

By the method described in "Molecular Cloning", "Sambrook, J., et al., Cold Spring Harbor Laboratory Press, 1989", DNAs encoding these individual regions are used singly or are conjugated together, for conjugation to the downstream of an appropriate promoter, to construct an expression system of PPARα or γ and the ECHLP interactive with PPAR in cells in vitro, and an expression system of PPARα or γ and the AOP2 interactive with PPAR in cells in vitro. In the same manner, an expression system of PPARγ and the FLJ13111 interactive with PPAR in cells in vitro can be constructed.

Specifically, the polynucleotide thus obtained may be integrated in an appropriate vector plasmid and then inserted in the plasmid form into a host cell. These may satisfactorily be constructed so that the two may be contained in one plasmid or the two may be contained separately in individually different plasmids. Otherwise, a cell with such construction integrated in the chromosomal DNA may satisfactorily be obtained and then used.

As to the reporter gene conjugated to a response element, the reporter gene is constructed using general gene recombination techniques; the resulting construct is once integrated in a vector plasmid; then, the resulting recombinant plasmid is inserted into a host cell; and the resulting reporter gene inserted in such manner is used. Otherwise, such construct is integrated into the chromosomal DNA of a cell, and the resulting cell is obtained to use the construct as it is.

PPAR may satisfactorily be inserted extraneously. In case that a fat-derived cell or a kidney-derived cell abundant in endogenous PPARγ is used as a host cell, a construct consisting of only a reporter conjugated to a response element and the ECHLP interactive with PPAR excluding PPARγ, a construct consisting of only a reporter conjugated to a response element and the AOP2 interactive with PPAR excluding PPARγ, a construct consisting of only a reporter conjugated to a response element and the FLJ13111 interactive with PPAR excluding PPARγ may satisfactorily be inserted.

More specifically, a fragment containing the isolated polynucleotide is again integrated in an appropriate vector plasmid, to thereby transform an eukaryotic or prokaryotic host cell. By further inserting an appropriate promoter and a sequence involved in gene expression into such vector, the gene can be expressed in the resulting individual host cells.

For example, the eukaryotic host cell includes cells of vertebrae animals, insects and yeast. As the cells of vertebrae animals, the following ones are often used: a monkey cell COS cell (Gluzman, Y. (1981) Cell, 23, 175-182), dihydrofolate-deficient Chinese hamster ovary cell (CHO) (Urlaub, G. and Chasin, L. A. (1980) Proc. Natl. Acad. Sci. USA, 77, 4216-4220), human embryonic kidney-derived HEK293 cell and 293-EBNA cell (manufactured by Invitrogen) prepared by inserting the EBNA-1 gene of Epstein Barr virus into the cell mentioned above. However, the cell is not limited to those described above. Any cell may be satisfactory, wherein the inhibition of the transcription induction ability of PPARα or γ with the ECHLP interactive with PPAR or the transcription induction activity of PPARα or γ with the AOP interactive with PPAR or the transcription induction activity of PPARγ with the FLJ13111 interactive with PPAR can be detected.

As the expression vector of vertebrae cells, generally, an expression vector with a promoter, RNA splicing sites, polyadenylation sites, a transcription termination sequence and the like as located upstream a gene to be expressed may satisfactorily be used. If necessary, the expression vector may have an origin of replication. The expression vector includes for example but is not limited to pSV2dhfr with the early SV40 promoter (Subramani, S. et al., (1981) Mol. Cell. Biol., 1, 854-864), pEF-BOS with a human elongation factor promoter (Mizushima, S. and Nagata, S. (1990) Nucleic acids Res., 18, 5322), and pCEP4 with a cytomegalovirus promoter (manufactured by Invitrogen).

A case of using the COS cell as the host cell is exemplified now. An expression vector with an origin of SV40 replication and autonomous proliferation ability in the COS cell and additionally with a transcription promoter, a transcription termination signal and an RNA splicing site may be used and includes pME18S (Maruyama, K. and Takebe, Y. (1990) Med. Immunol., 20, 27-32), pEF-BOS (Mizushima, S. and Nagata, S. (1990) Nucleic Acids Res., 18, 5322), and pCDM8 (Seed, B. (1987) Nature, 329, 840-842). The expression vector can be incorporated in the COS cell, by the DEAE-dextran method (Luthman, H. and Magnusson, G. (1983) Nucleic Acids Res., 11, 1295-1308), the calcium phosphate-DNA coprecipitation method (Graham, F. L. and van der Ed, A. J. (1973) Virology, 52, 456-457), the method by means of FuGENE6 (manufactured by Boehringer Mannheim), and electroporation with electric pulse (Neumann, E. et al. (1982) EMBO J., 1, 841-845). In such manner, a desired transformant cell can be obtained.

In case of using the CHO cell as such host cell, a vector capable of expressing the neo gene functioning as a G418 resistant marker, for example pRSVneo (Sambrook, J. et al. (1989): "Molecular Cloning-A Laboratory Manual", Cold Spring Harbor Laboratory, NY) and pSV2-neo (Southern, P. J. and Berg, P. (1982) J. Mol. Appl. Genet., 1, 327-341) is cotransfected together with the expression vector. By selecting a G418-resistance colony, a transformant cell stably generating the protein group can be obtained. In case of using the 293-EBNA cell as the host cell, additionally, an expression vector such as pCEP4 (Invitrogen) with an origin of Epstein Barr virus replication and autonomous proliferation ability in the 293-EBNA cell is used to obtain a desired transformant cell.

The resulting transformant obtained above can be cultured by conventional methods. Through the culturing, the intended protein group is generated in the cell. As the culture medium for use in the culturing, various culture media routinely used for the host cell selected can be selected appropriately. For the COS cells for example, culture media such as the RPMI-1641 culture medium and the Dulbecco's modified Eagle's minimum essential culture medium (DMEM) supplemented for example with the serum component of fetal bovine serum (FBS) on a needed basis may be used. For the 293-EBNA cells, additionally, the Dulbecco's modified Eagle's minimum essential culture medium (DMEM) supplemented for example with the serum component of fetal bovine serum (FBS) and additionally supplemented with G418 can be used.

Culturing a cell for testing in the presence of a test substance, the inhibition of the suppressive action of the ECHLP interactive with PPAR on the transcription induction ability of PPARα or γ due to the test substance can be detected and assayed on the basis of the expression of the reporter gene. (1) When a test substance reacts with the ECHLP interactive with PPAR or with PPAR and the suppressive effect of the ECHLP interactive with PPAR on the transcription induction activity of PPAR is reduced in a manner dependent on the action, it is observed that the reporter activity expressed reaches maximum. Such test substance can be identified as a promoting agent of the main action through PPAR. Additionally (2) when a test substance binds to PPAR to promote the transcription induction ability while the test substance inhibits the suppressive effect of the ECHLP interactive with PPAR, the increase of the expressed reporter activity is observed. Such test substance is identified as an agonist specific to the main action through PPAR. Further (3) when a test substance binds to the ECHLP interactive with PPAR to inhibit the suppressive effect of the transcription induction ability of PPAR, or when a test substance inhibits the expression of the ECHLP interactive with PPAR or promotes the decomposition thereof, it is observed similarly that the expressed reporter activity increases. Such substance can be identified as an inhibitor of the ECHLP interacting with PPAR to promote the main action through PPAR. Expectantly, any of these (1), (2) and (3) acts as a drug ameliorating insulin resistance, without the adverse action brought about by a PPAR agonist. More specifically, a drug ameliorating insulin resistance can be identified and screened by the methods described in Examples 5 and 9. Under the conditions described in Example 9, for example, a substance with IC50 of 10 μM or less, preferably 1 μM or less can be selected as a drug ameliorating insulin resistance.

Culturing a cell for testing in the presence of a test substance, it can be detected and assayed on the basis of the expression of the reporter gene that the promoting action of the AOP2 interactive with PPAR on the transcription induction ability of PPARα or γ can be suppressed by the test substance. (1) When a test substance reacts with the AOP2 interactive with PPAR or with PPARγ to reduce the promoting effect of the AOP2 interactive with PPAR on the transcription induction ability of PPARγ in a manner depending on the action, the decrease of the reporter activity expressed is observed. Such test substance can be identified as a substance suppressing the adverse action through PPARγ. Additionally (2) when a test substance binds to PPARγ to promote the transcription induction ability while the test substance inhibits the promoting effect of the AOP2 interactive with PPAR, it is observed that the reporter activity expressed is decreased to the same level as the state with no concurrent expression of the AOP2 interactive with PPAR. Such test substance is identified as an agonist selective to the main action through PPARγ without the adverse action. Further (3) when a test substance binds to the AOP2 interactive with PPAR to inhibit the promoting effect of the transcription induction ability of PPARγ, or when a test substance inhibits the expression of the AOP2 interactive with PPAR or promotes the decomposition thereof, it is observed similarly that the expressed reporter activity decreases. Such substance can be identified as an inhibitor of the AOP2 interactive with PPAR to suppress the adverse action through PPARγ. Expectantly, any of them acts as a drug ameliorating insulin resistance without the adverse action brought about by a PPARγ agonist. Meanwhile, when a test substance reacts for example with the AOP2 interactive with PPAR or with PPARγ to activate the promoting effect of AOP2 interactive with PPARγ on the transcription induction activity of PPARγ in a manner depending on the action, the increase of the reporter activity expressed is observed. Such test substance is identified as a substance strongly triggering the adverse action through PPARγ, so a drug ameliorating insulin resistance with no activity inducing edema can be screened by selecting a test substance never involving the increase of the reporter activity.

Culturing a cell for testing in the presence of a test substance, the activation with a test substance of the promoting action of the FLJ13111 interactive with PPAR on the transcription induction ability can be detected and assayed on the basis of the expression of the reporter gene. (1) When a test substance acts with the FLJ13111 interactive with PPAR or with PPARγ to enhance the promoting effect of the FLJ13111 interactive with PPAR on the PPARγ transcription induction activity in a manner dependent on the action, it is observed that the reporter activity expressed increases. Such test substance can be identified as a promoting agent of the main action through PPARγ. Additionally (2) when a test substance binds to PPAR to promote the transcription induction ability while the test substance enhances the promoting effect of the FLJ13111 interactive with PPAR, such test substance is identified as an agonist specific to the main action through PPAR. Further (3) when a test substance binds to the FLJ13111 interactive with PPAR to enhance the promotion effect of the transcription induction ability of PPAR or when a test substance promotes the expression of the FLJ13111 interactive with PPAR or suppresses the decomposition thereof, it is also observed that the reporter activity expressed is increased similarly. Such substance is identified as an activating agent of the FLJ13111 interactive with PPAR, to promote the main action through PPAR. Expectantly, any of these (1), (2) and (3) acts as a drug ameliorating insulin resistance without the adverse action brought about by a PPAR agonist. More specifically, a drug ameliorating insulin resistance can be identified and screened by the methods described in Examples 11 and 12. Under the conditions described in Example 12, for example, a substance with ED50 of 10 μM or less, preferably 1 μM or less can be selected as a drug ameliorating insulin resistance.

[Method for screening a drug ameliorating insulin resistance utilizing a promoter for FLJ13111 interactive with PPAR]

i) A drug ameliorating insulin resistance can be screened by a method comprising i) a step of allowing a test substance in contact with a cell transformed with a reporter gene fused to a polynucleotide consisting of the nucleotide sequence of SEQ ID NO: 26 or a polynucleotide comprising the nucleotide sequence of SEQ ID NO: 26 wherein 1 to 10 amino acids therein are deleted, substituted and/or inserted and also having a transcription promoter activity and ii) a step of analyzing the change of the transcription activity-inducing activity with the test substance, using as a marker the expression of the reporter gene.

The reporter gene assay (Tamura, et al., Transcription Factor Research Method, Yodosha, 1993) is a method for assaying the regulation of gene expression using as the marker the expression of a reporter gene. Generally, gene expression is regulated with a part called promoter region existing in the 5'-upstream region thereof. The gene expression level at the stage of transcription can be estimated by assaying the activity of the promoter. When a test substance activates a promoter, the transcription of the reporter gene arranged downstream the promoter region is activated. In such manner, the expression of the reporter gene can be detected in place of the promoter-activating action, namely the action of activating the expression. Thus, the expression of the reporter gene can be detected in place of the action of a test substance on the regulation of the expression of the FLJ13111 interactive with PPAR, by the reporter gene assay using the promoter region of the FLJ13111 interactive with PPAR. As the "reporter gene" to be fused to the FLJ13111 promoter region consisting of the nucleotide sequence of SEQ ID NO: 26, any reporter gene for general use is satisfactory with no specific limitation. For example, an enzyme gene readily assayable quantitatively is preferable. For example, the reporter gene includes chloramphenicol acetyltransferase gene (CAT) derived from bacteria transpozon, luciferase gene (Luc) derived from firefly and green fluorescence protein gene (GFP) derived from jellyfish. The reporter gene may satisfactorily be fused functionally to the FLJ13111 promoter region consisting of the nucleotide sequence of SEQ ID NO: 26. By comparing between the expression level of the reporter gene in case that a test substance is in contact with a cell transformed with the reporter gene fused to the promoter region of the FLJ13111 interactive with PPAR and the expression level thereof in case that a test substance is not in contact with the reporter gene, the change of the transcription induction activity depending on the test substance can be analyzed. By carrying out the step, screening a substance activating the expression of FLJ13111 and a substance ameliorating insulin resistance can be done. Specifically, the screening can be carried out by the method described in Example 14.

The test substance for use in the screening method of the invention includes but is not limited to commercially available compounds (including peptides), various known compounds (including peptides) registered in the chemical files, a group of compounds obtained by the combinatorial chemistry technique (N. K. Terrett, M. Gardner, D.W.Gordon, R.J.Kobylecki, J. Steele, Tetrahedron, 51, 8135-73 (1995)), bacterial culture supernatants, naturally occurring components derived from plants and marine organisms, animal tissue extracts or compounds (including peptides) chemically or biologically modified from compounds (including peptides) selected by the screening method of the invention.

[Method for producing a pharmaceutical composition for ameliorating insulin resistance]

The present invention encompasses a method for producing a pharmaceutical composition for ameliorating insulin resistance, comprising a screening step using the screening method of the invention and a formulation step using a substance obtained by the screening.

The formulation containing the substance obtained by the screening method of the invention as the active component can be prepared, using carriers, excipients and/or other additives for general use in the formulation of the active component, depending on the type of the active component.

The dosing includes oral dosing via tablets, pills, capsules, granules, fine granules, powders or oral liquids, or parenteral dosing via intravenous and intramuscular injections or injections into joints, suppositories, transcutaneous dosage forms or transmembrane dosage forms. For peptides to be digested in stomach, in particular, parenteral dosing such as intravenous injection is preferable.

A solid composition for oral dosing contains one or more active substances and at least one inert diluents, such as lactose, mannitol, glucose, microcrystalline cellulose, hydroxypropyl cellulose, starch, polyvinylpyrrolidone or magnesium aluminate metasilicate. The composition may contain additives other than inert diluents, for example lubricants, disintegrators, stabilizers or dissolution agents or auxiliary dissolution agents according to general methods. If necessary, tablets or pills may be coated with films such as sugar coating or stomach-soluble or enteric coatings.

The oral liquid composition may include for example emulsions, solutions, suspensions, syrups or elixirs and may contain inert diluents for general use, for example distilled water or ethanol. The composition may contain additives other than inert diluents, for example, emollients, suspending agents, sweeteners, flavoring agents or preservatives.

Non-parenteral injections may include aseptic, aqueous or non-aqueous solutions, suspensions or emulsions. The aqueous solutions or suspensions may contain for example water for injection or physiological saline as diluents. The diluents for non-aqueous solutions or suspensions include for example propylene glycol, polyethylene glycol, plant oils (for example, olive oil) and alcohols (for example, ethanol), or polysorbate 80. The composition may contain an emollient, an emulsifying agent, a dispersant, a stabilizer, a dissolution agent or an auxiliary dissolution agent, or a preservative. The composition can be sterilized by filtration through bacteria-trapping filters, blending of sterilizing agents or irradiation. Additionally, an aseptic solid composition is produced, which is then dissolved in aseptic water or other aseptic media for injection prior to use and is then used.

The dose can be appropriately determined, in view of the active component, namely a substance inhibiting the activation of the LTRPC2 protein or the intensity of the activity of a substance obtained by the screening method of the invention, the symptom, and age or sex of a subject for its dosing.

In case of oral dosing, for example, the daily dose is about 0.1 to 100 mg, preferably 0.1 to 50 mg per adult (with a body weight of 60 kg). In case of parenteral dosing in the form of an injection, the daily dose is 0.01 to 50 mg, preferably 0.01 to 10 mg.

## Examples

The invention is now described in detail in the following Examples. However, the Examples never limit the present invention. Unless otherwise stated, herein, the invention may be carried out according to the known method ("Molecular Cloning", Sambrook, J., et al., Cold Spring Harbor Laboratory Press, 1989, etc.). In case of using commercially available reagents or kits, the invention is also carried out according to the instructions of the commercially available products.

### (Example 1)

### Identification of main action ligand and adverse action ligand

Five types of thiazolidine derivatives reported to act as PPARγ agonists were the following compounds: GW7282 [(S)-3-[4-[2-(5-methyl-2-phenyloxazol-4-yl)ethoxy]phenyl]-2-(1-pyrolyl)propionic acid; Glaxo Smith Kline, Drug Data Rep 2001, 23(9): 889], GI-262570 [(S)-2-[(2-benzoylphenyl)amino]-3-[4-[2-(5-methyl-2-phenyloxazol-4-yl) ethoxy]phenyl]propionic acid; Glaxo Smith Kline, WO 00/38811], GL-100085[2-(3-(2-(5-methyl-2-phenyloxazol-4-yl) ethoxy)phenylmethylthio)acetic acid; Ono Pharmaceutical Co., Ltd, WO 99/46232], rosiglitazone [(±)-5-[4-[2-[N-methyl-N-(2-pyridyl)amino]ethoxy]benzyl]-2,4-thiazolidinedione maleate; Glaxo Smith Kline, WO 01/47529], pioglitazone [(+)-5-[4-[2-(5-ethyl-2-pyridinyl)ethoxy]benzyl]-2,4-thiazolidinedione; Takeda Chemical Industries, Ltd., JP-A-61-267580]. So as to elucidate the action mechanisms thereof, the compounds were first synthetically prepared according to the methods reported in the patent specifications or references thereof. In the presence of these compounds, the main action and the adverse action were individually measured by using animals. The resulting effects were quantified. Herein, the action of lowering blood glucose as a marker of the main action and the increase of the circulating plasma volume as a marker of the action of triggering edema were measured (Masa-aki Arakawa, Latest Internal Medicine Outline (Saishin Naikagaku Taikei), Vol. 3, Main Symptoms; Diagnosis based on Symptoms, 260-266, 1966; Kanazawa, et al., Diabetes Frontier, Vol.10, p. 811-818, 1999; Iwamoto, Diabetes Frontier, Vol.10, p.819-824, 1999).

(1) Assaying the action for lowering blood glucose in the compound administrated group

The individual compounds suspended in 0.5 % methyl cellulose (MC) and subsequently adjusted to a final concentration (1 - 10 mg/kg) were orally given once daily to $KKA^y$/Ta mice of age 7 to 8 weeks (Clea Japan, Inc.) continuously for 4 days. Only 0.5 % MC was given to a control group. 16 hours after the final dosing, blood was taken out from the murine tail vein. The blood glucose level was assayed with a commercial kit (Glucose CII Test Wako, Wako Pure Chemical Industries, Ltd.) using an enzyme method by means of a combination of mutarose and glucose oxidase (see Miwa I, et al., Clin Chim Acta Vol.37, p. 538, 1972). Provided that the blood glucose level in the control group was defined as 100 %, the compound concentration (ED25) estimated to reduce the blood glucose level in the control group by 25 % was calculated by linear regression using the least square method (Table 1).

(2) Assaying the activity for triggering edema of the compound group

A test compound was orally dosed once daily at a dose of 100 mg/kg (suspended in 0.5 % methyl cellulose) to rats (Sprague-Dawley rats; males of age 3 weeks) continuously for 2 weeks. The plasma volume was assayed, fundamentally according to the method described in J. Appl. Physiol. 69(6): 2091-2096, 1990. 0.25 % Evans Blue solution (in physiological saline) was administrated by intravenous injection into the lower leg of the rats at 0.25 ml (0.625 mg)/rat under anesthesia with ether. Five minutes later, blood was taken out from the inferior vena cava. The plasma was diluted with water. The Evans Blue concentration (mg/ml) based on the absorbance (620 nm) was divided by the injection amount (0.625 mg). The resulting value was defined as plasma volume. Further, the ratio (%) of the plasma volume on a body weight basis to that in the control group (vehicle-dosed group) was calculated (Table 1).

As the results of them, GW7282 strongly triggered the main action and the adverse action. Meanwhile, GI-262570 was at a relatively high value of triggering the main action but showed a weak action. Additionally, GL-100085 poorly triggered the main action but strongly triggered the adverse action.

Table 1

| Blood glucose-lowering action and circulating plasma volume-increasing action of PPARγ agonists | | |
|---|---|---|
| | Hypoglycemic Test ED25 (mg/kg) | Circulating plasma % of CTRL |
| GW-7282 | 0.41 | 130 |
| GI-262570 | 0.98 | 124 |
| GL-100085 | 17 | 133 |
| Pioglitazone | 10 | 110 |
| Rosiglitazone | 4.6 | 114 |

(Example 2)

Identification of protein interacting with PPARγ in a ligand-dependent manner

(1) Isolation of PPARγ gene

cDNA encoding the C-terminal 302 amino acids including the DNA binding region and ligand binding region of PPARγ was obtained from a cDNA library derived from human fat tissues (Clontech: Marathon Ready™ cDNA) by polymerase chain reaction (PCR). In order to insert the cDNA into an expression vector pDBtrp (Invitrogen; containing *TRP1* gene as a selective marker) for the yeast two-hybrid on the basis of the gene sequence of human PPARγ2 as described as the GenBank Accession No. U79012 in the gene database, regions homologous to the 40 nucleotides before and after the multicloning site of the vector was added to the cDNA. Further, primers of SEQ ID NOs.: 9 and 10 were designed so that individual recognition sites for restriction enzymes KpnI and SmaI were added on both the ends of the inserted gene fragment of PPARγ. Using a DNA polymerase (Pyrobest DNA polymerase; manufactured by TaKaRa, Co., Ltd.), PCR was done at 98 °C (for one minute) and subsequently by repeating a cycle of 98 °C (5 seconds)/ 55 °C (30 seconds) and 72 °C (3 minutes) 35 times. Consequently, the resulting DNA fragment of 1004 base pairs (bp) includes the coding region of PPARγ, which consists of 302 amino acids from the 204-th amino acid of the PPARγ2 to the amino acid immediately before the termination codon.

(2) Preparation of expression plasmid for use in yeast two-hybrid

The vector pDBtrp linearized by digestion with restriction enzymes SalI and NcoI and the PCR fragment containing the cDNA of PPARγ as obtained in (1) were simultaneously added to a yeast strain MaV203 (Invitrogen) for use in the two-hybrid, for transfection by the lithium acetate method (C Guthrie, R Fink Guide to Yeast Genetics and Molecular Biology, Academic, San Diego, 1991). Consequently, homologous recombination occurred in the yeast cell, so that a plasmid with the PPARγ cDNA inserted at the multicloning site of pDBtrp (abbreviated as pDB-PPARγ hereinbelow) was formed. The yeast cell carrying the plasmid was selected by culturing the cell on the solid synthetic minimum essential culture medium (DIFCO) (20 % agarose) deficient in tryptophan as a selective marker of the plasmid. The yeast cell was treated with Zymolyase (Seikagaku Corporation) at ambient temperature for 30 minutes. Subsequently, the plasmid was isolated and purified by the alkali method ("Molecular Cloning", Sambrook, J., et al., Cold Spring Harbor Laboratory Press, 1989), for the determination of the nucleotide sequence using a sequencing kit (Applied BioSystems) and a sequencer (ABI 3700 DNA sequencer, Applied BioSystems). Thus, a plasmid with the inserted cDNA of PPARγ was selected, where the reading frame of the cDNA of PPARγ matches with the reading frame of the pDBtrp region encoding the DNA binding region of GAL4.

(3) Yeast two-hybrid screening

The yeast strain MaV203 for use in the two-hybrid as transformed with the pDB-PPARγ was suspended in 400 ml of the YPD culture broth (DIFCO) and cultured under agitation at 30 °C for about 6 hours until the absorbance at a wavelength of 590 nanometer reached 0.1 to 0.4. Subsequently, the resulting cell was prepared into a competent cell by the lithium acetate method. The final volume was suspended in 1.0 ml of 0.1M lithium-Tris buffer. The cell was transformed with 20 μg each of the human kidney cDNA library, human liver library or human skeletal muscle (all from Clontech; Match Maker cDNA library). The resulting cells were screened by culturing on a solid synthetic minimum essential culture medium (DIFCO) (20 % agarose) deficient in tryptophan and leucine as selective markers for the pDB-PPARγ plasmid and the library plasmid, respectively, to obtain a transformant strain with both the plasmids inserted therein. Concurrently, the transformant cell was cultured at 30 °C for 5 days on the solid minimum essential culture medium from which histidine other than tryptophan and leucine was preliminarily eliminated and which was preliminarily supplemented with 20 mM 3AT (3-AMINO-1,2,4-TRIAZOLE; Sigma) as an inhibitor of the enzyme encoded by a reporter gene *HIS3,* so as to select a cell expressed in case that a fused protein of the GAL4 transcription-promoting region bound to an artificially expressed fusion protein of the GALA DNA binding region in the two-hybrid system, where the reporter gene *HIS3* was involved. A PPARγ agonist GW7282 strongly triggering both the main action and the adverse action was preliminarily added to the culture medium to a final concentration of 1.5 μM, to obtain a 3AT-resistant yeast colony showing the expression of a protein binding to PPARγ in the presence of the agonist. These yeast cells were grown on the YPD solid culture medium at a state with addition of the agonist GW7282 at a concentration of 15 μM or with no such addition for 24 hours. Subsequently, the expression of a binding-indicating reporter for the two-hybrid system, as a different reporter from *HIS3*, namely the lacZ gene, was examined, using as a marker the β-galactosidase activity. The β-galactosidase activity was assayed by transferring the yeast cells on the culture medium onto a nitrocellulose filter, freezing then the cells in liquid nitrogen, thawing the resulting cells at ambient temperature, leaving the filter to stand alone on a filter immersed with 0.4 % X-GAL (Sigma) solution at 37 °C for 24 hours and measuring the change of blue color with β-galactosidase. By selecting a colony with the cell contents transferred onto the filter being changed from white to blue, plural yeast cells expressing a protein binding to PPARγ in a manner depending on the presence of the agonist were identified. According to the method described in the Yeast Protocols Handbook of Clontech, a plasmid derived from the library was extracted from the cells. The nucleotide sequence of the gene fragment contained therein was sequenced, using the nucleotide sequence of SEQ ID NO: 11 (the sequence binding to the GAL4AD region; derived from Cloning vector pACT2 under GenBank Accession No. U29899) as primer and a sequencing kit (Applied BioSystems) and a sequencer (ABI 3700 DNA sequencer by Applied BioSystems). Consequently, it was verified by the homology screening by BLAST (NCBI) that clones containing a partial sequence of SEQ ID NO: 3 from ECHLP were contained in any of those derived from the three types of libraries. Additionally, clones containing a gene fragment of SRC-1 (Smith CL, et al., Natl. Acad. Sci. USA, Vol.20, No. 93(17), p. 8884-8888, 1996) and N-CoR (Nagy L., et al., Cell, Vol. 89, No.3, p.373-380 (1997)) were contained in those derived from the kidney-derived library. Thus, it was confirmed that the ligand-dependent coupling factor of PPARγ could be obtained by the screening described above.

Additionally, the same yeast two-hybrid screening was performed under the following conditions. As a library, a cell transformed with the human kidney cDNA library was used. GW7282 was added to a final concentration of 1 μM. A yeast cell expressing a protein binding to PPARγ in the presence of the agonist was grown in the YPD solid culture medium for 24 hours at a state with GW7282 added to a concentration of 10 μM. By assaying the β-galactosidase activity, plural yeast cells expressing the protein binding to PPARγ in a manner depending on the presence of the agonist were identified. From the cells, a plasmid derived from the library was extracted. The nucleotide sequence of the gene fragment contained therein was sequenced. Consequently, two independent clones containing a partial sequence of SEQ ID NO: 7 from AOP2 (GenBank Accession No.: XM_00415) were contained therein. Additionally, a clone containing gene fragments of SRC-1 (Smith CL, et al., Pro. Natl. Acad. Sci. USA, Vol.20, No. 93(17), p. 8884-8888, 1996) and N-CoR (Nagy L., et al., Cell, Vol. 89, No.3, p.373-380 (1997)) known as transcriptional cofactors of nuclear receptors were contained therein. It was now verified that the ligand-dependent transcription factor of PPARγ could be obtained by the screening.

Additionally, the same yeast two-hybrid screening was performed under the following conditions. As a library, cells transformed with the human liver cDNA library were used. GW7282 was added to a final concentration of 1 μM. A yeast cell expressing a protein binding to PPARγ in the presence of the agonist was grown in the YPD solid culture medium for 24 hours at a state with GW7282 added to a concentration of 10 μM. By assaying the β-galactosidase activity, plural yeast cells expressing the protein binding to PPARγ in a manner depending on the presence of the agonist were identified. From the cells, a plasmid derived from the library was extracted. The nucleotide sequence of the gene fragment contained therein was sequenced. Consequently, a clone containing a partial sequence of the novel gene of SEQ ID NO: 16 (FLJ13111-analogous gene; a one-base substituted gene from GenBank Accession No.: AK023173) was contained.

(Example 3)

Detection of ligand-selective interaction between PPARγ and ECHLP or AOP2

The agonist dependency of the interaction between a protein group mainly including ECHLP and AOP 2 as obtained in Example 2 and PPARγ was assayed, using two types of agonists with different effects on the main action and the adverse action, namely GI-262570 (at a final concentration of 5 μM or 0.5 μM) and GL-100085 (at a final concentration of 5 μM or 0.5 μM) and the β-galactosidase activity in the yeast two-hybrid system as a marker (Fig.1; solid arrow and striped arrow point out larger changes of the interactions due to the difference in concentration between the main action-selective compound and the adverse action-selective compound, respectively; open arrow points out larger changes of the interactions due to the difference in concentration between the main action- and adverse action-selective compounds). The details of the method are the same as in Example 2 except for the agonists used. Consequently, the compound with a higher effect on the main action, namely GI-262570 induced the binding between PPARγ and ECHLP similarly even when the concentration was lowered from 5 μM to 0.5 μM (Fig. 1b), while the compound with a relatively high effect on the adverse action, namely GL-100085 profoundly reduced the binding between PPARγ and ECHLP when the concentration was lowered from 5 μM to 0.5 μM (Fig. 1c). Alternatively, the compound GL-100085 with a relatively high effect on the adverse action similarly induces the binding between PPARγ and AOP2 even when the concentration was lowered from 5 μM to 0.5 μM (Fig. 1c), while the compound GI-262570 with a high effect on the action when added significantly reduced the binding between PPARγ and AOP2 even when the concentration was lowered from 5 μM to 0.5 μM (Fig. 1b). These may be due to the possible occurrence of a ligand-dependent interaction between PPARγ and ECHLP or between PPARγ and AOP2 because of the presence of the agonists GI-262570 and GL-100085. The results apparently indicate that ECHLP interacts with PPARγ at a high sensitivity due to the agonist with a high effect on the main action. Alternatively, it is shown that AOP2 interacts with PPARγ at a high sensitivity due to the agonist with a high effect on the adverse action (Fig. 1c). The results indicate the presence of coupling factors interactive with PPARγ in an agonist-dependent manner in correlation with the main action of an agonist or the adverse action of an agonist. ECHLP makes a more selective response to the agonist causing a stronger expression of the main action to interact with PPARγ. It was considered that by utilizing the ligand-dependent interaction between PPARγ and ECHLP, an agonist with a higher effect on the main action could be detected selectively. Meanwhile, the clones #1, 4, 5, 6, 7 and N-CoR showed lower binding levels with PPARγ when the concentration of any of the agonists GI-262570 and GL-100085 was reduced, so that no correlation with the main action of the agonists or with the adverse action thereof was observed in the clones.

(Example 4)

Assaying ECHLP expression level in normal mice and diabetic model mice

Based on the findings described above, it was anticipated that the interaction between ECHLP and PPARγ might be involved in the amelioration of glucose metabolism as the main action via PPARγ agonist. Therefore, the expression level of the messenger RNA (mRNA) of the mouse ortholog ech1 gene in the ECHLP gene was assayed in skeletal muscle and fat in two diabetic model types of mice, namely KKA$^y$/Ta (Iwatsuka, et al., Endocrinol. Japan., Vol.17, p. 23-25, 1970, Taketomi, et al., Horm. Metab. Res., Vol.7, p. 242-246, 1975) and C57BL/KsJ-db/db (Chen, et al., Cell, Vol. 84, p. 491-495, 1996, Lee, et al., Nature, Vol. 379, p. 632-635, 1996, Kaku, et al., Diabetologia, Vol. 32, p. 636-643, 1989), using DNA arrays (Affimetrix) (de Saizieu, et al., Nature Biotechnology, Vol. 16, p.45-48, 1998, Wodicka, et al., Nature Biotechnology, Vol. 15, p. 1359-1367, 1997, Lockhard, et al., Nature Biotechnology, Vol.14, p. 1675-1680, 1996), to compare the results with those in normal individual mice C57BL/6J and C57BL/KsJ-+m/+m.

(1) Resection of mouse tissues:

Male eight mice of each of C57BL/6J, $KKA^y$/Ta, C57BL/KsJ-+m/+m and C57BL/KsJ-db/db were purchased from Clea Japan, Inc. The C57BL/6J mice were fed as a group with general diet until 15 weeks old. The $KKA^y$/Ta mice were fed singly with a high calories diet (CMF, Oriental Yeast Co., Ltd.) until 15 weeks old. The C57BL/KsJ-+m/+m mice and the C57BL/KsJ-db/db mice were fed as groups with general diet until 12 weeks old. It was confirmed that compared with the normal mice, the $KKA^y$/Ta and C57BL/KsJ-db/db mice were hyperglycemic at larger body weights ($KKA^y$/Ta mice: blood glucose level at 514.2 ± 18.2 mg/dl, body weight at 49.9 ± 0.7 g; C57BL/KsJ-db/db mice: blood glucose level at 423.7 ± 14.1 mg/dl, body weight at 48.6 ± 0.5 g). The blood glucose level was assayed by taking blood from murine caudal vein and using a commercially available kit by means of the glucose oxidase method (Autopack A/glucose reagent, Boehringer Mannheim). These four murine species were anesthetized under diethyl ether, for resecting epididymal fat tissues and gastrocnemius muscles. Immediately after the resection, these tissues were frozen in liquid nitrogen and stored at -80 °C.

(2) mRNA extraction:

The tissues were disrupted using a cryo-press disruption apparatus CRYO-PRESS CP-100 (Microtec Nition). Adding ISOGEN as an RNA extraction reagent (Nippon Gene), the resulting mixture was homogenized using a homogenizer ULTRA-TURRAX T-8 (IKA Labortechnik). According to the manufacturer's instructions, RNA was extracted from these samples. The resulting RNA was treated with DNase (Nippon Gene), to decompose the contaminating DNA. Subsequently, the RNA was prepared by the phenol/chloroform extraction and ethanol precipitation, and was then dissolved in RNase-free $H_2O$. Using an RNA preparation reagent QuickPrep Micro mRNA Purification kit (Amersham) and according to the manufacturer's instructions, mRNA was extracted.

(3) Preparation of labeled cDNA:

According to the instructions of Affimetrix (GeneChip Expression Analysis Technical Manual), a first strand cDNA, a second strand cDNA and biotin-labeled cRNA were synthetically prepared from mRNA, and then, the labeled cRNA was fragmented.

(4) Hybridization:

The DNA array of Affimetrix (GeneChip U74) consists of 3 subarray sheets A, B and C. According to the instructions of Affimetrix, the labeled cRNA was hybridized with the DNA array and was then rinsed, for assaying the fluorescent intensity of each probe.

(5) Correction of inter-array assay values:

The assay values were corrected in an inter-sample manner and subsequently in an inter-array manner. The inter-sample correction was done by first determining the total value of the fluorescent intensities of genes on a specific subarray in a inter-sample manner and then multiplying the assay value of each gene on another subarray by a certain magnification factor per subarray so that the total value thereof might be equal to the array with the largest total value of the fluorescent intensities. Correction in an inter-subarray manner was done by determining the mean value of the fluorescent intensities of AFFX probe of each subarray and multiplying the assay value of each gene by a certain magnification factor per subarray so that the mean values thereof might be equal to each other among the subarrays A, B and C.

Consequently, it was confirmed that $KKA^y$/Ta mice of age 15 weeks apparently with the onset of the disease showed the expression level of ech1 mRNA 2-fold or more, compared with the 5-week-old $KKA^y$/Ta mice with no progress in the onset of the disease or the normal mice (Fig. 2). Similarly, the expression level of ech1 in the db/db mice was increased 2-fold or more compared with that in the normal mice.

Phlorizin is known as a resorption inhibitor of the glucose delivery through the uriniferous tubule in kidney. When phlorizin was administered at a dose of 100 mg/kg at an interval of 30 minutes three times in the abdominal cavity of $KKA^y$/Ta mice of age 15 weeks and the blood glucose level of the mice was back to the normal level, the activation of the ech1 expression level in the KKA$^y$/Ta mice of age 15 weeks did not change in the tissues 7 hours after the first administration of phlorizin. Therefore, it was considered that the expression of ech1 was not activated due to the change of the blood glucose level as the consequence of diabetic symptoms but the activation of the expression was one of the causative factors triggering diabetes mellitus.

Using the same DNA array as described above, the mRNA expression level for ech1 was assayed in each organ of a male normal mouse C57BL/6J of age 12 weeks.
Consequently, the expression of ech1 was prominent in fat, muscle, liver and kidney involving the PPARγ action, and also in heart and lung (Fig. 3). In view of the expression sites, additionally, this supports that ECHLP/Ech1 is the coupling factor of PPARγ.

(Example 5)

Detection of ECHLP regulatory action on the ligand-dependent transcription induction ability of PPARγ

The results described above indicated that ECHLP interacted with PPARγ through the ligands and was thereby involved in the main action (amelioration of glucose metabolism) and that the activated expression had some relation with symptoms of diabetes mellitus. Therefore, what kind of influences ECHLP had on the transcription induction activity of PPARγ was examined by reporter assay using a culture cell COS-1.

(1) Preparation of plasmid GAL-PPARγ for expression in animal cells

A chimera protein-encoding gene with cDNA encoding the ligand binding region of human PPARγ2 being fused to the C terminus of the DNA binding region (1-147 amino acids) of yeast Gal4 was integrated in the multicloning site in an animal cell expression vector pZeoSV (Invitrogen), to prepare an expression plasmid GAL-PPARγ. First, cutting out a DNA fragment encoding the DNA binding region of Gal4 from the plasmid pGBT9 (Clontech), using restriction enzymes HindIII and SmaI, the resulting DNA fragment was inserted at the site of the multicloning site of pZeoSV (abbreviated as pZeo-DB hereinafter). Then, cutting out a DNA fragment encoding the ligand binding region of PPARγ from the plasmid pDB-PPARγ, using KpnI and SmaI, the resulting DNA fragment was inserted in between KpnI and PvuII sites located in the multicloning site of pZeo-DB, to prepare an animal cell expression plasmid GAL-PPARγ.

(2) Preparation of a plasmid pcDNA-ECHLP for expression in animal cells

Using the primers of SEQ ID NOs: 12 and 13, cDNA fragment comprising 987 bp (base pairs) encoding the full-length ECHLP was obtained from the human skeletal muscle cDNA library (Clontech) by PCR. PCR was done at 98 °C (1 minute) and subsequently by carrying out a cycle of 98 °C (5 seconds)/55 °C (30 seconds) and 72 °C (3 minutes) repeatedly 35 times. The resulting cDNA fragment was inserted in pcDNA3.1/V5-HIS-TOPO vector (Invitrogen) by in vitro recombination by the TOPO cloning method (Invitrogen), to prepare a plasmid pcDNA-ECHLP for expression in animal cells. Herein, no termination codon was inserted for the ECHLP. Additionally, a primer was designed so that the vector-derived V5 epitope and HIS6 tag might be fused at the C terminus.

(3) Detection of ECHLP regulatory action on the ligand-dependent transcription induction ability of PPARγ

The culture cell COS-1 was cultured to a 70-% confluent state in a culture dish of 6-well culture plate (35-mm well diameter), to which 2 ml of the minimum essential culture medium DMEM (Gibco) supplemented with 10 % fetal bovine serum (Sigma) was preliminarily added to each well. By the calcium phosphate method (Graham L., et al., Virology, Vol. 52, p. 456, 1973, Naoko Arai, Gene Introduction and Expression/Analytical Method, p. 13-15, 1994), the cell was transiently cotransfected with the GAL-PPARγ (0.15 μg/well) and a reporter construct with the GAL4 binding region being repeatedly arranged in a number of eight upstream the luciferase gene (RE x 8-Luci; Shimokawa, et al., International Publication No. WO 99/04815) (0.8 μg/well), together with pcDNA-ECHLP (0.05-0.2 μg/well). After 2 μM of the PPARγ agonist or a test compound was added to the culture medium for 48-hour culturing, the culture medium was discarded and the cells were rinsed with a phosphate buffered saline (abbreviated as PBS hereinafter). Subsequently, a cell lysis solution (100 mM calcium phosphate, pH 7.8, 0.2 % Triton X-100) was added at 0.4 ml per each well, to make the cell lytic. To 100 μl of the solution of the lytic cell was added 100 μl of a luciferase substrate solution (Picker Gene), to assay the emission of light for 10 seconds using a chemiluminescence assay apparatus of Type AB-2100 (ATTO). Plasmid pCH110 (Amersham Pharmacia Biotech) with the luciferase reporter gene and concurrently with the β-galactosidase expression gene was cotransfected at 0.4 μg/well into the cell, to assay the β-galactosidase activity using a detection kit of β-galactosidase activity, namely Galacto-Light Plus™ system (Applied BioSystems) to express the activity in numerical figure. As the numerical figure was defined as the transfection efficiency of the introduced gene, the luciferase activity was corrected per each well.

As the results of the experiment, it was observed that the transcription induction activity of PPARγ in an agonist-dependent manner was distinctly inhibited, in a manner depending on the dose of the ECHLP expression plasmid transfected into the cell (Fig. 4). This apparently indicates that the occurrence of the ligand-dependent interaction between PPARγ and ECHLP suppressed the transcription induction activity of PPARγ. This fact highly coincides with the result showing that excess ECHLP/Ech1 was a causative factor of the disease in the diabetic model mice. In other words, it was considered that the occurrence of excess expression of ECHLP/Ech1 in the diabetic symptoms suppressed the transcription induction activity of PPARγ, so that insufficient expression of the downstream gene to be induced by PPARγ inhibited glucose metabolism.

ECHLP/Ech1 includes therein a structure speculated as a region for activating two enzyme types, namely enoyl-CoA hydratase and dienoyl-CoA isomerase working for fatty acid metabolism within the molecule (Filppula A, et al., Biol. Chem., Vol. 273, No.1: p. 349-355, 1988). Additionally, it has been known previously that inhibitors of enzymes for fatty acid metabolism reduce blood glucose levels in diabetic mice (Collier R, et al., Horm. Metab. Res., Vol.25, No.1: p.9-12, 1998). Based on the fact and the aforementioned finding that ECHLP has an action of suppressing the PPARγ activity, the presence of excess ECHLP suppresses glucose metabolism via PPARγ to promote the energy generation from lipid with the own enzyme activity for fatty acid metabolism. When ECHLP is reduced, ECHLP releases the PPARγ activity to shift the biological energy sources toward glucose metabolism. Thus, ECHLP was considered as a molecule responsible for the antagonistic regulation of glucose and fat metabolisms. When the amount of ECHLP interactive with PPARγ is reduced or when the suppressive action of PPARγ with the interactive ECHLP is inhibited, using ECHLP, biological energy sources may possibly be directed toward glucose metabolism to reduce blood glucose level. Using ECHLP, simultaneously, a compound with such action can readily be selected.

(Example 6)

Comparison of the amount of AOP2 protein in normal and diabetic model mice

Based on the finding, it was deduced that the interaction between AOP2 and PPARγ might be involved in the triggering of edema as an adverse action via a PPARγ agonist. Therefore, the contents of proteins in fats of diabetic model mouse KKA$^y$/Ta (Iwatsuka, et al., Endocrinol. Japon., Vol. 17, p. 23-35, 1970, Taketomi, et al., Horm. Metab. Res., Vol.7, p. 242-246, 16975) and normal mouse C57BL/6J were compared to each other, using the fluorescence-labeled two-dimensional difference electrophoresis (Unlu, et al., Electrophoresis, Vol. 18, p. 2071-2077, 1997, Tonge, et al., Proteomics, Vol. 1, p. 377-396, 2001). A group of proteins of which the contents differed by two-fold or more in the diabetic model mouse were analyzed by mass spectrometry, so as to identify the individual proteins.

(1) Resection of murine tissues

Male C57BL/6J and KKA$^y$/Ta mice were purchased from Clea Japan, Inc. The C57BL/6J mice were fed in a group with general diet until 12 weeks old. The KKA$^y$/Ta mice were singly fed with a high calories diet (CMF, Oriental Yeast Co., Ltd.) until 12 weeks old. Compared with the normal mice, it was confirmed that the KKA$^y$/Ta mice were at higher blood glucose levels and with larger body weights (KKA$^y$/Ta mice: blood glucose value of 514.2 ± 18.2 mg/dl; body weight of 49.9 ± 0.7 g). Subsequently, the two types of mice were anesthetized with diethyl ether, to resect the fat of epididymis. Immediately after resection, the epididymis was frozen in liquid nitrogen and stored at -80 °C.

(2) Preparation of protein samples

The frozen fat of epididymis was homogenized in a Tris buffer containing urea and an ampholytic detergent using a homogenizer ULTRA-TURRAX T-8 (Ika Labortechnik). According to the protocol attached by the manufacturer, centrifugation was done to obtain the supernatants of these samples for use as samples for two-dimensional electrophoresis.

(3) Two-dimensional electrophoresis

The protocol of Amersham Pharmacia Biotech was followed. By measuring the absorbance of each of the samples, the amount of the proteins contained therein was determined. Taking out an amount of a sample containing about 50 μg of proteins, the protein was labeled with individually different fluorescent dyes (Cy3 and Cy5, Amersham Pharmacia Biotech), and the labeled proteins were mixed together for first-dimensional isoelectric electrophoresis using an IPG strip (Amersham Pharmacia Biotech). Prior to second-dimensional electrophoresis, the IPG strip was equilibrated with a Tris buffer containing urea, sodium dodecylsulfate, glycerol, and dithiothreitol and further equilibrated with a Tris buffer containing urea dissolving iodo-acetamide therein, sodium dodecylsulfate, glycerol, and dithiothreitol. The second-dimensional electrophoresis was done using sodium dodecylsulfate polyacrylamide electrophoresis. The gel after completion of the second-dimensional electrophoresis was applied to a fluorescent imaging apparatus (Amersham Pharmacia Biotech) at excitation and detection wavelengths specific to the individual fluorescent dyes, to obtain the individual two-dimensional electrophoretic images. These two images were quantified using an analytical software (Amersham Pharmacia Biotech), to identify a spot with a difference in protein content by two-fold or more and cut out the spot with a spot picking apparatus (Amersham Pharmacia Biotech). Then, the protein was fragmented by the in-gel digestion method using trypsin (Schevchenko, et al., Analytical Chemistry, Vol. 68, p. 850-858, 1996), to recover a peptide mixture from the gel.

(4) Protein identification by mass spectrometry

The peptides in the resulting peptide mixture were separated by an acetonitrile gradient elution method on a capillary reverse-phase liquid chromatography column (0.075-mm diameter, 150-mm length, LC Packing) at a flow rate preset to about 200 nL per minute in the presence of 0.2 % formic acid. By a quadrupole ion trap mass spectrographic apparatus (Thermoquest) with an electrospray ion source directly connected to a liquid chromatography apparatus (Microme Bioresource), automatically, the product ion spectrum of each peptide was obtained by a method comprising a step of selecting the molecular ion of each peptide and measuring the product ion spectrum.

Individual product ion spectra of fragment peptides of a peptide in the fat of the epididymis of the KKA$^y$/Ta mice, as certified of the increase of the 2-fold or more content compared with the normal mice, were examined and compared with an analytical software Mascot (Matric Science), using a public protein database MSDB (Release 20010401). Consequently, the protein matched at partial amino acid sequences at its four positions with the murine AOP2 protein (AOP2/1-Cys Prx/non-selenium glutathione peroxidase; GenBank accession No.: AF004670, AF093852, Y12883). Thus, it was revealed that the protein was the murine AOP2 protein. Accordingly, this apparently indicates that the content of the AOP2 protein increases in diabetes mellitus.

(Example 7)

Comparison of AOP2 expression levels in tissues

Using the primers of SEQ ID NOs: 14 and 15, a 673-bp (base pairs) cDNA fragment encoding AOP2 as derived from the human cDNA library (Clontech) was amplified by PCR [using DNA polymerase (Pyrobest DNA polymerase; Takara Shuzo, Co., Ltd.) at 98 °C (1 minute) and subsequently by repeating a cycle of 98 °C (5 seconds) /55 °C (30 seconds) and 72 °C (3 minutes) 35 times], which was then detected by agarose gel electrophoresis. Consequently, the expression of AOP2 was distinct in fat, muscle, liver and kidney with PPARγ actions among the main organs, in addition to heart. This supports even based on the expressed sites that AOP2 is a transcriptional cofactor of PPARγ.

(Example 8)

Detection of AOP2 regulatory action on the ligand-dependent transcription induction ability of PPARγ

The results described above indicate that AOP2 interacts with PPARγ via a ligand to be involved in the triggering of edema and that the activation of the expression has a relation with diabetic symptoms. Therefore, a reporter assay using a culture cell COS-1 was done to examine what kind of influences AOP2 had on the transcription induction activity of PPARγ.

(1) Preparation of plasmid pcDNA-AOP2 for expression in animal cells

Using the primers of SEQ ID NOs: 14 and 15, a cDNA fragment comprising 673 bp (base pairs) encoding the full-length AOP2 was obtained from the human kidney cDNA library (Clontech) by PCR [using DNA polymerase (Pyrobest DNA polymerase; Takara Shuzo, Co., Ltd.) at 98 °C (1 minute) and subsequently by repeating a cycle of 98 °C (5 seconds)/55 °C (30 seconds) and 72 °C (3 minutes) 35 times]. This was inserted in pCDNA3.1/V5-His-TOPO vector (Invitrogen) by in vitro recombination by the TOPO cloning method (Invitrogen), to prepare a plasmid pcDNA-AOP2 for expression in animal cells. Herein, no termination codon was inserted for the AOP2. Additionally, a primer was designed so that the vector-derived V5 epitope and HIS6 tag might be fused at the C terminus.

(2) Detection of AOP2 regulatory action on the ligand-dependent transcription induction ability of PPARγ

The culture cell COS-1 was cultured to a 70-% confluent state in a culture dish of 6-well culture plate (35-mm well diameter), to which 2 ml of the minimum essential culture medium DMEM (Gibco) supplemented with 10 % fetal bovine serum (Sigma) was preliminarily added to each well. By the calcium phosphate method (Graham L., et al., Virology, Vol. 52, p. 456, 1973, Naoko Arai, Gene Introduction and Expression/Analytical Method, p. 13-15, 1994), the cell was transiently cotransfected with the GAL-PPARγ (0.15 μg/well) prepared in Example 5(1) and a reporter construct with the GAL4 binding region being repeatedly arranged in a number of eight upstream the luciferase gene (RE x 8-Luci; Shimokawa, et al., International Publication No. WO 99/04815) (0.8 μg/well) together with pcDNA-AOP2 (0.05-0.2 μg/well). After 2 mM of the PPARγ agonist GW7282 or a test compound was added to the culture medium for 48-hour culturing, the culture medium was discarded and the cells were rinsed with a phosphate buffered saline (abbreviated as PBS hereinafter). Subsequently, 0.4 ml of a cell lysis solution (100 mM calcium phosphate, pH 7.8, 0.2 % Triton X-100) was added to each well, to make the cell lytic. To 100 μl of the cell solution was added 100 μl of a luciferase substrate solution (Picker Gene), to assay the emission of light for 10 seconds using a chemiluminescence assay apparatus of Type AB-2100 (ATTO). Plasmid pCH110 (Amersham Pharmacia Biotech) with the luciferase reporter gene and concurrently with the β-galactosidase expression gene was cotransfected at 0.4 μg/well into the cell, to assay the β-galactosidase activity using a detection kit of β-galactosidase activity, namely Galacto-Light Plus™ system (Applied BioSystems) to express the activity in numerical figure. As the numerical figure was defined as the transfection efficiency of the introduced gene, the luciferase activity was corrected per each well.

As the results of the experiment, it was observed that the transcription induction activity of PPARγ in an agonist-dependent manner was distinctly inhibited, in a manner depending on the dose of AOP2 expression plasmid transfected into the cell (Fig. 5). This apparently indicates that the occurrence of the ligand-dependent interaction between PPARγ and ECHLP suppressed the transcription induction activity of PPARγ.

Based on the fact and the results described above that AOP2 was expressed in tissues including kidney and the amount of the AOP2 protein was increased in the diabetic model mice, it was believed that the amount of AOP2 in the cells in the diabetic symptoms was increased so that the following excess promotion of the PPARγ activity in specific tissues such as kidney caused the adverse action (edema).

Because AOP2 contains a peroxidase-like sequence within the molecule, AOP2 is called anti-oxidant protein 2 (GenBank Accession No. XM_001415) due to the homology in amino acid sequence. However, a report tells that as an actual physiological activity thereof, AOP2 functions as an acidic calcium-independent phospholipase A2 (Kim TS, et al., J. Biol. Chem., Vol.272, No.16, p.10981, 1997), while another report tells that the gene locus of the Aop2 protein is the etiological gene of polycystic nephropathia in mouse (LTW4/Aop2; lakoubova OA, et al., Genomics, Vol. 42, No. 3, p. 474-478, 1997). As described above, apparently, AOP2 has an action different from the molecular function speculated on the basis of the structure of the amino acid sequence. Therefore, the essential physiological function has not yet been identified. The finding by the inventors that AOP2 binds to PPARγ in a ligand-dependent manner and functions as a transcriptional cofactor thereof is a novel finding from the standpoint of the function of the molecule. The use of AOP2 enables discovering and eliminating PPARγ agonists triggering edema.

(Example 9)

Screening system for compounds selectively activating the main action via PPARγ

Based on the findings, a novel therapeutic agent of diabetes mellitus by ameliorating glucose metabolism and thereby making contributions to the recovery from diabetic symptoms can be screened by screening a compound inhibiting the interaction between ECHLP and PPARγ and the suppression of the ligand-dependent transcription promoting ability of PPARγ with ECHLP, which are detectable in the reporter assay system in Example 5. A therapeutic agent of diabetes mellitus by making contributions to the recovery from diabetic symptoms with no occurrence of edema as the adverse action can be screened by screening a substance never triggering the interaction between AOP2 and PPARγ and the activation of the ligand-dependent transcription promoting ability of PPARγ with AOP2, which are detectable in the reporter assay system in Example 8, among the test substances obtained thereby.

Test compounds can be screened in the assay system of the reporter activity, which is absolutely the same as in Examples 5 and 8. Nonetheless, the following reporter assay system was constructed so as to efficiently screen a greater number of test compounds.

The detailed method was the same as shown in Example 5. Under conditions involving the inhibition of the transcription activating ability of PPARγ with ECHLP in the presence of a PPAR agonist, compounds inhibiting the suppressive action of the transcription activating ability were screened by making an excess amount of a test compound concurrently exist for competition. Specifically, the culture cell COS-1 was cultured to a 70-% confluent state in a 6-well culture plate containing the minimum essential culture medium DMEM supplemented with 10 % fetal bovine serum.

By the calcium phosphate method, the cell was transiently cotransfected with the GAL-PPARγ (0.15 μg/well) and RE x 8-Luci (0.8 μg/well) together with pcDNA-ECHLP (0.15 μg/well). Under the condition that GW7282 as a PPARγ agonist was added to the culture to a final concentration of 0.1 μM, a test compound (10-1.0 μM) was added to the culture medium for 48-hour culturing in their concurrent presence thereof. Subsequently, the cell was rinsed with PBS, to which the cell lysis solution was added at 0.4 ml/well to each well, to make the cell lytic. 100 μl of the solution was transferred into a 96-well plate. According to the method of Example 5, then, the luciferase activity and the β-galactosidase activity were assayed to numerically express the activation of PPARγ. Based on the suppression of the ligand-dependent transcription induction ability of PPARγ (ratio of corrected luciferase activity value) via the ECHLP expression as observed under the condition involving the presence of a low concentration of GW7282 (0.1 μM) added as a PPARγ agonist, a compound inhibiting the transcription induction ability was screened under the condition of an excess amount of a test compound added at 10 or 0.1 μM. The standard for screening a substance inhibiting the suppression of the PPARγ transcription induction ability via ECHLP is preferably 10 μM or less, more preferably 1.0 μM or less on the basis of the intensity of the inhibitory activity (IC50). In this screening system, the aforementioned compound GI-262570 at 10 μM partially inhibited the suppression of the ligand (0.1 μM GW7282)-dependent PPARγ transcription induction ability with ECHLP (Fig.6a). Alternatively, the compound GL-100085 even at 10 μM never inhibited the suppression of the transcription induction ability, while GI-262570 was highly specific to the main action through PPARγ. Thus, GL-100085 could be actually selected as a compound with a low degree of the main action.

Continuously, the individual compounds (10-1.0 μM) selected in the screening system were singly added to a screening system where pcDNA-ECHLP in the above screening system was substituted with pcDNA-AOP2 (0.15 μg/well), so as to examine whether or not the promotion of the transcription induction ability of PPARγ with AOP2 in a manner depending on the test compound existed, by assaying the luciferase activity corrected in the same manner as described above. In the screening system, it was confirmed that the compounds GW7282 and GL-100085 at 1.0 to 10 μM promoted the transcription induction ability of PPARγ in the presence of AOP2 about 4 to 5-fold or 4- to 6-fold in a manner depending on the presence of each of the compounds. Meanwhile, the compound GI-262570 at any concentration of 1.0 μM and 10 μM promoted the transcription inducing ability, only about 3.5-fold (Fig. 6B). This enabled actual selection of GL-100085 as a compound highly specific to the adverse action through PPARγ in particular and GI-262570 as a compound with a relatively low specificity to the triggering of the adverse action.

(Example 10)

Comparison of expression levels of FLJ13111 in tissues

Using the primers of SEQ ID NOs: 18 and 19, a cDNA fragment encoding FLJ13111 was amplified from the human cDNA library (Clontech) by PCR [DNA polymerase (Pyrobest DNA polymerase; Takara Shuzo Co., Ltd.) was used for treatment at 98 °C (one minute) and subsequent treatment with a cycle of 98 °C (5 seconds)/55 °C (30 seconds) and 72 °C (3 minutes) 35 times], which was then detected by agarose gel electrophoresis. Consequently, FLJ13111 was distinctly expressed in muscle and liver where the PPARγ action could be observed among the main organs and additionally in mammary gland, lung, placenta, ovary, lymphocyte, and leukocyte. In kidney responsible for the triggering of edema with a PPARγ ligand, however, almost no expression was observed. This supported that FLJ13111 was a transcriptional cofactor of PPARγ.

(Example 11)

Detection of the regulatory action of FLJ13111 on the ligand-dependent transcription induction ability of PPARγ

The results of the yeast two-hybrid analysis indicated that FLJ13111 interacted with PPARγ via the ligands thereof. Therefore, it was examined by the reporter assay using the culture cell COS-1 what kind of influences FLJ13111 had on the transcription induction activity of PPARγ.

(1) Preparation of plasmid pcDNA-FLJ13111 for animal cell expression

Using the primers of SEQ ID NOs: 18 and 19, a cDNA fragment of 897 bp consisting of SEQ ID NO: 16 and encoding FLJ13111 was obtained from the human liver cDNA library (Clontech) by PCR [DNA polymerase (Pyrobest DNA polymerase; Takara Shuzo Co., Ltd.) was used for treatment at 98 °C (one minute) and for subsequent 35-time repetition of a cycle of 98 °C (5 seconds)/55 °C (30 seconds) and 72 °C (3 minutes)]. By the TOPO cloning method (Invitrogen), this cDNA fragment was inserted in pCDNA3.1 /V5-His-TOPO vector (Invitrogen) by in vitro recombination, for preparing a plasmid pcDNA-FLJ13111 for animal cell expression. Herein, no termination codon was inserted for the FLJ13111. A primer was designed so that the vector-derived V5 epitope and the His tag might be fused at the C terminus.

(2) Detection of the regulatory action of FLJ13111 on the ligand-dependent transcription induction ability of PPARγ

By the same method as in Example 5(3), pcDNA-ECHLP was substituted with pcDNA-FLJ13111 to thereby prepare a system for assaying the FLJ13111 action on the ligand-dependent transcription induction ability of PPARγ by the reporter assay. Additionally, 1 mM rosiglitazone was used as a PPARγ agonist. Rosiglitazone was added to assay the luciferase activity. Consequently, it was observed that the agonist-dependent transcription induction activity of PPARγ was promoted in a manner depending on the dose of the FLJ13111-expressing plasmid having transfected the cell (Fig.7). This apparently showed that the occurrence of the agonist-dependent interaction between PPARγ and FLJ13111 promoted the transcription induction activity of PPARγ.

This fact and almost no expression of FLJ13111 in kidney responsible for the triggering of edema with a PPARγ ligand suggested that the promotion of the PPARγ activity by FLJ13111 enhanced not the adverse action but the main action.

FLJ13111 is a protein with unknown function. Although the presence of a nuclear targeting sequence having been suggested to exist in cell nucleus and the presence of a possible N-glycosylation site, both within the molecule, could be speculated from the amino acid sequence, no additional information has existed yet suggesting the molecular function of FLJ13111 based on the amino acid sequence and structure. The finding of the inventors that FLJ13111 binds to PPARγ in a ligand-dependent manner and functions as its transcriptional cofactor is a novel finding about the function of the molecule. Utilizing the FLJ13111 can lead to the discovery of an agonist selective to the main action through PPARγ.

(Example 12)

Detection of the ligand selective action of FLJ13111 via PPARγ and screening system for compounds selectively activating the main action through PPARγ

Based on the findings, a novel therapeutic agent of diabetic mellitus by ameliorating glucose metabolism and making contributions to the recovery from diabetic symptoms can be screened, by screening a compound promoting the FLJ13111 action of activating the ligand-dependent transcription promoting ability of PPARγ, which can be detected with the reporter assay system in Example 11. From the test compounds thus obtained, additionally, a substance inhibiting the interaction of AOP2 and PPARγ and the activation of AOP2 on the ligand-dependent transcription promoting ability of PPARγ as detectable with the reporter assay in Example 8 can be screened, to screen for a therapeutic agent of diabetes mellitus making contributions to the recovery from diabetic symptoms with no occurrence of edema as the adverse action.

With absolutely the same reporter activity assay system as in Example 11, specifically, test compounds can be screened. For efficient screening of a greater number of test compounds, the reporter assay was done under the following preset conditions. Plasmid pCH110 (0.4 μg/well) with GAL-PPARγ (0.15 μg/well), a reporter construct (RE × 8-Luci; 0.8 μg/well) and the β-galactosidase expression gene was transiently cotransfected in COS-1 cell together with pcDNA-FLJ13111 (0.1 μg/well). To the resulting mixture in the culture medium was added a test compound to a final concentration of 10-0.1 μM for culturing for 48 hours, to assay the luciferase activity and the β-galactosidase activity and express the activation of PPARγ in numerical figure. Details of the transfection method and the method for assaying luciferase under conditions except for the conditions described above followed Examples 5 and 9. The test compounds were screened using as a marker the promotion of the transcription induction ability of PPARγ via FLJ13111 expression (ratio of corrected luciferase activity values) under individual conditions with added test compounds or without any test compound added. The standard for screening a substance promoting the PPARγ transcription induction ability with FLJ13111 was based on the effective concentration (ED50) being preferably 10 μM or less, more preferably 1.0 μM or less. In the screening system, rosiglitazone and pioglitazone both at 1 μM promoted the PPARγ transcription induction ability with FLJ13111. Meanwhile, the compound GL-100085 even at 10 μM did not promote the transcription induction ability; rosiglitazone and pioglitazone were highly specific to the main action through PPARγ; and GL-100085 could actually be selected as a compound with a low level of the main action.

(Example 13)

Assaying FLJ13111 expression level in normal mouse and diabetic model mouse

Based on the finding, it was deduced that the interaction between the FLJ13111 protein and PPARγ might be involved in the amelioration of glucose metabolism as the main action via a PPARγ agonist. Thus, the messenger RNA (mRNA) expression level of the mouse ortholog gene in the FLJ13111 gene was assayed in the muscle of the two types of diabetic model mice in Example 4, namely KKA$^y$/Ta and C57BL/KsJ-db/db, and was compared with the level in normal individual mice C57BL/6J and C57BL/KsJ-m+/m+. As to the expression level of the gene, the expression level of the FLJ13111 gene in accordance with the invention was assayed and corrected on the basis of the expression level of the glyceraldehyde 3-phosphate dehydrogenase (G3PDH) gene concurrently assayed. As the assay system, PRISM™ 7700 Sequence Detection System and SYBR Green PCR Master Mix (Applied BioSystems) were used. The fluorescence intensity of the dye SYBR Green I incorporated by double-stranded DNA amplified by PCR was subjected to real-time detection and assaying, to determine the expression level of the intended gene.

Specifically, the following procedures were used for the assay.

(1) Resection of mouse tissues and extraction of mRNA

By the same methods as in Example 4, the tissues and mRNA were prepared.

(2) Synthetic preparation of single-stranded cDNA

Reverse transcription from total RNA to single-stranded DNA was done in a system of 20 μl, using individually (1) 1 μg of RNA prepared above in (1) (muscle from mice of age 15 or 12 weeks old) and a reverse-transcription kit (Advantage™ RT-for-PCR kit; Clontech). After reverse-transcription, 180 μl of aseptic water was added to the resulting DNA, for storage at -20 °C.

(3) Preparation of PCR primer

Four oligonucleotides (SEQ ID NOs: 20 through 24) were designed as the PCR primers described in the item (4). A combination of SEQ ID NOs: 20 and 21 was used for the FLJ13111 gene, while a combination of SEQ ID NOs: 22 and 23 was used for the G3PDH gene.

(4) Assaying gene expression level

The real-time assay of the PCR amplification with PRISM™ 7700 Sequence Detection System was done in a 25-μl system according to the instruction manual. For each system, 5 μl of single-stranded cDNA, 12.5 μl of the 2 × SYBR Green reagent and 7.5 pmol of each of the primers were used. Herein, the cDNA prepared in (1) was used; the cDNA was diluted 30-fold for G3PDH; and the cDNA was diluted 10-fold for FLJ13111. For preparing a standard curve, an appropriate dilution of the murine genome DNA at 0.1 μg/μl (Clontech) was used at a volume of 5 μl, in place of the single-stranded cDNA. PCR was done at 50 °C for 10 minutes and continuously at 95 °C for 10 minutes, and subsequently by repeating a two-step process consisting of 95 °C for 15 seconds and 60 °C for 60 seconds 45 times.

The expression level of the murine FLJ13111 gene in each sample was corrected on the basis of the expression level of the G3PDH gene according to the following formula.

[Corrected FLJ13111 expression level] = [Expression level (raw data) of FLJ13111 gene]/ = [Expression level (raw data) of G3PDH]

For comparison of the expression level, the relative value thereof is shown in Fig.8, when the expression level of C57BL/6J mouse was defined as 100. As shown in Fig.8, apparently, the expression of the FLJ13111 gene was lowered markedly in the muscle of the diabetic model mice. Thus, it is considered that the reduction of the FLJ13111 expression level in the muscle triggers insulin resistance. Based on those described above, it is concluded that FLJ13111 is largely involved in insulin resistance.

Additionally, the results in this Example apparently indicated that diabetic symptoms can be diagnosed by assaying the expression level of FLJ13111.

(Example 14)

Identification of FLJ13111 promoter sequence, and screening system for a compound selectively activating the main action, utilizing the transcription induction activity of the sequence

Based on the finding in Example 11 above, apparently, the increase of the existing FLJ13111 enhances the action of a PPARγ ligand with a high effect on the triggering of the main action. Based on the fact, the possibility of ameliorating insulin resistance is anticipated by positively adjusting the FLJ13111 expression level from the FLJ13111 gene. However, not any promoter sequence responsible for the regulation of the expression of the FLJ13111 is known. Therefore, attempts were made to obtain an FLJ13111 promoter sequence. First, a pair of primers of SEQ ID NOs: 24 and 25 were designed. Using these primers under the same PCR conditions as described in Example 11 (1), the amplification of the FLJ13111 promoter sequence was attempted. Finally, success was made in the amplification of a cDNA fragment of about 1.8 kbp. By the same method as in the Example, the nucleotide sequence of the fragment was determined. Thus, it was found that the fragment was the polynucleotide of SEQ ID NO: 26, containing a part of the coding sequence of the FLJ13111 gene at the 3' terminus. It was determined by the following method whether or not the polynucleotide sequence had a promoter activity regulating the expression of FLJ13111. Inserting the nucleotide at the multicloning site of pGL3-Basic Vector (Promega) as a luciferase reporter vector, using restriction enzymes BglII and HindIII, a reporter plasmid named pGL3-FLJ13111p was prepared. The plasmid was transfected in COS-1 cell. By comparison with a case of transfection in pGL3-Basic Vector (vacant vector) never carrying the polynucleotide, the activity of the polynucleotide as promoter to induce the expression was assayed, using the luciferase activity as a marker. The correction of the transfection efficiency into cells and the luciferase assay were precisely the same as used in the method described in Example 5(3).
Consequently, a significant promoter activity depending on the presence of the polynucleotide was confirmed as shown in Fig.9. Further, it was revealed that the promoter activity was activated when pioglitazone (0.1 μM) as a PPARγ ligand was added to the transfected cell. At this experiment, additionally, the co-transfection with pcDNA-FLJ13111 as the FLJ13111 expression plasmid lowered the promoter activity of the polynucleotide as shown in Fig.9. These facts show that the cloned polynucleotide sequence contained the promoter sequence regulating the FLJ13111 expression, and the promoter was positively regulated with PPARγ ligands reducing insulin resistance such as pioglitazone and was negatively regulated with FLJ13111 itself. Based on this, it is deduced that not only FLJ13111 activates the activity of PPARγ via a ligand but also the expression level of FLJ13111 per se is activated with a PPARγ ligand known to have an effect of reducing insulin resistance, both of which synergistically act for reducing insulin resistance.
Based on the findings, the assay of the FLJ13111 promoter in the Example can be utilized for screening a PPARγ ligand or a drug ameliorating insulin resistance with no use of the PPARγ protein.

(Example 15)

Assaying adipocyte differentiation in cells expressing ECHLP excessively

As described above, it was shown that the ECHLP protein bound to PPARγ in a manner depending on the presence of a PPARγ ligand to suppress the transcription induction activity of PPARγ. Because the expression level of ECHLP is increased in diabetic symptoms, further, the excess expression thereof triggers insulin resistance via the suppression of the PPARγ activity to cause Type 2 diabetes mellitus. Meanwhile, it is known that PPARγ promotes the adipocyte differentiation by the induction of the transcription activity depending on the ligand, so that differentiated adipocytes incorporate blood glucose and thereby, glucose metabolism is ameliorated and insulin resistance is reduced. It was examined at the following experiments whether or not overexpression of ECHLP in cells had an actual influence on the adipocyte differentiation, which had a relation with insulin resistance.

(1) Establishment of L1 cell expressing ECHLP excessively

So as to recombine ECHLP with the FLAG sequence consisting of DYKDDDDK being added at the C terminus in a retrovirus vector pCLNCX (Immunogenetics), a BamHI-NotI fragment of about 1 kb was prepared from the pcDNA-ECHLP plasmid, using restriction enzymes. So as to prepare a DNA fragment including a NotI site-FLAG sequence-XbaI site, further, two synthetic oligo DNAs of SEQ ID NOs: 27 and 28 were mixed together, heated and annealed to prepare double-stranded DNA fragments. These DNA fragments were recombined together at the BamHI and XbaI sites of pCLNCX, to prepare a pCLNCX-ECHLP-Flag vector. The pCLNCX-ECHLP-FLAG vector and the pCL-Eco vector (Immunogen) were both introduced into the 293 cell by the calcium phosphate method for the transfection. 24 and 48 hours after the transduction, the recombinant virus in the culture supernatant was recovered. The virus was diluted 2-fold with a fresh cell culture broth [Minimum essential culture medium DMEM (Gibco)] not yet used, to which polybrene (Sigma) was added to a final concentration of 8 μg/ml, to make the virus infect a murine cultured precursor adipocyte 3T3-L1 (ATCC). 48 hours after the infection and thereafter, the virus-infected cell was screened for with 1.5 mg/ml G418 (Nakarai), to establish an L1 cell expressing ECHLP-FLAG in a stable fashion. As a control, a cell infected with pCLNCX vector (vacant vector) was also prepared. The expression of ECHLP-FLAG in the established cell was confirmed by Western blotting using anti-FLAG M2 antibody (Sigma). Specifically, 10 μl of 2 × SDS sample buffer (125 mM Tris-HCl, pH 6.8, 3 % sodium laurylsulfate, 20 % glycerin, 0.14 M β-mercaptoethanol, 0.02 % bromophenol blue) was added to 10 μl of the solution of the lytic cell expressing ECHLP-FLAG, for treatment at 100 °C for 2 minutes, for 10 % SDS polyacrylamide gel electrophoresis, to separate the protein contained in the sample. Using a semi-dry type blotting apparatus (BioRad), the protein in the polyacrylamide was transferred onto a nitrocellulose membrane, to detect the ECHLP protein on the nitrocellulose by general procedures according to Western blotting. A monoclonal antibody recognizing the FLAG epitope fused at the C terminus of ECHLP was used as a primary antibody, while the rabbit IgG-HRP fused antibody (BioRad) was used as a secondary antibody. Consequently, it was confirmed that the protein representing the ECHLP-FLAG fused protein was detected in a manner depending on the cell introduction of the ECHLP-FLAG expression vector.

(2) Adipocyte Differentiation with pioglitazone

The vacant vector-infected L1 cell or the ECHLP-infected L1 cell established by the method was cultured at $10^4$ cells/well in a 96-well plate. 48 hours later and thereafter, the cell was differentiated and induced into adipocyte, using insulin (1 μg/ml) and pioglitazone (0.1-3 μM). Concerning the degree of the adipocyte differentiation, the content of triglyceride incorporated in the cell was used as a marker. The cell on day 7 after the start of the induction of the differentiation was used to assay the content of triglyceride.

(3) Assaying intracellular triglyceride

The cell in the 2 wells was dissolved in 40 μl of 0.1 % SDS solution, to which 1 ml of a triglyceride assay reagent (Triglyceride G-test Wako, Wako Pure Chemical Industries Ltd.) was added, for heating at 37 °C for 10 minutes. The absorbance of the reaction solution at a wavelength of 505 nm (OD505) was measured. As shown in Fig.10, consequently, it was observed that the intracellular triglyceride increased in the control cell (vacant vector-infected L1 cell) in a manner dependent on the dose of pioglitazone (0.1-3 μM), indicating the adipocyte differentiation. Alternatively, the triglyceride increase induced by pioglitazone (0.1-3 μM) at any of the dose in the cell expressing ECHLP excessively (ECHLP-infected L1 cell) was suppressed to 43 to 57 % of the increment in the control cell.

The suppression of the adipocyte differentiation decreases the total amount of glucose incorporation induced by adipocyte. Thus, the above results clearly show that the excess expression of ECHLP suppresses the adipocyte differentiation, so that it works as a causative factor of type 2 diabetes mellitus.

(Example 16)

Identification of ligand selectively inducing the binding between FLJ13111 and PPARγ

Screening by the same reporter assay as shown above in Example 12 was done. Consequently, a compound XF promoting the transcription induction activity of PPARγ was obtained (Fig. 11). It was shown that the titer thereof at 10 μM was approximately comparable to 0.1 μM of pioglitazone. Further, it was found that the promotion action of the compound XF on the transcription activating ability of PPARγ was activated by excess expression of FLJ13111 (0.1 μg/well) in the same fashion as in the case of pioglitazone.

In the system for assaying the ligand-dependent binding of PPARγ to FLJ13111 by the yeast two-hybrid method as shown in Example 2, the compound XF was experimentally used under the same conditions in place of GW7282. It was found that the compound XF never induced the binding of PPARγ with the aforementioned proteins SRC-1, ECHLP and AOP2, but induced only the binding of PPARγ with FLJ13111.

(Example 17)

Assaying the expression level of sodium-potassium ATPase with FLJ13111-selective PPARγ ligand

Edema caused by PPARγ ligand is induced by the increase of circulating plasma volume, which is known to occur in relation with the increase of the expression level of the sodium-potassium ATPase in renal cell. Therefore, it was examined whether or not the compound XF influenced the expression level of the sodium-potassium ATPase in renal cell, which has a relation with triggering edema.

Specifically, feline renal epithelial cell MDCK was cultured at $1.5 \times 10^5$ cells/well in a 24-well culture plate, using the minimum essential culture medium DMEM (Gibco) supplemented with 10 % fetal bovine serum (Sigma) at 37 °C for 48 hours. The solvent (dimethylsulfoxide) alone or pioglitazone (to a final concentration of 0.1 to 10 μM) or the test compound XF (to a final concentration of 0.1 to 10 μM) was added to the liquid culture, for culturing for another 6 hours. After the cell was rinsed two times with 1 ml of an assay buffer (3 mM $MgSO_4$, 3 mM $Na_2HPO_4$, 10 mM Tris-HCl, 250 mM sucrose), 200 μl of an assay buffer containing $^3$H-ouabain (74 Bq/μl; Amersham Bioscience) and 2 μM ouabain was added to the cell, which was then left to stand at 37 °C for 2 hours. The radioactivity bound under the conditions was defined as total binding. For assaying non-specific binding, additionally, $^3$H-ouabain (74 Bq/μl) and 1 mM ouabain were used. After removing the reaction solution under aspiration, the cell was rinsed three times with 1 ml of an ice-cold assay buffer, to solubilize the cell with an aqueous 0.5 N NaOH solution (250 μl). After the resulting solution was made neutral with an equal volume of aqueous 0.5N HCl solution, 5 ml of a liquid scintillator was added to count the radioactivity with a liquid scintillation counter. The specific binding with $^3$H-ouabain was determined by subtracting the non-specific binding value from the total binding value, to determine the expression level of the sodium-potassium ATPase.

As shown in Fig.12, the results are that pioglitazone added at 0.1 μM exerted a significant action of enhancing the expression level of the sodium-potassium ATPase, compared with the control cell with the solvent added alone. Meanwhile, the compound XF never enhanced the expression level of the sodium-potassium ATPase even when the compound XF was added at a 10-μM concentration at which the XF showed almost the same effect on the PPARγ transcription activation as pioglitazone did. In other words, it was revealed that the compound XF as an FLJ13111-selective PPARγ ligand never induced the increase of the expression level of the sodium-potassium ATPase triggering edema. Thus, it was shown that the compound XF was never involved in triggering edema.

(Example 18)

Assay of the differentiation ability into adipocyte via FLJ13111-selective PPARγ ligand

The same method as in Example 15 was used to examine whether or not the addition of the compound XF had an influence on the adipocyte differentiation, which had a relation with the reduction of insulin resistance. Specifically, the compound XF was added (1.0-10.0 μM) to the precursor adipocyte 3T3-L1 (ATCC) cultured in mouse. Using the amount of triglyceride on day 7 in the cell as a marker, the differentiation level into adipocyte was measured. Consequently, it was observed that the compound XF increased the triglyceride amount by about 20 %, compared with the cell with a solvent added alone.

The promotion of the adipocyte differentiation increases the total glucose uptake for which adipocyte is responsible, so that insulin resistance is ameliorated. Thus the results above indicate that the compound XF has an action of ameliorating insulin resistance with no induction of edema.

The results described above clearly show that FLJ13111 can be used for screening a compound selectively having the main action but never causing the adverse action, namely a drug ameliorating insulin resistance.

Industrial Applicability

By the yeast two-hybrid screening method to be carried out in the presence of ligands in accordance with the invention, a protein interactive with PPARγ in a ligand-dependent manner working as a useful tool for screening a drug ameliorating insulin resistance without the adverse action can be screened. The use of the main action ligand-dependent PPAR-binding molecule ECHLP, the main action ligand-selective PPARy-interactive factor FLJ13111, and the adverse action ligand-dependent PPAR-binding molecule AOP2 as obtained by the methods enables the identification and screening of a compound having selectively the main action but never causing the adverse action. The substance selected with the screening system is useful as a candidate substance as a drug for ameliorating insulin resistance.

Sequence Listing Free Text

In the numerical title [223] in the Sequence Listing below, the [Artificial Sequence] is described.
Specifically, individual nucleotide sequences of SEQ ID NOs: 9, 10, 11, 13, 24, 25, 27 and 28 in the Sequence Listing are primer sequences artificially prepared synthetically.

The invention has been described above with reference to the specific embodiments. Variations and modifications thereof obvious to persons skilled in the art are also encompassed within the scope of the invention.

## Claims

1. A method for screening a protein interactive with PPARγ in a ligand-dependent manner, utilizing a yeast two-hybrid system in the presence of a PPAR ligand with a high potency of triggering the action ameliorating glucose metabolism, wherein a polynucleotide encoding a region containing at least the position 204 to position 505 of the PPARγ protein represented by SEQ ID NO: 2 is used as bait and a cDNA library is used as prey.

2. A method for screening a protein interactive with PPARγ in a ligand-dependent manner, utilizing a yeast two-hybrid system in the presence of a PPAR ligand with a high potency of triggering edema, wherein a polynucleotide encoding a region containing at least the position 204 to position 505 of the PPARγ protein represented by SEQ ID NO: 2 is used as bait and a cDNA library is used as prey.

3. A cell transformed by i) a polynucleotide encoding a polypeptide consisting of an amino acid sequence of SEQ ID NO: 4 or a polynucleotide encoding a polypeptide comprising an amino acid sequence represented by SEQ ID NO: 4 wherein 1 to 10 amino acids therein are deleted, substituted and/or inserted and also interacting with PPAR in a ligand-dependent manner, ii) a gene encoding a fusion protein comprising at least the ligand binding region of the PPAR protein represented by SEQ ID NO: 2 or 6 and the DNA binding region of a transcription factor, and iii) a reporter gene fused to a response element to which said DNA binding region of the transcription factor is capable of binding; or a cell transformed by i) a polynucleotide encoding a polypeptide consisting of an amino acid sequence of SEQ ID NO: 4 or a polynucleotide encoding a polypeptide comprising an amino acid sequence represented by SEQ ID NO: 4 wherein 1 to 10 amino acids therein are deleted, substituted and/or inserted and additionally interacting with PPAR in a ligand-dependent manner and ii) a reporter gene fused to a response element to which the DNA binding region of the PPAR protein represented by SEQ ID NO: 2 or 6 is capable of binding, said cell expressing a) a polypeptide consisting of an amino acid sequence of SEQ ID NO: 4 or a polypeptide comprising an amino acid sequence represented by SEQ ID NO: 4 wherein 1 to 10 amino acids therein are deleted, substituted and/or inserted and interacting with PPAR in a ligand-dependent manner and b) the PPAR protein represented by SEQ ID NO: 2 or 6.

4. A cell transformed by i) a polynucleotide encoding a polypeptide consisting of an amino acid sequence of SEQ ID NO: 8 or a polynucleotide encoding a polypeptide comprising an amino acid sequence represented by SEQ ID NO: 8 wherein 1 to 10 amino acids therein are deleted, substituted and/or inserted and additionally interacting with PPAR in a ligand-dependent manner, ii) a gene encoding a fusion protein comprising at least the ligand binding region of the PPAR protein represented by SEQ ID NO: 2 or 6 and the DNA binding region of a transcription factor, and iii) a reporter gene fused to a response element to which said DNA binding region of the transcription factor is capable of binding,; or a cell transformed by i) a polynucleotide encoding a polypeptide consisting of an amino acid sequence of SEQ ID NO: 8 or a polynucleotide encoding a polypeptide comprising an amino acid sequence represented by SEQ ID NO: 8 wherein 1 to 10 amino acids therein are deleted, substituted and/or inserted and additionally interacting with PPAR in a ligand-dependent manner and ii) a reporter gene fused to a response element to which the PPAR protein represented by SEQ ID NO: 2 or 6 is capable of binding, said cell expressing a) a polypeptide consisting of an amino acid sequence of SEQ ID NO: 8 or a polypeptide comprising an amino acid sequence represented by SEQ ID NO: 8 wherein 1 to 10 amino acids therein are deleted, substituted and/or inserted and interacting with PPAR in a ligand-dependent manner, and b) the PPAR protein represented by SEQ ID NO: 2 or 6.

**5.** A cell according to claim 3 or 4, wherein the transcription factor is the GAL4 protein of yeast.

**6.** A cell according to claim 3 or 4, wherein the reporter gene is luciferase gene.

**7.** A method for detecting whether or not a test substance promotes the action of ameliorating glucose metabolism via PPAR, comprising i) a step of allowing a cell according to claim 3, a PPAR ligand and a test substance in contact with each other, and ii) a step of analyzing the change of the ligand-dependent interaction or the change of the transcriptional activity induced by ligand-activated PPAR, using the expression of a reporter gene as a marker.

**8.** A method for screening a drug ameliorating insulin resistance, comprising i) a step of allowing a cell according to claim 3, a PPAR ligand and a test substance in contact with each other, and ii) a step of analyzing the change of the ligand-dependent interaction or the change of the transcriptional activity induced by ligand-activated PPAR, using the expression of a reporter gene as a marker.

**9.** A method for screening according to claim 8, wherein the drug ameliorating insulin resistance is a drug ameliorating glucose metabolism.

**10.** A method for detecting whether or not a test substance promotes the activity triggering edema via PPAR, comprising i) a step of allowing a test substance in contact with a cell according to claim 4, and ii) a step of analyzing the change of the interaction due to the test substance or the change of the transcriptional activity induced via PPAR due to the test substance using the expression of a reporter gene as a marker.

**11.** A method for screening a drug ameliorating insulin resistance with no activity of triggering edema, comprising i) a step of allowing a test substance in contact with a cell according to claim 4, ii) a step of analyzing the change of the interaction due to the test substance or the change of the transcriptional activity induced via PPAR due to the test substance, using the expression of a reporter gene as a marker; and iii) a step of selecting a test substance not enhancing the reporter activity.

**12.** A method for screening according to claim 11, wherein the drug ameliorating insulin resistance is a drug ameliorating glucose metabolism.

**13.** A cell transformed by i) a polynucleotide encoding a polypeptide consisting of an amino acid sequence of SEQ ID NO: 17 or a polynucleotide encoding a polypeptide comprising an amino acid sequence represented by SEQ ID NO: 17 wherein 1 to 10 amino acids therein are deleted, substituted and/or inserted and also interacting with PPAR in a ligand-dependent manner, ii) a gene encoding a fusion protein comprising at least the ligand binding region of the PPAR protein represented by SEQ ID NO: 2 or 6 and the DNA binding region of a transcription factor, and iii) a reporter gene fused to a response element to which said DNA binding region of the transcription factor is capable of binding; or
a cell transformed by i) a polynucleotide encoding a polypeptide consisting of an amino acid sequence of SEQ ID NO: 17 or a polynucleotide encoding a polypeptide comprising an amino acid sequence represented by SEQ ID NO: 17 wherein 1 to 10 amino acids therein are deleted, substituted and/or inserted and additionally interacting with PPAR in a ligand-dependent manner and ii) a reporter gene fused to a response element to which the PPAR protein represented by SEQ ID NO: 2 or 6 is capable of binding, said cell expressing a) a polypeptide consisting of an amino acid sequence of SEQ ID NO: 17 or a polypeptide comprising an amino acid sequence represented by SEQ ID NO: 17 wherein 1 to 10 amino acids therein are deleted, substituted and/or inserted and interacting with PPAR in a ligand-dependent manner, and b) the PPAR protein represented by SEQ ID NO: 2 or 6.

**14.** A method for detecting whether or not a test substance promotes the action of ameliorating glucose metabolism via PPAR, comprising i) a step of allowing a test substance in contact with a cell according to claim 13, and ii) a step of analyzing the change of the interaction due to the test substance or the change of the transcriptional activity induced via PPAR due to the test substance, using the expression of a reporter gene as a marker.

**15.** A method for screening a drug ameliorating insulin resistance, comprising i) a step of allowing a cell according to claim 13 in contact with a test substance, and ii) a step of analyzing the change of the interaction due to the test substance or the change of the transcriptional activity induced via PPAR due to the test substance, using the expression of a reporter gene as a marker.

**16.** A method for screening according to claim 15, wherein the drug ameliorating insulin resistance is a drug ameliorating glucose metabolism.

**17.** A method for screening a drug ameliorating insulin resistance, comprising i) a step of allowing a test substance in contact with a cell transformed with a reporter gene fused to a polynucleotide consisting of a nucleotide sequence of SEQ ID NO: 26 or a polynucleotide comprising a nucleotide sequence represented by SEQ ID NO: 26 wherein 1 to 10 bases therein are deleted, substituted and/or inserted and also having a transcription promoter activity, and ii) a step of analyzing the change of the activity for transcriptional induction due to the test substance, using the expression of a reporter gene as a marker.

**18.** A method for screening according to claim 17, wherein the reporter gene is the luciferase gene.

**19.** A method for producing a pharmaceutical composition for ameliorating insulin resistance, comprising a screening step using a screening method according to claim 8, 11, 15 and/or 17 and a formulation step using a substance obtained by the screening.

Fig. 1

(a)

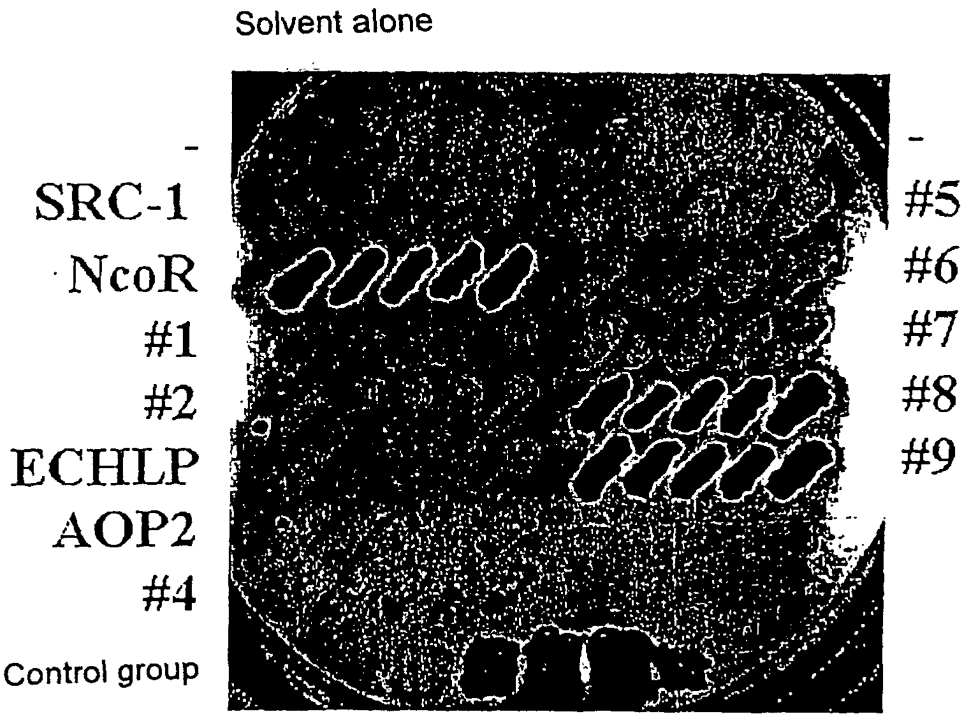
Solvent alone
-
SRC-1
NcoR
#1
#2
ECHLP
AOP2
#4
Control group
-
#5
#6
#7
#8
#9

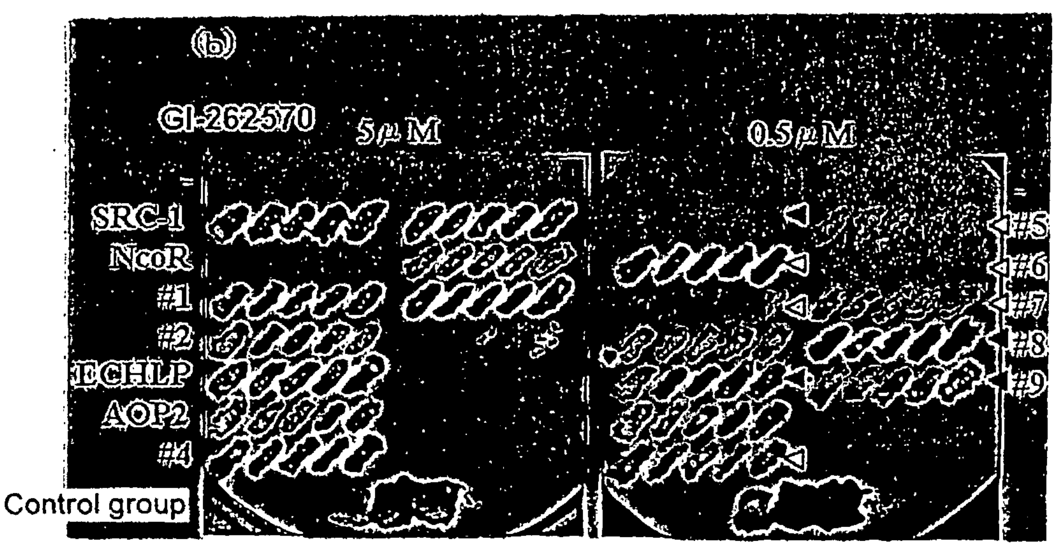
(b)
GI-262570
5μM
0.5μM
SRC-1
NcoR
#1
#2
ECHLP
AOP2
#4
Control group
#5
#6
#7
#8
#9

(c)

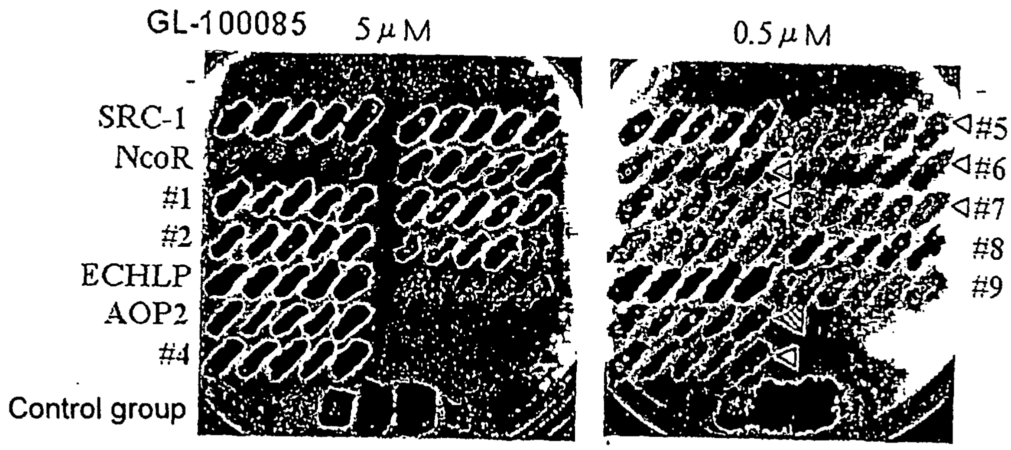
GL-100085
5μM
0.5μM
-
SRC-1
NcoR
#1
#2
ECHLP
AOP2
#4
Control group
-
#5
#6
#7
#8
#9

Fig. 2

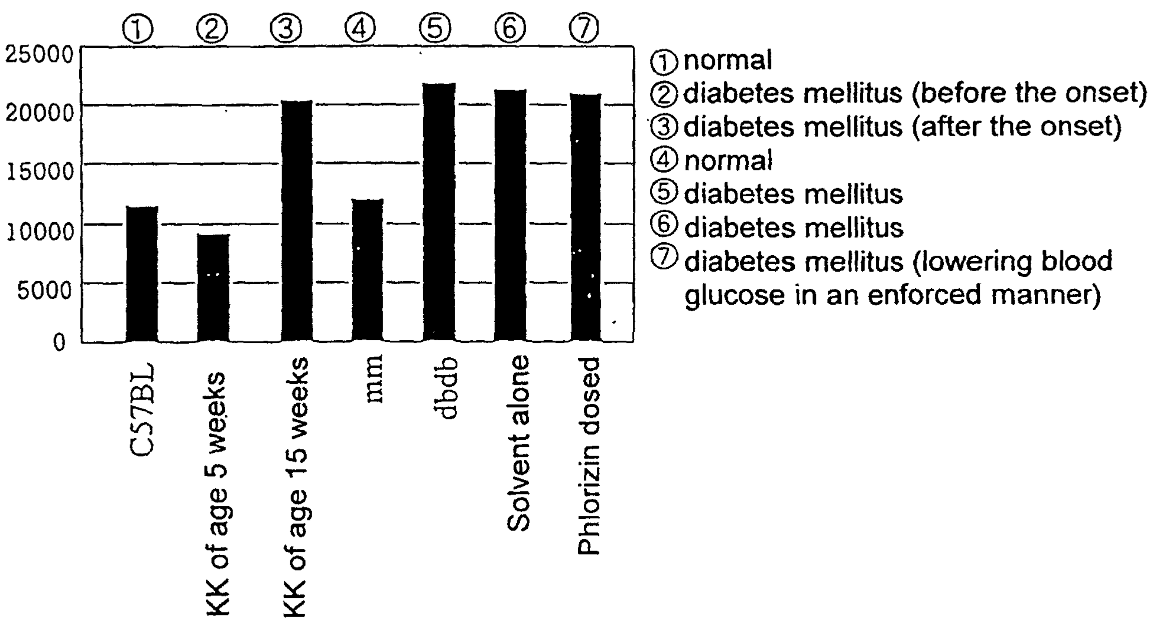
① ② ③ ④ ⑤ ⑥ ⑦
25000
20000
15000
10000
5000
0
C57BL
KK of age 5 weeks
KK of age 15 weeks
mm
dbdb
Solvent alone
Phlorizin dosed
① normal
② diabetes mellitus (before the onset)
③ diabetes mellitus (after the onset)
④ normal
⑤ diabetes mellitus
⑥ diabetes mellitus
⑦ diabetes mellitus (lowering blood glucose in an enforced manner)

Fig. 3

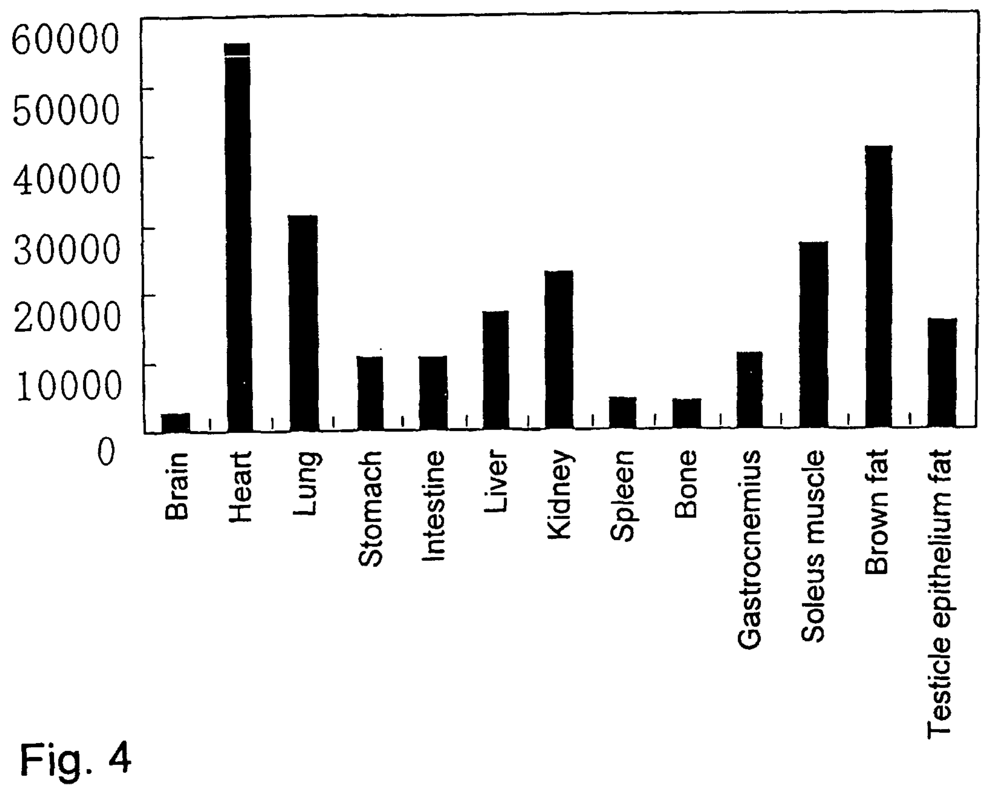
60000
50000
40000
30000
20000
10000
0
Brain
Heart
Lung
Stomach
Intestine
Liver
Kidney
Spleen
Bone
Gastrocnemius
Soleus muscle
Brown fat
Testicle epithelium fat

Fig. 4

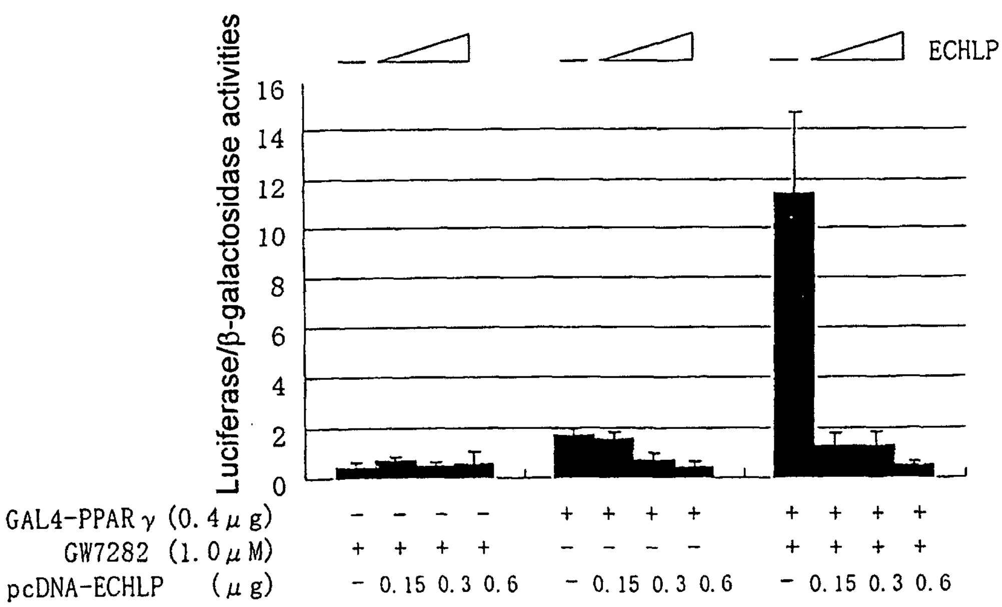
Luciferase/β-galactosidase activities
ECHLP
16
14
12
10
8
6
4
2
0
GAL4-PPARγ (0.4μg) - - - - + + + + + + + +
GW7282 (1.0μM) + + + + - - - - + + + +
pcDNA-ECHLP (μg) - 0.15 0.3 0.6 - 0.15 0.3 0.6 - 0.15 0.3 0.6

Fig. 5

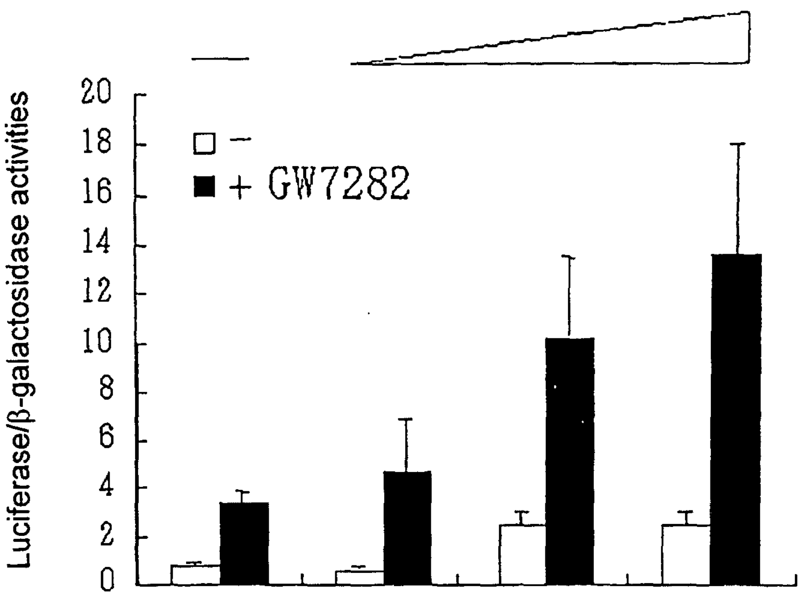


| | | | | | |
|---|---|---|---|---|---|
| GAL4-PPARγ | (0.4 μg) | + | + | + | + |
| GW7282 | (1.0 μM) | + | + | + | + |
| pcDNA-AOP2 | (μg) | − | 0.15 | 0.3 | 0.6 |

Fig. 6

(a)

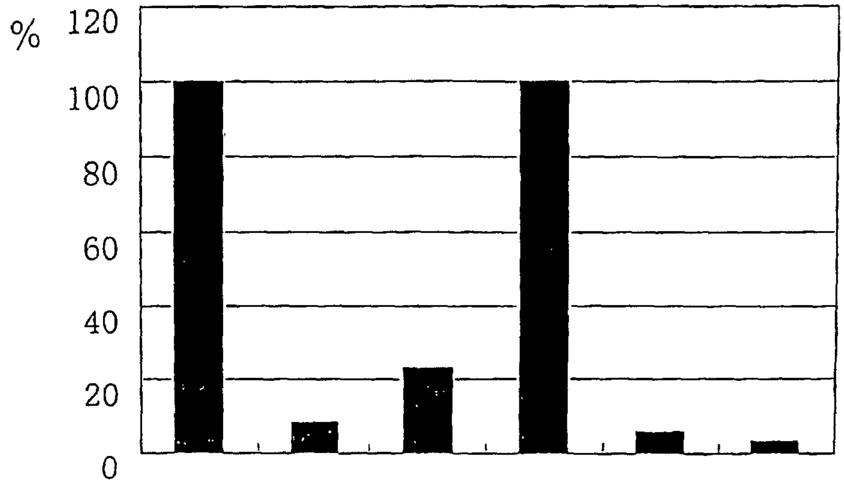


| | | | | | | |
|---|---|---|---|---|---|---|
| GAL4-PPARγ (0.4 μg) | + | + | + | + | + | + |
| ECHLP (μg) | − | 0.6 | 0.6 | − | 0.6 | 0.6 |
| GW7282 (0.1 μM) | + | + | + | + | + | + |
| GI-262570 (μM) | − | 1.0 | 10 | − | − | − |
| GL-100085 (μM) | − | − | − | − | 1.0 | 10 |

(b)

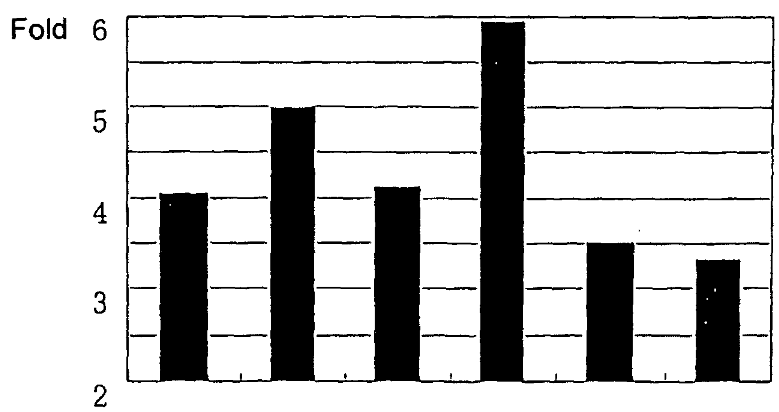


| | | | | | | |
|---|---|---|---|---|---|---|
| GAL4-PPARγ (0.4 μg) | + | + | + | + | + | + |
| AOP2 (μg) | 0.6 | 0.6 | 0.6 | 0.6 | 0.6 | 0.6 |
| GW7282 (0.1 μM) | 1.0 | 10 | − | − | − | − |
| GL-100085 (μM) | − | − | 1.0 | 10 | − | − |
| GI-262570 (μM) | − | − | − | − | 1.0 | 10 |

Fig. 7

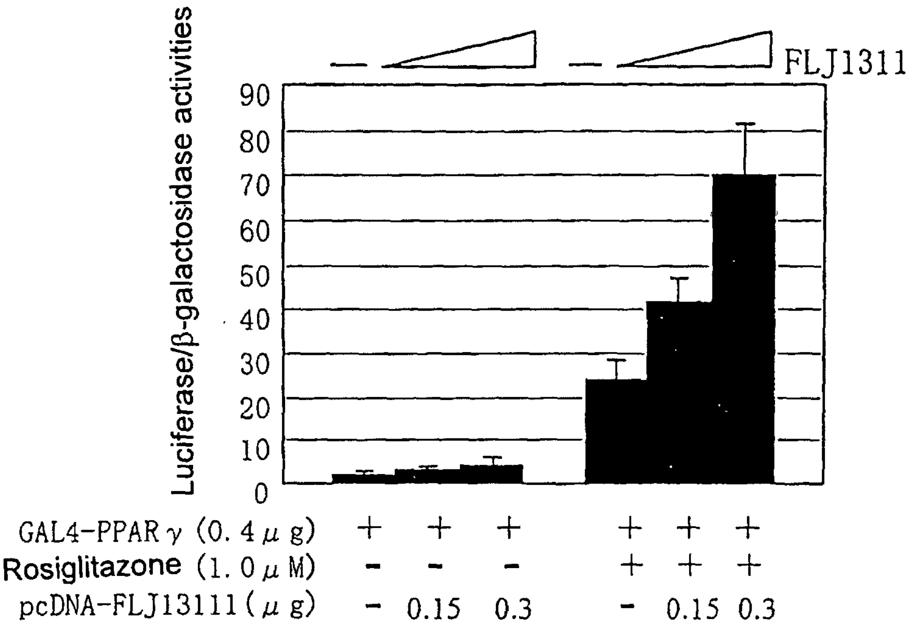
FLJ1311
Luciferase/β-galactosidase activities
90
80
70
60
50
40
30
20
10
0
GAL4-PPARγ (0.4 μg) + + + + + +
Rosiglitazone (1.0 μM) - - - + + +
pcDNA-FLJ13111 (μg) - 0.15 0.3 - 0.15 0.3

Fig. 8

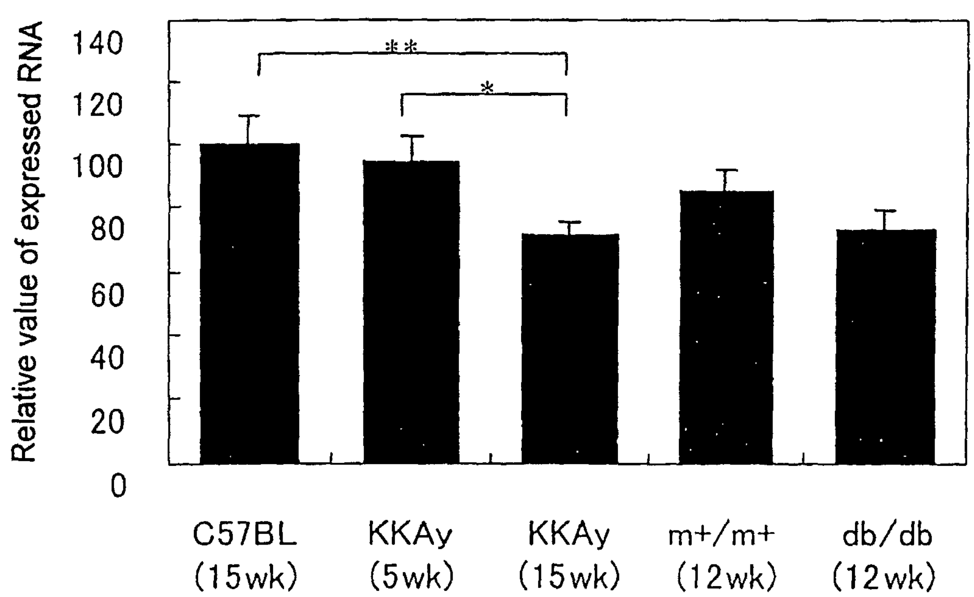
Relative value of expressed RNA
140
120
100
80
60
40
20
0
**
*
C57BL (15wk)
KKAy (5wk)
KKAy (15wk)
m+/m+ (12wk)
db/db (12wk)

Fig. 9

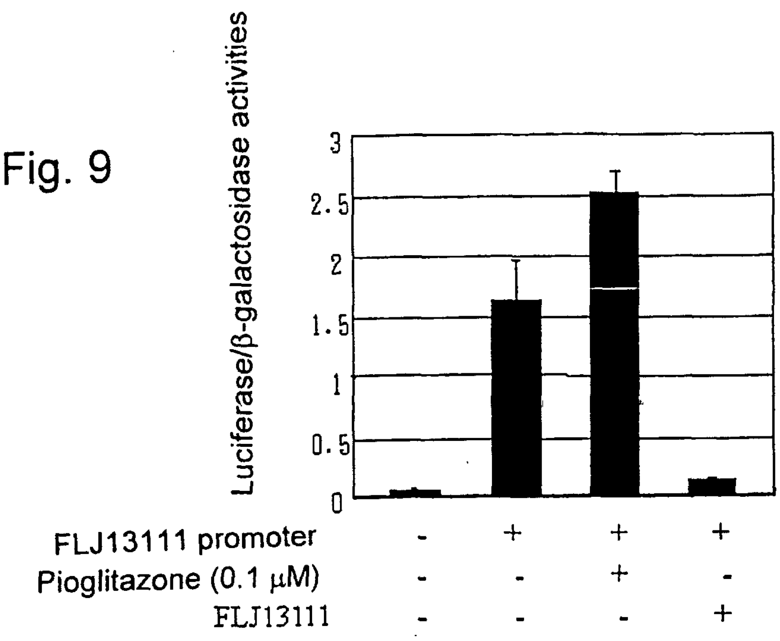


Fig. 10

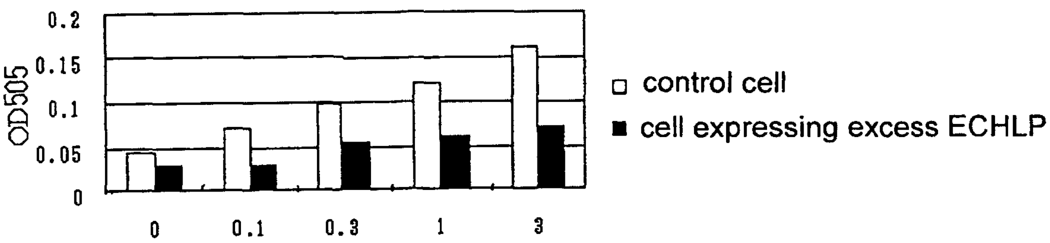


Fig. 11

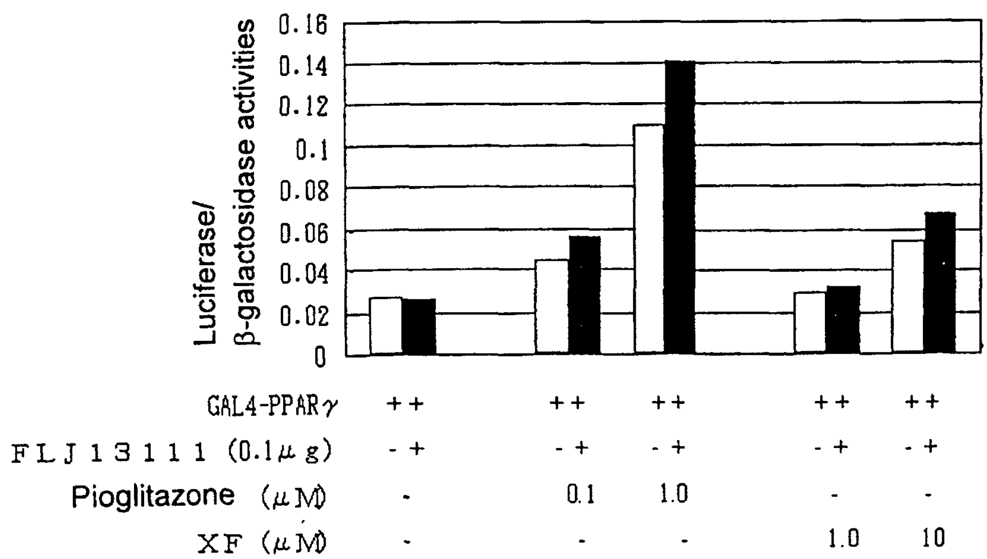

Fig. 12

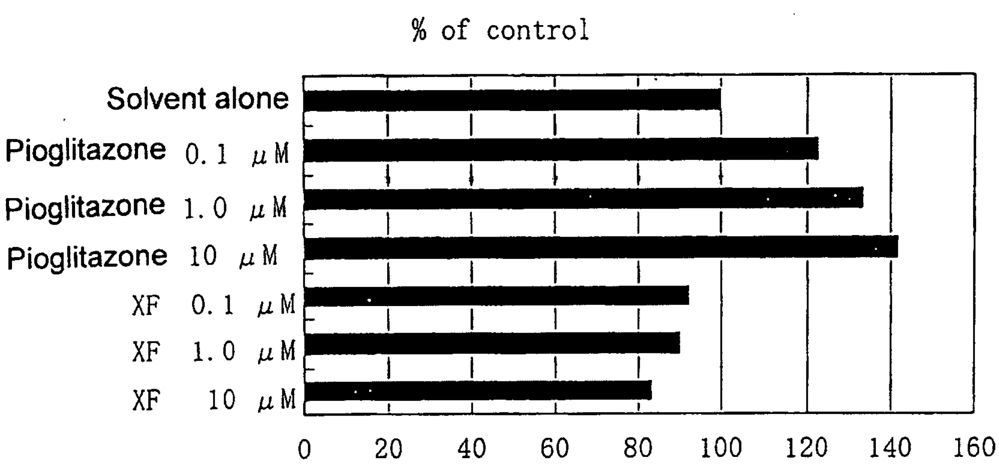
% of control
Solvent alone
Pioglitazone 0.1 μM
Pioglitazone 1.0 μM
Pioglitazone 10 μM
XF 0.1 μM
XF 1.0 μM
XF 10 μM
0
20
40
60
80
100
120
140
160

## INTERNATIONAL SEARCH REPORT

International application No.
PCT/JP03/00546

**A. CLASSIFICATION OF SUBJECT MATTER**
Int.Cl[7] C12N15/12, C12N15/62, C12N15/81, C07K14/705, C07K16/18, C12N1/19, C12N1/21, A61P3/10, C12Q1/66

According to International Patent Classification (IPC) or to both national classification and IPC

**B. FIELDS SEARCHED**

Minimum documentation searched (classification system followed by classification symbols)
Int.Cl[7] C12N15/12, C12N15/62, C12N15/81, C07K14/705, C07K16/18, C12N1/19, C12N1/21, A61P3/10, C12Q1/66

Documentation searched other than minimum documentation to the extent that such documents are included in the fields searched

Electronic data base consulted during the international search (name of data base and, where practicable, search terms used)
CA(STN), PubMed, BIOSIS(DIALOG), WPI(DIALOG), SwissProt/PIR/GeneSeq/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| A | EP 193256 A (Takeda Chemical Industries, Ltd.), 03 September, 1986 (03.09.86), & JP 61-267580 A & US 4687777 A | 1-18 |
| A | JP 2001-340080 A (Yoshitomo OKA), 11 December, 2001 (11.12.01), (Family: none) | 1-18 |
| A | WO 99/12534 A1 (Ono Pharmaceutical Co., Ltd.), 08 March, 1999 (08.03.99), & AU 9889966 A | 1-18 |
| A | TREUTER E. et al., A regulatory role for RIP140 in Nuclear receptor activation., Molecular Endocrinology, 1998, Vol.21, No.6, pages 864 to 881 | 1-18 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 April, 2003 (10.04.03) | 30 April, 2003 (30.04.03) |
| Name and mailing address of the ISA/ Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP03/00546

C (Continuation). DOCUMENTS CONSIDERED TO BE RELEVANT

| Category* | Citation of document, with indication, where appropriate, of the relevant passages | Relevant to claim No. |
|---|---|---|
| A | EP 1057896 A1 (Tanabe Seiyaku Co., Ltd.), 06 December, 2000 (06.12.00), & WO 99/10532 A1 & JP 11-56369 A & US 6365361 B1 | 1-18 |
| A | EP 930299 A1 (Japan Tobacco Inc.), 21 July, 1999 (21.07.99), & JP 9-323982 A & WO 98/07699 A1 & US 6204277 B1 | 1-18 |
| A | WO 97/31907 A1 (GLAXO GROUP LTD.), 04 September, 1997 (04.09.97), & EP 888317 A1 & JP 2000-507216 A & US 6294580 B1 | 1-18 |

Form PCT/ISA/210 (continuation of second sheet) (July 1998)

**INTERNATIONAL SEARCH REPORT**

International application No.
PCT/JP03/00546

**Box I Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 19
because they relate to parts of the international application that do not comply with the prescribed requirements to such an extent that no meaningful international search can be carried out, specifically:
Concerning the "substance obtained by screening" as set forth in the above claim, it is completely unknown what specific compounds are involved and what are not, even referring to the statement in the description (p. 29, lines 2 to 9). Thus this claim is described in (continued to extra sheet)

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)

**INTERNATIONAL SEARCH REPORT**

International application No.
PCT/JP03/00546

Continuation of Box No.I-2 of continuation of first sheet(1)

an extremely unclear manner and, therefore, no meaningful international search can be made thereon.

Form PCT/ISA/210 (extra sheet) (July 1998)